# EUROPEAN PATENT APPLICATION

(11) **EP 4 249 061 A2**
(43) Date of publication of application: **27.09.2023**
(21) Application number: 23184552.0
(22) Date of filing: 02.09.2020
(51) Int. Cl.: A61P 31/04

(54) **IMMUNOGENIC COMPOSITIONS AGAINST ENTERIC DISEASES AND METHODS FOR ITS PREPARATION THEREOF**

(30) Priority: 03.09.2019 IN 201921035435
(62) Divisional of application: 20781107.6
(71) Applicant: Serum Institute Of India Private Limited, Pune, Maharashtra 411 028 (IN)
(72) Inventor: DHERE, Rajeev Mhalasakant, 411 028 Pune Maharashtra (IN); PISAL, Sambhaji Shankar, 411 028 Pune Maharashtra (IN); ANNAMRAJU, Dattatreya Sarma, 411 028 Pune Maharashtra (IN); AVALASKAR, Nikhil Dattatray, 411 028 Pune Maharashtra (IN); HUNDEKARI, Yogesh Tukaram, 411 028 Pune, Maharashtra (IN); TAKLIKAR, Anil Pirajirao, 411 028 Pune Maharashtra (IN); GOEL, Sunil Kumar, 411 028 Pune, Maharashtra (IN); KAMAT, Chandrashekhar Dwarkanath, 411 028 Pune, Maharashtra (IN); CHAVAN, Vishal Bharat, 411 028 Pune, Maharashtra (IN)
(74) Representative: Cabinet Grosset-Fournier & Demachy

(57) **Abstract**

The present disclosure relates to novel immunogenic monovalent and multivalent polysaccharide-protein conjugate vaccine compositions comprising a polysaccharide selected from *Salmonella* serovar strains *S. typhi; S. paratyphi A; S. typhimurium* and *S. enteritidis* and alternative improved methods of polysaccharide fermentation, polysaccharide purification, polysaccharide-protein conjugation and stable formulation. The present disclosure further relates to methods for inducing an immune response in subjects against *Salmonella* typhi and non-typhi related diseases and/or for reducing or preventing *Salmonella* typhi and non-typhi related diseases in subjects using the compositions disclosed herein. The vaccine elicits bactericidal antibodies and is useful for prevention of gastroenteritis, enteric and typhoid fever.

## Description

### FIELD

The present disclosure relates to the field of vaccine manufacturing, more particularly, it relates to an immunogenic composition for prophylaxis against infections caused by *Salmonella* and Non-typhoidal *Salmonella* infections and processes for its preparation.

### BACKGROUND

The background information herein below relates to the present disclosure but is not necessarily a prior art.

*Salmonella* infection remains a serious health problem throughout the world, particularly in developing countries affecting millions of people each year. *Salmonella* infection can cause enteritis which may be complicated by bacteraemia (enteric fever) and gastroenteritis in both normal and immunocompromised individuals.

The genus *Salmonella* belongs to the family of Enterobacteriaceae and comprises Gram-negative, non-spore forming, facultative anaerobic bacilli. *Salmonella* enterica serovar *typhi* (S. *typhi)* and *Salmonella* enterica serovar *paratyphi* (S. *paratyphi)* A and B cause enteric fever, a systemic febrile illness, occurring only in humans that is distinguished from the more commonly self-limited acute gastroenteritis caused by other numerous *Salmonella* serotypes. Enteric fever caused by members of the genus *Salmonella,* including *typhi* and *paratyphi,* continues to constitute a significant disease and mortality burden among populations in developing countries (Lancet 2005; 366:749 762) and represents a notable risk for travellers (Lancet Infect Dis. 2005:5(10):623-628). Typhoid fever remains endemic in low- and middle-income countries (LMICs). Between 12.5-20.6 million cases of enteric fever occur each year in LMICs particularly in south Asia and sub-Saharan Africa (Lancet Glob Heal. 2014; 2: e570-e580 & J Glob Health. 2012; 2: 10401). *Salmonella paratyphi* is responsible for increasing proportion of enteric in parts of Asia, including in Nepal, Cambodia, and China. *Salmonella paratyphi* has highest burdens on the Indian subcontinent and South East Asia. In one of the studies in Nepal where typhoid fever is highly endemic, the municipal water was found to be contaminated with both *S. typhi* and *Salmonella enterica* serovar *paratyphi* A (PLoS Negl Trop Dis. 2016 Jan; 10(1):e0004346 & PLoS Negl Trop Dis. 2013; 7(8):e2391).

Non-typhoidal *Salmonella enterica* (NTS) serovars are important causes of invasive *Salmonella* disease worldwide. Of the more than 2,500 NTS serovars, NTS serovars *typhimurium* and *enteritidis* account for nearly 80 percent of all human isolates of NTS reported globally. Further, invasive non-typhoidal *Salmonella* (iNTS) infections caused by serovars *enteritidis* (SE) and *typhimurium* (STm) are major pediatric health problems in sub-Saharan Africa.NTS has been increasingly recognized recently as a major cause of invasive bacterial infections in young children and immunocompromised individuals, as well as elderly worldwide. These two serovars are also the major cause of gastroenteritis in healthy children and adults in industrialized countries. NTS can also cause severe extra-intestinal, invasive bacteremia, which is referred to as iNTS. It usually presents as a febrile illness. In fact, iNTS frequently occurs without gastrointestinal symptoms in both adults and children.

Of the more than 2,500 non-typhoidal serovars, *Salmonella* enterica subsp. enterica serovar *typhimurium (S. typhimurium)* and *Salmonella* enterica serovar *enteritidis (S. enteritidis)* account for nearly 80 percent of all human isolates of NTS reported globally. NTS has been increasingly recognized recently as a major cause of invasive bacterial infections in young children and HIV-infected individuals in sub-Saharan Africa, as well as elderly and immunocompromised individuals worldwide.

The global incidence of NTS gastroenteritis in 2010 was estimated to be 93 million cases, some 80.3 million of which were via food-borne transmission, with 155,000 deaths. The economic burden of NTS is significant in the developed world. In the United States alone, NTS costs US$3.3 billion per year, with a loss of 17,000 quality-adjusted life years, the most of any food-borne pathogen. As mentioned previously, NTS can also cause severe extra-intestinal, invasive bacteremia, which is referred to as iNTS. Invasive infections of *Salmonella* are more common throughout the developing world and have become the most common cause of bacteremia in tropical Africa, especially among young children and individuals with HIV. It usually presents as a febrile illness. In fact, iNTS frequently occurs without gastrointestinal symptoms in both adults and children. Symptoms of iNTS are similar to malaria and include fever and sweats (more than 90 percent) as well as splenomegaly (40 percent). It is not clear why iNTS is such a problem in Africa, but this could be related to: increased invasiveness of the distinct clades of iNTS bacteria (such as S. *typhimurium* ST313) that are found in Africa and not elsewhere; decreased host immunity related to HIV infection, malaria, and malnutrition; and increased opportunities for human-to-human transmission, e.g., through contaminated water supplies., and NTS bacteremia in HIV-infected African adults has an associated high mortality (up to 47 percent) and recurrence rate (43 percent) rate.

Antibiotics have been used to treat typhoidal and Non-typhoidal *Salmonella* related infections, and the choice of antimicrobials and length of treatment are determined by the cost and availability of antibiotics, local pattern of resistance, and a patient's treatment response. It is becoming increasingly recognized both in the developed and developing world, that multiple antibiotic-resistant strains are emerging as important causes of invasive bacteraemia and gastroenteritis complications, resulting in hospitalizations and deaths.

As available tools for treatment become less effective, the problem of Typhoidal and Non-typhoidal *Salmonella* related infections is likely to continue to increase, making vaccine development an important priority for disease control efforts. It is also an important protective tool for people travelling into areas where Typhoidal and Non-typhoidal *Salmonella* related infections are endemic.

Currently three types of typhoid vaccines are licensed for use i)typhoid conjugate vaccine (TCV) ii) unconjugated Vi polysaccharide (ViPS) vaccine and iii) live attenuated Ty21a vaccine. World Health Organization (WHO) has recommended greater use of typhoid vaccines with preference given to Typhoid Conjugate Vaccines (TCV) (WHO position paper. Wkly Epidemiol Rec 2018; 93:153-72).

Vaccines for *S. paratyphi* are currently not available. Likewise, there is a need for an immunogenic composition/ vaccine which is able to simultaneously confer immunity against typhoidal and non-typhoidal *Salmonella.*

A drawback of the currently-available vaccines is that they are all directed against only S. *typhi. S. paratyphi* A causes enteric fever with the same geographic distribution as S. *typhi,* and the diseases are clinically indistinguishable. Hence, for South and South-East Asia, a vaccine that can protect against both serovars would be more valuable than a vaccine that is restricted to one. Also, in sub-Saharan Africa, the same is true for *S. typhimurium* and *S. enteritidis,* indicating the importance of a vaccine that can additionally protect against NTS serovars for this region.

It is unclear as to whether vaccine candidates in pipeline will be protective against both the gastroenteritis and invasive manifestations of iNTS. While understanding of the disease burden of typhoid in LMICs is growing, the global medical demand for a conjugate vaccine protecting against typhoidal and non-typhoidal *Salmonella* infections has attained significance. It is estimated that the peak demand for a typhoid conjugate vaccine is likely to occur between 2023 and 2026, approaching 300 million annual doses for 133 countries (Clin Infect Dis. 2019 Mar 15; 68(Suppl 2): 5154-5160).

Further, Upstream, Downstream, conjugation and formulation development can often be the rate-limiting step in early introduction of biopharmaceuticals into the market and in meeting the demands of the population. Upstream includes the entire process from early cell isolation and cultivation, to cell banking, to the culture expansion of the bacterial fermentation process and the final harvest. The cell culture is scaled up from 100 to 500 millilitres to a bioreactor of 3 to 20,000 litres. Further steps include primary recovery of *Salmonella* polysaccharide, and elimination of cell and debris. Further, in order to facilitate cost-effective *Salmonella* polysaccharide based conjugate vaccine development, it is necessary to obtain structurally intact polysaccharides with higher yields as well as high purity. Less than 40% yield has been previously reported for *Salmonella* spp polysaccharides. Increasing capsular polysaccharide (CPS) "fermentation harvest stage yield" by employing novel feed strategies, and improved fermentation medium has been one of the preferable approaches to achieve said objective. Merritt et al. (2000) showed that fed batch culture at 500 litre manufacturing scale bioreactor increased the cell density and the yield of capsular polysaccharide approximately fourfold when compared to batch culture.

Studies of capsular polysaccharide production by other pathogenic bacteria such as *Haemophilus influenzae Type B,* and *Neisseria meningitidis* showed that production was dependent upon the fermentation conditions (temperature, pH, DO, osmolality) and the media components and those optimal conditions differed for each bacterium. Zhan et al. (2002) similarly showed that pH control and changing to fed batch fermentation increased the yield of cells and the production of capsular polysaccharide.

The expression of capsular polysaccharide is highly regulated in relation to certain fermentation conditions, such as osmolality wherein reduced synthesis of polysaccharide have been reported when osmolality was high.

The polysaccharides have been produced under different concentrations of glucose, casamino acids, and phosphate ions.

Baruque-Ramos et al., 2005 showed that higher yields of capsular polysaccharide were obtained when *N. Meningitidis* (serogroup C) was cultured in media when the glucose concentration was maintained below 1.0 g/l and that low oxygen tension favoured higher polysaccharide production. Further, it has been observed that concentrated casamino acids in the feed solution limited the final cell density. Further, growth and polysaccharide yield in a defined medium is also dependent on the ratio of carbohydrate to nitrogen source. In one of the Fed Batch Fermentation Process for S. *typhi,* ammonia was supplied as a nitrogen source along with the feed medium. However, it has been observed the Polysaccharide formation by *Aureobasidium pullulans* was affected by the ammonia nitrogen source in the medium, and its yield fell when excess ammonium ions were present, even under conditions which otherwise supported its synthesis (Appl Microbiol Biotechnol (1990)32:637-644).

Growth and polysaccharide synthesis in a defined medium were greatest when amino acids were substituted for ammonia as a nitrogen source.

Use of casamino acid as the nitrogen source in a defined medium has been reported. However, animal derived Casamino acid most probably from bovine casein has been reported as allergen and are restricted in use. Further, use of Casein Digest/tryptone medium as the nitrogen source in a defined medium has been reported however, it may not support the growth of fastidious organisms.

Addition of animal-component-free hydrolysates (Bacto TC Yeastolate, Phytone Peptone) to chemically defined media is one of the approaches to increase cell density, culture viability and productivity in a timely manner. Hydrolysates are protein digests composed of amino acids, small peptides, carbohydrates, vitamins and minerals that provide nutrient supplements to the media. Non- animal derived hydrolysates from soy, wheat and yeast are used commonly in cell culture media and feeds to improve polysaccharide yield (Refer US9284371). However, because of its composition complexity, lot-to-lot variations, undesirable attribute of making culture viscous, Yeast extract and hydrolysates can be a significant source of medium variability. Formation of foam during large scale fermentation could i) reduce capsular polysaccharide yield due to loss of cells and culture medium to the foam phase, ii) can be detrimental to cells since when bubbles burst they exert sheer forces iii) result in a loss of sterility if the foam escapes and iv) can lead to over-pressure if a foam-out blocks an exit filter.

The fermentation cell supernatant is subjected to different steps of purification to isolate purified polysaccharide and eliminate host cell impurities such as proteins, nucleic acid and lipopolysaccharide. Filtration techniques play an important role in downstream processing or purification of bacterial polysaccharides from host cell impurities. Downstream involves inactivation of bacterial culture, separation of the cells from the media, isolation of the product, concentration, purification. Downstream processing is the most challenging part of the process because of its complexity.

Each bacterium has different capsular polysaccharides and different serotypes of the same bacteria further differ in chemical structure of bacterial capsular polysaccharides. A case in point is S. *typhi* expresses a Vi polysaccharide capsule which is a linear homopolymer of α(1-4)-D-GalpA N-acetylated at C-2 and O-acetylated at C-3. The N and O acetyls dominate the surface and are essential for both antigenicity and immunogenicity of Vi, whereas in contrast, S. *paratyphi* A and B and NTS (with rare exceptions) do not express capsular polysaccharides. Rather, their surface polysaccharides are the O polysaccharide (OPS) of lipopolysaccharide. They share a common trisaccharide backbone →2)-α-D-Man*p*-(1→4)-α-L-Rha*p*-(1→3)-α-D-Gal*p*-(1→) (which serologically constitutes epitope 12). However, a dideoxy hexose saccharide linked α-(3→6) at the mannose of the repeating trisaccharide results in an immunodominant epitope that confers *Salmonella* group identity. In case of S. *typhimurium,* the galactose of the trisaccharide backbone epitope 12 becomes α-(1→6) glucosylated. (Refer: Lindberg AA, Le Minor L. Serology of Salmonella In: Bergan T, ed. Methods in Microbiology: Academic Press, 1984:1-141).

This diversity of bacterial polysaccharide structure makes the purification of these polysaccharides more challenging and difficult. The vaccine comprising a polysaccharide needs to meet a certain quality standard.

Previous method of purification that can be performed at large scale volumes includes lysing and precipitation of impurities such as nucleic acids and lipids using solvents, pH manipulation and using detergent such as sodium deoxycholate, Triton-X.

Sodium Deoxycholate (DOC) is a mild detergent and is one of the most commonly used detergent in polysaccharide purifications. Sodium Deoxycholate with a core steroidal structure is less denaturing and limited in its solubilising strength, it breaks the endotoxins without affecting the chemical structure; and hence upon removal of sodium deoxycholate, endotoxins regain their biological activity. Also, DOC based procedures do not work efficiently for removal of contaminants from polysaccharides, especially sialic acid containing polysaccharides. This could be due to weak detergent activity of DOC on Lipopolysaccharide- Protein association formed during the downstream processing, resulting in high level of Endotoxins and protein content in the final isolated polysaccharide. Further Sodium deoxycholate being an animal-origin product, even its residual presence in final product may lead to non-acceptance of product by regulatory agencies and certain religious communities.

The disadvantage of using Triton-X is that the residual detergent persists in the extraction phase and elimination requires extensive washing to remove all the residues.

For some CPS types, precipitation with Zinc acetate/Ammonium sulphate/Sodium citrate for removal of protein contaminants is also included. However, in order to achieve the high level of purity one needs to perform repetitive ammonium sulfate precipitations making the process more tedious and labor intensive. Further, at times it also precipitates capsular polysaccharides, resulting in loss of total polysaccharide.

Some other methods make use of enzymes that help in degradation of proteins and nucleic acid contaminants; however the removal of enzymes and hydrolyzed material is a daunting task and may result in loss of the product of interest. Furthermore, regulatory agencies have restricted the use of animal enzymes in products for humans because of the risk of contamination with prions. The usage of enzymes, besides the fact of high cost, will introduce more regulatory issues in the cGMP framework e.g. the origins of enzymes (from animal or recombinant), enzyme activity variations between different vendors and lots, etc.

Some other methods made use of Benzonase, Proteinase K or Nargase for degradation of residual proteins and/or nucleic acid materials followed by chromatographic purification resulting in high costs and process which can't be scaled up easily.

Some other methods made use of toxic organic solvents like Phenol, butanol, Toluene and chloroform for the separation of endotoxins of bacterial polysaccharides. This method is expensive and time consuming. Furthermore, it is unpleasant to work with toxic organic solvents that produce toxic waste.

The high purities required for polysaccharides specific to vaccine have led to the development of new purification methods based on fractional precipitation; ion exchange chromatography; gel filtration; and affinity chromatography. Chromatographic techniques like Size Exclusion chromatography, Ion exchange chromatography, and hydrophobic interaction chromatography have been successfully used for isolation of bacterial polysaccharides with effective removal of protein and nucleic acid contaminants. Despite the successful isolation of bacterial polysaccharide with WHO specifications, the use of chromatographic techniques involves multistep labour and time consuming sample preparation, involves scalability issues, drastically compromises the recovery of the capsular polysaccharides and thus is not a feasible low cost option for industrial scale downstream processing. Further, the addition of new purification steps to eliminate these contaminants increases the complexity of the process, decreasing the final yields and increasing the economic costs.

The *Salmonella typhi* purified polysaccharides obtained by previously reported purification processes have endotoxin content between 25-50 EU/µg.

Hence, there exists a need of alternative purification methodologies, aimed to maximize recovery of *Salmonella* polysaccharides while removing impurities to acceptable levels.

Various methods such as acid hydrolysis, alkaline degradation, oxidation by periodate, ozonolysis (Wang et al. Carb. Res. 1999, 319, 1-4,141-147), enzymatic hydrolysis, sonication (Pawlowski et al. Vaccine, 2000, 18.18, 1873-1885), electron beam fragmentation (Pawlowski et al. Micro Lett, 1999, 174.2, 255-263) have been described for the depolymerisation (sizing) of bacterial and non-bacterial polysaccharides. However, acid hydrolysis, and alkaline degradation are time consuming and the resultant size reduced sample has high polydispersity. Also periodate oxidation has deleterious effects on labile antigenic epitopes of some polysaccharides. Further, ozonolysis can only be used with polysaccharides containing β-D-aldosidic linkages, and only few endoglycanases have been isolated till date.US 20090041802 discloses fragmentation of Meningococcal polysaccharides by Emulsiflex C-50 (conventional homogenizer) (Avestin). However, Conventional homogenizers operate at peak pressures for mere moments (approximately 7%) of each cycle, leading to wider deviations, less stable products and the need to run more passes or use higher pressures than should be required.

Treatments with ultrasounds have been used to depolymerise polysaccharides (see for example WO 2010/055250). However, ultrasonic depolymerization method is not suited for industrial depolymerization of a large bulk of polysaccharide, because of its low efficiency.

US20090234108 describe method of partial deacetylation of Pneumococcal serotype 1 polysaccharide by chemical treatment with Sodium carbonate buffer (pH 9.0). This process is time consuming and prone to destruction of immunogenic moieties which may affect immunogenicity of conjugates.

For all the reasons stated above, a simple and low-cost process for the depolymerisation of polysaccharides or polysaccharides derivatives is still desirable in the art.

Producing polysaccharide - protein conjugate vaccine is specific to the particular carrier protein and the native polysaccharide involved in the conjugation process.

Various conjugation techniques are known in the art. Conjugates can be prepared by direct reductive amination methods, carbodiimide conjugation chemistry, hydrazides, active esters, norborane, p- nitrobenzoic acid, N- hydroxysuccinimide, S-NHS, EDC, using TSTU. 1-cyano-4-dimethylamino pyridinium tetrafluoroborate (CDAP) conjugation chemistry.

The activated polysaccharide is coupled directly or via a spacer (linker) group to an amino group on to the carrier protein. Linkers used for conjugation as disclosed in prior-art are N-succinimidyl-3-(2- pyridyldithio) propionate (SPDP) (SZU ET AL; 1987). Other linkers, B-propionamido (WO 00/10599), nitrophenyl - ethylamine (Gever et al (1979) Med Microbiol Immunol 165; 171-288), haloalkyl halide (U.S. Pat. No. 4,057,685) glycosidic bond (U.S. Pat. No. 4,673,574), hexane diamine and 6-aminocaproic acid (U.S. Pat. No. 4,459,286). Marburg et al., J. Am. Chem. Soc., 108, 5282 (1986), disclosed one means of conjugating polysaccharides and immunogenic proteins through bigeneric spacers. The Protein (PRO) was derivatized to exhibit pendant nucleophilic or electrophilic groups (PRO*), while a partner Polysaccharide (Ps) was functionalized so as to exhibit pendant groups of opposite reactivity (Ps*). Upon combining Ps* with PRO*, bigeneric spacers were formed, covalently joining Ps to PRO (Ps-PRO). Upon acid hydrolysis, the bigeneric spacer (linker) is released as an unusual amino acid, quantitated by amino acid analysis, and thereby providing a means of proving covalency.

The polysaccharide component of polysaccharide-protein conjugate vaccines undergoes gradual depolymerization at a rate that depends on the type of conjugate, formulation components and storage conditions This results in an increase in free polysaccharide which may adversely affect stability of product. Polysaccharide-carrier protein conjugates are known to release free polysaccharide after conjugation while further processing, lyophilization or storage in liquid as well as solid formulations. Only the *Salmonella* polysaccharide that is covalently bound to the carrier protein (i.e. Conjugated polysaccharide is immunologically important for clinical protection and excessive levels of unbound polysaccharide could potentially result in immunological hyporesponsiveness to polysaccharide (Refer WHO/TRS/924 Page No. 14, A.3.3.5). Particularly, the *Salmonella typhi* conjugates reported in literature have a high free polysaccharide content upto 34% and high free protein content above 5% which indicates lower conjugation efficiency and lower stability of conjugates that is not desirable. Accordingly there is a need for vaccines demonstrating free polysaccharide content less than 10%.

Indeed, if it was possible to have a generic process that could be employed for manufacturing and formulating all vaccine candidates it would greatly reduce the time and resources needed for process development. This can have a significant impact on the number of clinical candidates that can be introduced into clinical trials. Also, processes developed for early stage clinical trials, including those developed using a platform, may be non-optimal with respect to process economics, yield, pool volumes, and throughput and may not be suitable for producing the quantities required for late stage or commercial campaigns. Another important consideration is the speed of process development given that process development needs to occur prior to introduction of a therapeutic candidate into clinical trials. Refer Abhinav A. Shukla et al Journal of Chromatography B, 848 (2007) 28-39.

Further, *Salmonella* disease burden is high in developing countries where availability of electrical power and refrigeration are often inadequate and therefore vaccine stability across temperature excursions assumes greater relevance for these regions.

Thus, there is a need for an efficient platform process for manufacturing an effective vaccine against *Salmonella* serovar strains S. *typhi; S. paratyphi A; S. typhimurium* and S. *enteritidis.* that meets multiple criterion including good immunogenicity, safety and affordability, in particular a platform that provides i) improved polysaccharide yield across fermentation and purification processes; ii) improved purification processes showing optimal percentage recovery and minimum impurity levels; and iii) improved ratio of polysaccharide - protein conjugate in the vaccine iv) improved formulation showing low viscosity, devoid of aggregation; showing long-term stability across wide temperature ranges.

To overcome the aforementioned limitations of prior art and to resolve the long felt unmet global medical need, Applicant proposes improved, alternative fermentation, purification, conjugation processes, formulation(s) for preparing a monovalent *Salmonella* typhoid conjugate as well as multivalent vaccine(s) comprising of atleast one additional conjugates from *Salmonella paratyphi (S. paratyphi* A, B, C), and Non-typhoidal *Salmonella* enterica serovars *typhimurium (S. typhimurium)* and *enteritidis (S. enteritidis)*

### OBJECTS

An object of the present disclosure is to ameliorate one or more problems of the prior art or to at least provide a useful alternative.

It is an object of the present disclosure to develop an effective vaccine formulation for prophylaxis and treatment of infections caused by *Salmonella* serovar strains S. *typhi; S. paratyphi A; S. typhimurium* and S. *enteritidis* in humans.

Another object of the present disclosure is to provide improved processes for the production of polysaccharide - protein conjugate vaccine comprising polysaccharide derived from *Salmonella* serovar strains S. *typhi; S. paratyphi A; S. typhimurium* and S. *enteritidis* that may be employed on an industrial scale.

Yet another object of the present disclosure is to provide a monovalent polysaccharide - protein conjugate vaccine comprising polysaccharide derived from either of *Salmonella* serovar strains S. *typhi; S. paratyphi A; S. typhimurium* and S. *enteritidis.*

Yet another object of the present disclosure is to provide a bivalent polysaccharide - protein conjugate vaccine comprising polysaccharide derived from either of *Salmonella* serovar strains S. *typhi; S. paratyphi A; S. typhimurium* and S. *enteritidis in any combination thereof.*

Yet another object of the present disclosure is to provide a multivalent polysaccharide - protein conjugate vaccine comprising polysaccharide derived from *Salmonella* serovar strains *S. typhi; S. paratyphi A; S. typhimurium* and S. *enteritidis in any combination thereof.*

Yet another objective of the present disclosure is to provide an improved fed-batch methods for production of polysaccharide of *Salmonella* serovar strains *S. typhi; S. paratyphi A; S. typhimurium* and *S. enteritidis.*

Yet another objective of the present disclosure is to provide an improved purification method of polysaccharide derived from *Salmonella* serovar strains *S. typhi; S. paratyphi A; S. typhimurium* and *S. enteritidis.*

Yet another objective of the present disclosure is to provide an improved methods of conjugation of polysaccharide (with or without size reduction) derived from *Salmonella* serovar strains *S. typhi; S. paratyphi A; S. typhimurium* and *S. enteritidis.* to a carrier protein.

Yet another object of the present disclosure is to provide a method of conjugation of polysaccharide (with or without size reduction) derived from *Salmonella* serovar strains *S. typhi; S. paratyphi A; S. typhimurium* and *S. enteritidis* to a carrier protein with or without a linker (spacer) molecule.

Yet another object of the present disclosure is to provide immunogenic vaccine formulations comprising polysaccharide-protein conjugates in an appropriate single dose and multidose vials to be administered in infants and adults at appropriate concentrations effective to confer protection or treatment of infections against *Salmonella* serovar strains *S. typhi; S. paratyphi A; S. typhimurium* and *S. enteritidis* or to prevent, ameliorate, or delay the onset or progression of the clinical manifestations thereof.

Other objects and advantages of the present disclosure will be more apparent from the following description, which is not intended to limit the scope of the present disclosure.

### SUMMARY

The present disclosure provides:
a) an immunogenic composition comprising one or more of polysaccharide-protein conjugate wherein polysaccharide is derived from *Salmonella* serovar strains *S. typhi; S. paratyphi A; S. typhimurium* and *S. enteritidis;*
b) fed-batch methods for cultivation and processing of polysaccharide derived from *Salmonella* serovar strains *S. typhi; S. paratyphi A; S. typhimurium* and *S. enteritidis;*
c) downstream processing steps to obtain polysaccharide derived from *Salmonella* serovar strains *S. typhi; S. paratyphi A; S. typhimurium* and *S. enteritidis;*
d) Methods of Conjugation of polysaccharide (with or without size reduction) derived from *Salmonella* serovar strains *S. typhi; S. paratyphi A; S. typhimurium* and *S. enteritidis* to the carrier protein in the presence or absence of a linker molecule; and
e) methods for eliciting an immune response in a subject via administering a subject of a therapeutically effective amount of the immunogenic composition to confer protection or treatment of infections against *Salmonella* serovar strains *S. typhi; S. paratyphi A; S. typhimurium* and *S. enteritidis* or to prevent, ameliorate, or delay the onset or progression of the clinical manifestations thereof.

### DESCRIPTION

Although the present disclosure may be susceptible to different embodiments, certain embodiments are shown in the following detailed discussion, with the understanding that the present disclosure can be considered an exemplification of the principles of the disclosure and is not intended to limit the scope of disclosure to that which is illustrated and disclosed in this description.

Embodiments are provided so as to thoroughly and fully convey the scope of the present disclosure to the person skilled in the art. Numerous details are set forth, relating to specific components, and processes, to provide a complete understanding of embodiments of the present disclosure. It will be apparent to the person skilled in the art that the details provided in the embodiments should not be construed to limit the scope of the present disclosure. In some embodiments, well-known composition, well-known processes, and well-known techniques are not described in detail.

The terminology used, in the present disclosure, is only for the purpose of explaining a particular embodiment and such terminology shall not be considered to limit the scope of the present disclosure. As used in the present disclosure, the forms "a," "an," and "the" may be intended to include the plural forms as well, unless the context clearly suggests otherwise.

The terms "comprises," "comprising," "including," and "having," are open ended transitional phrases and therefore specify the presence of stated features, integers, steps, operations, elements, modules, units and/or components, but do not forbid the presence or addition of one or more other features, integers, steps, operations, elements, components, and/or groups thereof. The particular order of steps disclosed in the process of the present disclosure is not to be construed as necessarily requiring their performance as described or illustrated. It is also to be understood that additional or alternative steps may be employed.

The terms first, second, third, etc., should not be construed to limit the scope of the present disclosure as the aforementioned terms may be only used to distinguish one element, component, region, layer or section from another component, region, layer or section. Terms such as first, second, third etc., when used herein do not imply a specific sequence or order unless clearly suggested by the present disclosure. The present disclosure provides an immunogenic composition and a process for preparing the same.

The term "vaccine" is optionally substitutable with the term "immunogenic composition" and vice versa.

"D-antigen units" (also referred to as "international units" or IU): The D antigenic form of the poliovirus induces protective neutralising antibodies. D antigen units referred to herein (for instance in the vaccines of the invention) are the measured total D antigen units of each unadsorbed bulk IPV antigen type prior to formulation of the final vaccine which are added in each human dose of formulated vaccine (typically 0.5mL final volume). Reliable methods of measuring D-antigen units are well known in the art and are published, for instance, by the European Pharmacopoeia. For instance, D-antigen units may be measured using the ELISA test as described in Example 1 ("D-antigen quantification by ELISA") below. European Pharmacopoeia provides a test sample (European Pharmacopoeia Biological Reference Preparation - available from Ph. Eur. Secretariat, e.g. Code P 216 0000) for standardisation of such methods between manufacturers (Pharmeuropa Special Issue, Bio 96-2). Thus the D-antigen unit value is well understood in the art.

The term "dose" herein is typically one administration of the vaccine of the invention, which is typically one injection. A typical human dose is 0.5mL. Of course various doses may be administered in a vaccine administration schedule.

The term "IPV" or a immunogenic composition comprising these components herein is intended to mean inactivated polio virus type 1 (e.g. Mahoney, as preferably used), type 2 (e.g. MEF-1), or type 3 (e.g. Saukett), or a Sabin Serotype 1, 2, 3 combination of either two or all three of these types. An example of a full (or standard) dose (40-8-32 D antigen units of Salk based IPV types 1, 2 and 3 respectively) IPV immunogenic composition for the purposes of this invention could be Poliovac^{®} (Serum Institute of India Pvt. Ltd.).

The term "saccharide" throughout this specification may indicate polysaccharide or oligosaccharide and includes both. The capsular saccharide antigen may be a full length polysaccharide or it may be extended to bacterial 'sized-saccharides' and 'oligosaccharides' (which naturally have a low number of repeat units, or which are polysaccharides reduced in size for manageability, but are still capable of inducing a protective immune response in a host.

According to a first embodiment of the present disclosure, the immunogenic composition may comprise one or more of the polysaccharide-protein conjugate, wherein the polysaccharide is derived from *Salmonella* serovar strains *S. typhi; S. paratyphi A; S. typhimurium* and *S. enteritidis.*

According to a second embodiment of the present disclosure, the method of obtaining the polysaccharide derived from *Salmonella* serovar strains *S. typhi; S. paratyphi A; S. typhimurium* and *S. enteritidis,* by fed-batch process may comprise any subset or all of the following steps:
1. Inoculation, cultivation and Harvesting of bacteria in a Fermentation medium compositions,
2. Inactivation,
3. Separation,
4. Clarification, and
5. Sterilized Filtration.

According to a first aspect of second embodiment, the fermentation medium compositions may comprise a carbon source, a magnesium salt, a phosphate source, yeast extract and soy hydrolysate. The carbon source can be selected from the group consisting of glucose, mannitol, sucrose, lactose, fructose, and trehalose, preferably Glucose. The magnesium salt may be selected from magnesium chloride, magnesium sulfate, preferably Magnesium sulfate heptahydrate. The potassium source may be selected from Di-sodium hydrogen phosphate heptahydrate, sodium di-hydrogen phosphate monohydrate, potassium phosphate, and dipotassium phosphate. Preferably the potassium source is a combination of Di-sodium hydrogen phosphate heptahydrate, sodium di-hydrogen phosphate. Preferably, the soy hydrolysate is hysoy.

Yet accordingly the fermentation medium may additionally comprise an anti-foam agent selected from the group of Antifoam 204, Antifoam C, SE-15, Y-30, Antifoam EX-Cell, S184 (pure silicon oil), SLM54474 (polypropylene glycol: PPG), VP1133 (silicon oil/PPG mixture), BREOX (polyalkylene glycol), J673 STRUKTOL (Alkoxylated fatty acid esters on vegetable base) and SE9 (aqueous emulsion with 10% silicon oil component) of Wacker-Chemie Co. The anti-foam agent in combination with soy hydrolysate and yeast extract may aid in improved yield of the polysaccharides. Yet preferably the anti-foam agent may be Antifoam C or J673 STRUKTOL.

In accordance with the embodiments of the present disclosure, the yeast extract may be a yeast autolysate, an ultrafiltered yeast extract, or a synthetic yeast extract. The yeast extract may be selected from BD BBL^{™}, BD BACTO^{™}, Difco^{™} and the like. In a preferred embodiment, the yeast extract may be an ultrafiltered yeast extract, such as Difco^{™} Yeast Extract, UF. The soy hydrolysate may be selected from, but not limited to, soybean meal, soy peptone, and soy flour. In one embodiment, the soy hydrolysate may be Difco^{™} Select Phytone^{™} UF. In another embodiment, the soy hydrolysate may be hysoy.

The combination of antifoam, soya peptone and yeast extract results in improved harvest yield as compared to other media.

According to a second aspect of second embodiment, the process may follow a two shot strategy by incorporating the feed contents at a fixed proportion at particular fixed time intervals during when the fermentation is already undergoing and/or allowing continuous feed throughout the fed-batch mode of fermentation comprising multiple stages with the proportionate increase in the batch size at every stage.

According to a third aspect of second embodiment, the fermentation parameters may comprise of:
Temperature: 36.0 ± 2° C
Agitation: 150 to 600 rpm
pH: 7.0 ± 0.5
Dissolved Oxygen: 30% to 90%
Air (nl/min) : 2 to 10
Gas flow: 60 - 600 nl/min
Osmolality: 400 - 600 mOsm/kg

According to a fourth aspect of second embodiment, the inactivation of the bacterial culture may be carried out using formalin.

Yet preferably the inactivation of the bacterial culture may be carried out by using formaldehyde in the range of 0.1 to 2 % v/v, preferably 0.5 % v/; incubated at 34 to 38 deg C , preferably 36 deg C; for 5-12 hrs, preferably 8 to 12 hrs.

According to a fifth aspect of second embodiment, the separation may be carried out by centrifugation. Yet preferably the separation may be carried out by centrifugation with parameters set at temperature 2-8 deg C; RPM - 7000-8000; Centrifuge time 40 -60 min.

According to a sixth aspect of second embodiment, the clarification may be carried out through depth filtration.

According to a seventh aspect of second embodiment, the clarified harvest may be sterilized through filtration using 0.2 µM sterile filters.

According to eighth aspect of second embodiment, the crude *Salmonella enterica* serovar *typhi* Vi-polysaccharide (ViPs) yield at the fermentation stage may be at least 40% and the average Vi-polysaccharide yield could be in the range of 100 mg/L to 5000 mg/L; more preferably 100-700 mg/L.

According to a third embodiment of the present disclosure, the fermentation harvest may be subjected to any subset or in any order or all of the following downstream purification steps to obtain desired quality of Vi-polysaccharide (ViPs):
a) Clarification of a bacterial capsular polysaccharide harvest by direct flow filtration (DFF) through at least one membrane having a pore size of about 0.2 micrometers;
b) concentration by tangential flow ultrafiltration (TFF) and buffer exchange by diafiltration (DF) using a membrane having 10 - 300 kDa or kD molecular weight cut off (MWCO);
c) treatment with anionic or cationic detergent, ethylene diamine tetra-acetic acid (EDTA) (4 to 10mM) and Sodium acetate (5% to 10%) for denaturation of proteins, nucleic acids and lipopolysaccharide;
d) alcohol precipitation (40% to 70%);
e) Centrifugation and filtration by direct flow filtration (DFF) through at least one clarification filter having a pore size of about 0.2 µM;
f) treatment with akali salt for removal of excess detergent followed by Centrifugation and filtration by direct flow filtration (DFF) through at least one clarification filter having a pore size of about 0.2 µM;
g) concentration by tangential flow filtration (TFF) and buffer exchange by diafiltration (DF) using a membrane having 10 - 300 kDa or kD molecular weight cut off (MWCO);
h) Treatment with anionic or cationic detergent;
i) centrifugation and filtration by direct flow filtration (DFF) through at least one clarification filter having a pore size of about 0.45 micrometers to about 0.2 micrometers;
j) removal of protein and nucleic acid impurities by washing pellet with alcohol (50% to 70%) in presence of sodium chloride (0.1M to 2M);
k) selective precipitation of polysaccharide by utilizing alcohol (<75% OR>95% );
l) dissolving polysaccharide in WFI and subjecting to concentration by tangential flow filtration (TFF) and buffer exchange by diafiltration (DF) using a membrane having 10 - 300kDa or kD molecular weight cut off (MWCO); and
m) Sterile Filtration through at least one sterile filter having a pore size of about 0.2 micrometers under sterile conditions.

According to a first aspect of third embodiment, the purification process may be devoid of any chromatography step.

According to a second aspect of third embodiment, the purification process may result in significant recovery of about 40% to 65% with the desired O-acetyl levels ( greater than 2.0 mmol/g polysaccharide), purified Vi polysaccharide yield could be in the range of 1000 to 4000 mg/L, average molecular weight could be in the range of 40 to 400 kDa, contains less than 1% proteins/peptides, less than 2% nucleic acids, less than 100 EU of endotoxins per µg of polysaccharide (PS), Molecular size distribution (greater than 50% of PS is eluted before a distribution coefficient (KD) of 0.25 is reached)

Yet preferably the average molecular weight of the purified Vi polysaccharide could be in the range of 40 to 400 kDa.

According to a third aspect of third embodiment, the anionic detergent may be selected from the group comprising of alkyl sulfates, sodium dodecyl sulfate (SDS), sodium deoxycholate, sodium dodecyl sulfonate, sodium s-alkyl sulfates, sodium fatty alcohol polyoxyethylene ether sulfates, sodium oleyl sulfate, N-oleoyl poly (amino acid) sodium, sodium alkylbenzene sulfonates, sodium alpha olefin sulfonate , sodium alkyl sulfonates, alpha-sulfo monocarboxylic acid esters, fatty acid sulfoalkyl esters, succinate sulfonate, alkyl naphthalene sulfonates, sodium alkane sulfonates, sodium ligninsulfonate , and sodium alkyl glyceryl ether sulfonates.

Yet preferably, said anionic detergent could be alkyl sulphate, more preferably sodium dodecyl sulphate at a final concentration in the range of 0.1% to 20%, more preferably in the range of 1-20% may be added to the retentate and stirred at 25°C - 30°C for 2 hour.

According to a fourth aspect of third embodiment, the alcohol precipitation may be carried out using methanol, ethanol, n-propyl alcohol, isopropyl alcohol, acetone or t-butyl alcohol; or a combination thereof.

Yet preferably the said alcohol could be ethanol.

According to a fifth aspect of third embodiment, the alkali salt may be selected from the group of sodium, potassium, calcium and magnesium salt. More preferably the alkali salt may be potassium salt selected from the group consisting of potassium chloride, potassium acetate, potassium sulfate, potassium carbonate, potassium bicarbonate, potassium phosphate, potassium hydrogen phosphate, potassium dihydrogen phosphate, potassium nitrate, and other potassium salts, or a combination of two or more thereof.

Yet preferably, the said potassium salt could be potassium chloride at a final concentration in the range of 0.1M to 2M mixed with the supernatant, and upon its dissolution the mixture was incubated at 2-8°C for >3 hours.

According to a sixth aspect of third embodiment, the cationic detergent may be selected from the group comprising of cetyltrimethylammonium salt, tetrabutylammonium salt, myristyltrimethylammonium salt and hexadimethrine bromide; or a combination thereof.

Yet preferably, said cationic detergent could be Cetyl trimethylammonium bromide (CTAB) at a final concentration in the range of 0.1% to 12%; preferably at 2% - 3% may be added to the retentate and stirred at 25°C - 30°C for 1 -2 hour.

According to a seventh aspect of third embodiment, the final purified polysaccharide bulk may be stored at less than or equal to -20°C.

According to a fourth embodiment of the present disclosure, the fermentation harvest may be subjected to any subset or in any order or all of the following downstream purification steps to obtain desired quality of O-specific polysaccharide from *Salmonella Paratyphi A* lipopolysaccharide (LPS):
a) Centrifugation and separation
b) concentration by tangential flow ultrafiltration (TFF) and buffer exchange by diafiltration (DF) using a membrane having 10 - 300kDa molecular weight cut off (MWCO);
c) Acid hydrolysis of LPS
d) Centrifugation and separation
e) Neutralization
f) Clarification of a LPS by direct flow filtration (DFF) through at least one membrane having a pore size of about 0.45 and 0.2 micrometers;
g) treatment with anionic or cationic detergent,
h) Centrifugation and separation
i) Direct flow filtration (DFF) through at least one membrane having a pore size of about 0.45 and 0.2 micrometers;
j) concentration by tangential flow ultrafiltration (TFF) and buffer exchange by diafiltration (DF) using a membrane having 10 - 300kDa molecular weight cut off (MWCO);
k) alcohol precipitation (40% to 70%);
l) Centrifugation and filtration by direct flow filtration (DFF) through at least one clarification filter having a pore size of about 0.2 µM;
m) treatment with akali salt for removal of excess detergent followed by Centrifugation and filtration by direct flow filtration (DFF) through at least one clarification filter having a pore size of about 0.2 µM;
n) concentration by tangential flow filtration (TFF) and buffer exchange by diafiltration (DF) using a membrane having 10 - 300kDa molecular weight cut off (MWCO);
o) removal of protein and nucleic acid impurities by washing pellet with alcohol (50% to 70%) in presence of sodium chloride (0.1M to 2M);
p) dissolving polysaccharide in WFI and subjecting to concentration by tangential flow filtration (TFF) and buffer exchange by diafiltration (DF) using a membrane having 10 - 300kDa molecular weight cut off (MWCO); and
q) Sterile Filtration through at least one sterile filters having a pore size of about 0.2 micrometers under sterile conditions.

According to a first aspect of fourth embodiment, the Acid hydrolysis of LPS may be carried out preferably using acetic acid (final concentration 0.5 -5%) pH~2.0 - 3.0; temperature 30 to 90 deg Celsius and time for about 100 to 200 minutes.

According to a second aspect of fourth embodiment, Acid hydrolysis neutralization may be carried out preferably using liquor ammonia to achieve final pH of 7.0.

According to a third aspect of fourth embodiment, the anionic detergent may be selected from the group comprising of alkyl sulfates, sodium dodecyl sulfate, sodium deoxycholate, sodium dodecyl sulfonate, sodium s-alkyl sulfates, sodium fatty alcohol polyoxyethylene ether sulfates, sodium oleyl sulfate, N-oleoyl poly (amino acid) sodium, sodium alkylbenzene sulfonates, sodium alpha olefin sulfonate , sodium alkyl sulfonates, alpha-sulfo monocarboxylic acid esters, fatty acid sulfoalkyl esters, succinate sulfonate, alkyl naphthalene sulfonates, sodium alkane sulfonates, sodium ligninsulfonate , and sodium alkyl glyceryl ether sulfonates.

Yet preferably, said anionic detergent could be sodium deoxycholate at a final concentration in the range of 0.1% to 20%, more preferably in the range of 1-2% may be added to the retentate and stirred at 25°C - 30°C for 10 - 120 minutes.

According to a fourth aspect of fourth embodiment, the alcohol precipitation may be carried out using methanol, ethanol, n-propyl alcohol, isopropyl alcohol, acetone or t-butyl alcohol; or a combination thereof.

Yet preferably the said alcohol could be ethanol.

According to a fifth aspect of fourth embodiment, the alkali salt may be selected from the group of sodium, potassium, calcium and magnesium salt. More preferably the alkali salt may be potassium salt selected from the group consisting of potassium chloride, potassium acetate, potassium sulfate, potassium carbonate, potassium bicarbonate, potassium phosphate, potassium hydrogen phosphate, potassium dihydrogen phosphate, potassium nitrate, and other potassium salts, or a combination of two or more thereof.

Yet preferably, the said potassium salt could be potassium chloride at a final concentration in the range of 0.1M to 2M mixed with the supernatant, and upon its dissolution the mixture was incubated at 2-8°C for >3 hours.

According to a sixth aspect of fourth embodiment, the cationic detergent may be selected from the group comprising of cetyltrimethylammonium salt, tetrabutylammonium salt, myristyltrimethylammonium salt and hexadimethrine bromide; or a combination thereof.

According to a seventh aspect of fourth embodiment, the purification process may be devoid of any chromatography step.

According to an eighth aspect of fourth embodiment, the purification process may result in

According to a ninth aspect of fourth embodiment, the process results in significant reduction of endotoxin (< 100EU of endotoxin per µg of PS), protein (< 1%) and nucleic acid (< 2%) impurities, higher recovery of capsular polysaccharide suitably in the range of 40% to 65%, with the desired O-acetyl levels (> 2.0 mmol/g polysaccharide), Molecular size distribution (>50% of PS is eluted before a distribution coefficient (KD) of 0.25 is reached) and average molecular weight of the purified O-specific polysaccharide could be in the range of 40 to 200 kDa.

According to a tenth aspect of fourth embodiment, the final purified polysaccharide bulk may be stored at less than or equal to -20°C.

According to a fifth embodiment of the present disclosure, the purified *Salmonella enterica* serovar strains *S. typhi; S. paratyphi A; S. typhimurium* and *S. enteritidis,* polysaccharide may be covalently bound to carrier protein (CP) using a carbodiimide, reductive amination, or cyanylation conjugation reaction.

According to a first aspect of fifth embodiment, the purified *Salmonella enterica* serovar strains *S. typhi; S. paratyphi A; S. typhimurium* and *S. enteritidis,* polysaccharide may be covalently bound to carrier protein (CP) selected from the group comprising of tetanus toxin, tetanus toxoid (TT), diphtheria toxoid (DT), CRM197, Pseudomonas aeruginosa toxoid, Bordetella pertussis toxoid, Clostridium perfringens toxoid, *E.coli* LT, E. coli ST, Escherichia coli heat-labile toxin - B subunit, Neisseria meningitidis outer membrane complex, rEPA, protein D of *H. influenzae,* Flagellin FliC, Horseshoe crab Haemocyanin, exotoxin A from *Pseudomonas aeruginosa,* outer membrane complex c (OMPC), porins, transferrin binding proteins, pneumolysin, pneumococcal surface protein A (PspA), pneumococcal surface adhesin A (PsaA), pneumococcal PhtD, pneumococcal surface proteins BVH-3 and BVH-11, protective antigen (PA) of *Bacillus anthracis* and detoxified edema factor (EF) and lethal factor (LF) of Bacillus anthracis, ovalbumin, keyhole limpet hemocyanin (KLH), human serum albumin, bovine serum albumin (BSA), purified protein derivative of tuberculin (PPD), synthetic peptides, heat shock proteins, pertussis proteins, cytokines, lymphokines, hormones, growth factors, artificial proteins comprising multiple human CD4+ T cell epitopes from various pathogen-derived antigens such as N 19, iron-uptake proteins, toxin A or B from C. difficile and *S.agalactiae* proteins with or without linker and fragments, derivatives, modifications thereof.

Yet preferably the carrier protein used for conjugation with *Salmonella* typhi Vi polysaccharide may be tetanus toxoid. The polysaccharides derived from *S. paratyphi A, S. typhimurium* and *S. enteritidis* may be preferably individually conjugated to a carrier protein selected from tetanus toxoid, diphtheria toxoid or CRM197. Immunogenic compositions comprising the following polysaccharide-carrier proteins conjugates are envisaged in accordance with the present disclosure: *S. typhi* conjugated to tetanus toxoid, *S. paratyphi A* conjugated to TT or DT or CRM197; *S. typhimurium* conjugated to TT or DT or CRM197 and *S. enteritidis* conjugated to TT or DT or CRM197; *S. typhi* conjugated to tetanus toxoid, *S. paratyphi A* conjugated to tetanus toxoid; *S. typhimurium* conjugated to CRM197 and *S. enteritidis* conjugated to CRM197;and S. *typhi* conjugated to tetanus toxoid, *S. paratyphi A* conjugated to DT; *S. typhimurium* conjugated to CRM197 and *S. enteritidis* conjugated to tetanus toxoid and *S. typhi* conjugated to tetanus toxoid, *S. paratyphi A* conjugated to CRM197; *S. typhimurium* conjugated to CRM197 and *S. enteritidis* conjugated to tetanus toxoid.

In accordance with the present disclosure, CRM197 is procured from Recombinant Strain CS463-003 (MB 101) of *Pseudomonas fluorescens* from Pfenex USA.

In accordance with the present disclosure, TT is procured from Clostridium Tetani (Harvard No 49205) obtained from Central research Institute (CRI), National Control Authority, Kasauli, Himachal Pradesh, India. Central research Institute (CRI) procured this strain from NVI, Netherland.

In accordance with the present disclosure, DT is produced from cultures of Cornynebacterium diphtheriae Park-Williams Number 8 strain, the strain is obtained from Central Research Institute (CRI), Kasauli, Himachal Pradesh, India.

According to a second aspect of fifth embodiment, before conjugation the purified *Salmonella enterica* serovar strains *S. typhi; S. paratyphi A; S. typhimurium* and *S. enteritidis,* polysaccharide may be subjected to depolymerization/sizing by chemical means selected from the group of FeCl3, H2O2, sodium metaperiodate and sodium acetate or mechanical means selected from the group of High pressure cell disruption and homogenizer.

Yet preferably, the purified *Salmonella enterica* serovar strains *S. typhi; S. paratyphi A; S. typhimurium* and *S. enteritidis,* polysaccharide may be subjected to depolymerization/sizing by High pressure cell disruption.

Yet preferably, the purified *Salmonella enterica* serovar *typhi* polysaccharide (ViPs) may be subjected to depolymerization/sizing by sodium acetate (5% to 10%) wherein the average molecular weight of the ViPs could be in the range of 40 to 400 kDa.

Yet preferably, the purified *Salmonella enterica* serovar *typhi* polysaccharide (ViPs) may be subjected to depolymerization/sizing by sodium acetate (5% to 10%) wherein the average molecular weight of the ViPs could be in the range of 100- 250 kDa.

Applicant has found that the conjugation efficiency/conjugate yield, immunogenicity of conjugates prepared from using partially size reduced polysaccharides was higher as compared to conjugates prepared from full length polysaccharides Also Size reduction of polysaccharides decreases the viscosity of the solution and increases the number of reactive end groups, both factors contribute to an increased frequency of covalent bond formation.

Yet alternatively the purified *Salmonella enterica* serovar strains *S. typhi; S. paratyphi A; S. typhimurium* and *S. enteritidis,* polysaccharide may not be subjected to depolymerization/sizing.

According to a third aspect of fifth embodiment, before conjugation the carrier protein (CP) may be derivatized to comprise, amino and/or carboxyl groups via a hetero or homo-bifunctional linker selected from the group consisting of hydrazine, carbohydrazide, hydrazine chloride, a dihydrazide, ε-aminohexanoic acid, chlorohexanol dimethyl acetal, D-glucuronolactone, cystamine and p-nitrophenylethyl amine, hexanediamine, ethylenediamine, 1,6-diaminooxyhexane or β-propinamido, nitrophenyl ethylamine, haloalkyl halide, 6-amino caproic acid, and combinations thereof using a carbodiimide, reductive amination or cyanylation reaction.

Yet preferably the hetero or homo-bifunctional linker may be dihydrazide, more preferably adipic acid dihydrazide.

Hydrazide groups can be introduced into proteins through the carboxyl groups of aspartic acid and glutamic acid residues on the protein using a carbodiimide, reductive amination, cyanylation, reaction, for example, by reaction with hydrazine, carbohydrazide, succinyl dihydrazide, adipic acid dihydrazide, hydrazine chloride (e.g., hydrazine dihydrochloride) or any other dihydrazides in the presence of carbodiimide, such as 1-ethyl-3(3-dimethylaminopropyl) carbodiimide (EDC). EDC is employed as a catalyst to activate and modify the protein reactant with hydrazine or the dihydrazide.

Yet preferably, reaction of carrier protein (CP) with adipic acid dihydrazide (ADH) in the presence of carbodiimide, such as 1-ethyl-3(3-dimethylaminopropyl) carbodiimide (EDC) may be carried out at a pH of 5 to 7; more preferably 6.

Accordingly the reaction of carrier protein (CP) with adipic acid dihydrazide (ADH) in the presence of carbodiimide, such as 1-ethyl-3(3-dimethylaminopropyl) carbodiimide (EDC) may be stopped by raising the pH from about 6 to about 7 to 8.

Alternatively, after derivatization of carrier protein with ADH, the method may additionally comprise the step of buffer exchange by diafiltration (DF) using a membrane either of 10 kDa or 30 kDa or 50 kDa molecular weight cut off (MWCO) and sterile filtration using 0.2 u filter; whereby the ADH derivatized carrier protein is either buffer exchanged at least 10 volumes or passed through a suitable gel filtration column and substantially all unreacted compounds, residual ADH and residual EDC are removed, yielding a purified ADH derivatized carrier protein.

Yet alternatively before conjugation the carrier protein may not be derivatized to comprise, amino and/or carboxyl groups via a hetero or homo-bifunctional linker.

According to a third aspect of fifth embodiment, before conjugation the purified *Salmonella enterica* serovar strains *S. typhi; S. paratyphi A; S. typhimurium* and *S. enteritidis,* polysaccharide may be derivatized to comprise, amino and/or carboxyl groups via a hetero or homo-bifunctional linker selected from the group consisting of hydrazine, carbohydrazide, hydrazine chloride, a dihydrazide, a mixture thereof, ε-aminohexanoic acid, chlorohexanol dimethyl acetal, D-glucuronolactone, cystamine and p-nitrophenylethyl amine, hexanediamine, ethylenediamine, 1,6-diaminooxyhexane or β-propinamido, nitrophenyl ethylamine, haloalkyl halide, 6-amino caproic acid, and combinations thereof using a carbodiimide, reductive amination or cyanylation reaction.

Yet preferably the hetero or homo-bifunctional linker may be dihydrazide more preferably adipic acid dihydrazide.

Hydrazide groups can be introduced into the *Salmonella enterica* serovar strains *S. typhi; S. paratyphi A; S. typhimurium* and *S. enteritidis,* polysaccharide by using a carbodiimide, reductive amination, cyanylation reaction for example reaction of polysaccharide with hydrazine, carbohydrazide, succinyl dihydrazide, adipic acid dihydrazide, hydrazine chloride (e.g., hydrazine dihydrochloride) or any other dihydrazides in the presence of cyanogen bromide (CNBr) may form hydrazide derivatives of Vi polysaccharide.

Yet preferably the *Salmonella enterica serovar Paratyphi A* OSP is derivatized with an adipic acid dihydrazide (ADH) linker using cyanylation conjugation chemistry wherein the cyanylation reagent is selected from a group of 1-cyano- 4-pyrrolidinopyridinium tetrafluoroborate (CPPT) (CPIP), 1- cyano- imidazole (1-CI), 1-cyanobenzotriazole (1-CBT), 1-cyano-4-(dimethylamino)-pyridinium tetrafluoroborate ('CDAP'), p-nitrophenylcyanate and N-cyanotriethylammonium tetrafluoroborate ('CTEA') or 2-cyanopyridazine -3(2H) one (2-CPO).

Yet preferably the *Salmonella enterica serovar Paratyphi A* OSP is derivatized with an adipic acid dihydrazide (ADH) linker using cyanylation conjugation chemistry wherein the cyanylation reagent is 1-cyano- 4-pyrrolidinopyridinium tetrafluoroborate (CPPT) (CPIP) mixed in a ratio of 1:1 to 1:10 by weight of OSP: ADH and the ratio of OSP: CPPT may be in between 0.5 and 1.5, reaction carried out at a pH in range of 7-10, reaction duration for 1 - 2 hour, temperature in range of 2°C to 30°C.

Yet another aspect of fifth embodiment, before conjugation the polysaccharide may not be derivatized to comprise, amino and/or carboxyl groups via a hetero or homo-bifunctional linker.

A preferred aspect of fifth embodiment wherein, *Salmonella enterica serovar typhi* polysaccharide (ViPs) may be covalently bound to carrier protein using carbodiimide conjugation chemistry wherein any water-soluble carbodiimide more preferably 1-ethyl-3(3-dimethylaminopropyl) carbodiimide (EDC) can be used as a catalyst.

More preferably the Vi polysaccharide may be covalently bound to ADH derivatized carrier protein using carbodiimide conjugation chemistry wherein any water-soluble carbodiimide more preferably 1-ethyl-3(3-dimethylaminopropyl) carbodiimide (EDC) can be used as a catalyst.

More preferably the Vi polysaccharide may be covalently bound to ADH derivatized tetanus toxoid using carbodiimide conjugation chemistry wherein any water-soluble carbodiimide more preferably 1-ethyl-3(3-dimethylaminopropyl) carbodiimide (EDC) can be used as a catalyst.

Yet alternatively the ADH derivatized Vi polysaccharide may be covalently bound to ADH derivatized tetanus toxoid using carbodiimide conjugation chemistry wherein any water-soluble carbodiimide more preferably 1-ethyl-3(3-dimethylaminopropyl) carbodiimide (EDC) can be used as a catalyst.

Yet alternatively the ADH derivatized Vi polysaccharide may be covalently bound to tetanus toxoid using carbodiimide conjugation chemistry wherein any water-soluble carbodiimide more preferably 1-ethyl-3(3-dimethylaminopropyl) carbodiimide (EDC) can be used as a catalyst.

Yet preferably the Purified ViPs is covalently bound to carrier protein (CP) using carbodiimide conjugation reaction in presence of 1-ethyl-3(3-dimethylaminopropyl) carbodiimide (EDAC) mixed in a ratio of 1:0.5 to 1:2 by weight of ViPs: EDAC and the ratio of Vi polysaccharide and carrier protein may be in between 0.5 and 1.5, reaction carried out at a pH in range of 5-7, temperature in range of 2°C to 30°C.

Yet another aspect of fifth embodiment, the Vi polysaccharide (ViPs) may be covalently bound to ADH derivatized tetanus toxoid (TT) in presence of 1-ethyl-3(3-dimethylaminopropyl) carbodiimide (EDC) wherein the ratio by weight of ViPs:TT:EDC could be 1:1:2 and the concentration of Vi polysaccharide and tetanus toxoid may be between 0.1 mg/mL - 10.0 mg/mL and the ratio of Vi polysaccharide and tetanus toxoid may be in between 0.5 and 1.5.

Yet another aspect of fifth embodiment, the reaction of Vi polysaccharide (ViPs) with derivatized tetanus toxoid (TT) in presence of 1-ethyl-3(3-dimethylaminopropyl) carbodiimide (EDC) may be carried out at a pH in range of 5 to 7; more preferably 6; temperature in range of 2°C to 30°C; more preferably 10 to 25°C and the conjugation conversion efficiency is ≥70% more preferably ≥90% and molecular size of conjugate is preferably between 1000 to 1600 kDa
Further the reaction of derivatized tetanus toxoid (TT) and Vi polysaccharide (ViPs) in the presence of carbodiimide, such as 1-ethyl-3(3-dimethylaminopropyl) carbodiimide (EDC) may be stopped by raising the pH from about 6 to about 7 to 8.

According to one aspect of the fifth embodiment, *S. paratyphi A* polysaccharide (OSP) may be conjugated to a carrier protein using cyanylation conjugation chemistry wherein the cyanylation reagent is selected from a group of 1-cyano- 4-pyrrolidinopyridinium tetrafluoroborate (CPPT), 1- cyano- imidazole (1-CI), 1-cyanobenzotriazole (1-CBT), 1-cyano-4-(dimethylamino)-pyridinium tetrafluoroborate ('CDAP'), p-nitrophenylcyanate and N-cyanotriethylammonium tetrafluoroborate ('CTEA') or 2-cyanopyridazine -3(2H) one (2-CPO).

More preferably *S. paratyphi A* OSP may be conjugated to a carrier protein using cyanylation conjugation chemistry wherein the cyanylation reagent is 1-cyano- 4-pyrrolidinopyridinium tetrafluoroborate (CPPT) or (CPIP).

Yet preferably the purified *S. paratyphi A* OSP is covalently bound to carrier protein (CP) using cyanylation conjugation chemistry wherein the cyanylation reagent is 1-cyano- 4-pyrrolidinopyridinium tetrafluoroborate (CPPT) mixed in a ratio of 1:0.5 to 1:2 by weight of OSP: CPPT and the ratio of OSP and carrier protein may be in between 0.5 and 1.5, reaction carried out at a pH in range of 5-7, temperature in range of 2°C to 30°C.

Yet preferably the carrier protein used for conjugation with *S. paratyphi A* polysaccharide OSP may be Tetanus toxoid.

Yet preferably the carrier protein used for conjugation with *S. paratyphi A* polysaccharide OSP may be Diphtheria toxoid.

Yet preferably the carrier protein used for conjugation with *S. paratyphi A* polysaccharide OSP may be CRM 197.

Yet another aspect of the fifth embodiment, *S. typhimurium* polysaccharide may be conjugated to a carrier protein using cyanylation conjugation chemistry wherein the cyanylation reagent is selected from a group of 1-cyano- 4-pyrrolidinopyridinium tetrafluoroborate (CPPT), 1- cyano- imidazole (1-CI), 1-cyanobenzotriazole (1-CBT), 1-cyano-4-(dimethylamino)-pyridinium tetrafluoroborate ('CDAP'), p-nitrophenylcyanate and N-cyanotriethylammonium tetrafluoroborate ('CTEA') or 2-cyanopyridazine -3(2H) one (2-CPO).

More preferably *S. typhimurium* polysaccharide may be conjugated to a carrier protein using cyanylation conjugation chemistry wherein the cyanylation reagent is 1-cyano- 4-pyrrolidinopyridinium tetrafluoroborate (CPPT).

Yet preferably the carrier protein used for conjugation with *S. typhimurium* polysaccharide may be Tetanus toxoid.

Yet preferably the carrier protein used for conjugation with *S. typhimurium* polysaccharide may be Diphtheria toxoid.

Yet preferably the carrier protein used for conjugation with *S. typhimurium* polysaccharide may be CRM 197.

Yet another aspect of the fifth embodiment, *S. enteritidis* polysaccharide may be conjugated to a carrier protein using cyanylation conjugation chemistry wherein the cyanylation reagent is selected from a group of 1-cyano- 4-pyrrolidinopyridinium tetrafluoroborate (CPPT), 1-cyano- imidazole (1-CI), 1-cyanobenzotriazole (1-CBT), 1-cyano-4-(dimethylamino)-pyridinium tetrafluoroborate ('CDAP'), p-nitrophenylcyanate and N-cyanotriethylammonium tetrafluoroborate ('CTEA') or 2-cyanopyridazine -3(2H) one (2-CPO).

More preferably *S. enteritidis* polysaccharide may be conjugated to a carrier protein using cyanylation conjugation chemistry wherein the cyanylation reagent is 1-cyano- 4-pyrrolidinopyridinium tetrafluoroborate (CPPT).

Yet preferably the carrier protein used for conjugation with *S. enteritidis* polysaccharide may be Tetanus toxoid.

Yet preferably the carrier protein used for conjugation with *S. enteritidis* polysaccharide may be Diphtheria toxoid.

Yet preferably the carrier protein used for conjugation with *S. enteritidis* polysaccharide may be CRM 197.

Applicant has found methods for stabilizing final polysaccharide - protein conjugate by utilizing the alternative methods of conjugation, ratio of polysaccharide to protein, ratio of polysaccharide to coupling agents, using appropriate linkers, appropriate size of the polysaccharide. The same can result in improvement in ratio of polysaccharide - protein conjugate in the vaccine which in turn can reduce the number of free saccharide and free protein in the conjugate, reduced carrier protein suppression, improved sterile filterability of the conjugate, better control of the conjugation, and greater intra-moiety cross-links indirectly provides a good immune response.

Applicant has found that various factors influence the coupling of polysaccharides and proteins which depend upon molecular weight of the ViPs, type of carrier protein selected, the ratio of the amount of polysaccharide: carrier protein used, activation of the functional groups, use of spacers and conjugation chemistry.

Yet another aspect of fifth embodiment, after conjugation reaction the method may comprise the step of concentration by tangential flow ultrafiltration (TFF) and buffer exchange by diafiltration (DF) using a membrane having either of 100 kDa or 300 kDa molecular weight cut off (MWCO); whereby the conjugate bulk is concentrated at least 3 fold and substantially all unreacted compounds, unconjugated polysaccharide, unconjugated protein and residual EDC are removed, yielding a purified Vi polysaccharide conjugate vaccine.

Additionally the method may comprise of Gel filtration chromatography whereby the conjugate bulk is concentrated at least 3 fold and substantially all unreacted compounds, unconjugated polysaccharides, unconjugated proteins and residual EDC are removed, yielding a purified *S. typhi; S. paratyphi A; S. typhimurium* and *S. enteritidis,* polysaccharide conjugate vaccine wherein the conjugate yield is ≥ 50%.

Further method of purification may comprise of combination of ultrafiltration and gel filtration chromatography.

According to a sixth embodiment, the immunogenic composition may be a monovalent vaccine comprising either of *S. typhi* Vi polysaccharide conjugated to a carrier protein or *S. paratyphi A* polysaccharide conjugated to a carrier protein or *S. typhimurium* conjugated to a carrier protein or *S. enteritidis* conjugated to a carrier protein.

According to a seventh embodiment of the present disclosure, wherein the immunogenic composition may comprise of atleast one bivalent combination:
a) *Salmonella enterica serovar typhi* saccharide-carrier protein (CP) conjugate antigen;
b) *Salmonella enterica serovar Paratyphi A* saccharide- carrier protein (CP) conjugate antigen;
or
a) *Salmonella enterica serovar typhi* saccharide-carrier protein (CP) conjugate antigen;
b) *Salmonella enterica serovar typhimurium* saccharide-carrier protein (CP) conjugate antigen;
or
a) *Salmonella enterica serovar typhi* saccharide-carrier protein conjugate antigen;
b) *Salmonella enterica serovar enteritidis* saccharide-carrier protein conjugate antigen;
or
a) *Salmonella enterica serovar Paratyphi A* saccharide-carrier protein conjugate antigen;
b) *Salmonella enterica serovar typhimurium* saccharide-carrier protein conjugate antigen;
or
a) *Salmonella enterica serovar Paratyphi A* saccharide-carrier protein conjugate antigen;
b) *Salmonella enterica serovar enteritidis* saccharide-carrier protein conjugate antigen;
or
a) *Salmonella enterica serovar typhimurium* saccharide-carrier protein conjugate antigen;
b) *Salmonella enterica serovar enteritidis* saccharide-carrier protein conjugate antigen;

Yet preferably 0.5 ml of the composition comprises of *Salmonella enterica serovar typhi* ViPs-TT conjugate antigen in a dose range of 1.25 - 50 µg;
Yet preferably 0.5 ml of the composition comprises of *Salmonella enterica serovar Paratyphi A* OSP-CP conjugate antigen in a dose range of 1.25 - 50 µg; wherein the CP is either TT or DT or CRM197
Yet preferably 0.5 ml of the composition comprises of *Salmonella enterica serovar typhimurium* saccharide-CP conjugate antigen in a dose range of 1.25 - 50 µg; wherein the CP is either TT or DT or CRM197
Yet preferably 0.5 ml of the composition comprises of *Salmonella enterica serovar enteritidis* saccharide-CP conjugate antigen in a dose range of 1.25 - 50 µg; wherein the CP is either TT or DT or CRM197

According to an eighth embodiment of the present disclosure, wherein the immunogenic composition may comprise of atleast one trivalent combination:
a) *Salmonella enterica serovar typhi* saccharide-carrier protein (CP) conjugate antigen;
b) *Salmonella enterica serovar Paratyphi A* saccharide- carrier protein conjugate antigen;
c) *Salmonella enterica serovar typhimurium* saccharide-carrier protein conjugate antigen;
or
a) *Salmonella enterica serovar typhi* saccharide-carrier protein conjugate antigen;
b) *Salmonella enterica serovar typhimurium* saccharide-carrier protein conjugate antigen;
c) *Salmonella enterica serovar enteritidis* saccharide-carrier protein conjugate antigen;
or
a) *Salmonella enterica serovar Paratyphi A* saccharide- carrier protein conjugate antigen;
b) *Salmonella enterica serovar enteritidis* saccharide-carrier protein conjugate antigen;
c) *Salmonella enterica serovar typhimurium* saccharide-carrier protein conjugate antigen;

Yet preferably 0.5 ml of the composition comprises of *Salmonella enterica serovar typhi* ViPs-TT conjugate antigen in a dose range of 1.25 - 50 µg;
Yet preferably 0.5 ml of the composition comprises of *Salmonella enterica serovar Paratyphi A* OSP-CP conjugate antigen in a dose range of 1.25 - 50 µg; wherein the CP is either TT or DT or CRM197
Yet preferably 0.5 ml of the composition comprises of *Salmonella enterica serovar typhimurium* saccharide-CP conjugate antigen in a dose range of 1.25 - 50 µg; wherein the CP is either TT or DT or CRM197
Yet preferably 0.5 ml of the composition comprises of *Salmonella enterica serovar enteritidis* saccharide-CP conjugate antigen in a dose range of 1.25 - 50 µg; wherein the CP is either TT or DT or CRM197

According to a ninth embodiment of the present disclosure, wherein the tetravalent immunogenic composition may comprise of: i) *S. typhi* Vi polysaccharide conjugated to a carrier protein (CP), ii) *S. paratyphi A* polysaccharide conjugated to a carrier protein, iii) S. *enteritidis* polysaccharide conjugated to a carrier protein, and iv) S. *typhimurium* polysaccharide conjugated to a carrier protein.
Yet preferably 0.5 ml of the composition comprises of *Salmonella enterica serovar typhi* ViPs-TT conjugate antigen in a dose range of 1.25 - 50 µg;
Yet preferably 0.5 ml of the composition comprises of *Salmonella enterica serovar Paratyphi A* OSP-CP conjugate antigen in a dose range of 1.25 - 50 µg; wherein the CP is either TT or DT or CRM197
Yet preferably 0.5 ml of the composition comprises of *Salmonella enterica serovar typhimurium* saccharide-CP conjugate antigen in a dose range of 1.25 - 50 µg; wherein the CP is either TT or DT or CRM197
Yet preferably 0.5 ml of the composition comprises of *Salmonella enterica serovar enteritidis* saccharide-CP conjugate antigen in a dose range of 1.25 - 50 µg; wherein the CP is either TT or DT or CRM197

According to a tenth embodiment of the present disclosure, the immunogenic composition may further comprise one or more antigen selected from the group consisting of but not limited to *Salmonella paratyphi* B, *Salmonella paratyphi* C, Salmonella antigens such as Outer membrane vesicles, Outer membrane proteins (eg, OmpC, OmpD, OmpF), siderophores (enterobactin), type III secretion system proteins (eg, SipB, SipD, SseB, SseC, and PrgI), flagellin, *Non-typhoidal Salmonella spp. Shigella, Shigella sonnei, Shigella dysenteriae, Shigella flexneri, Shigella boydii, Escherichia coli, Enterobacter* species, *Yersinia* species, *Pseudomonas* species, *Pseudomonas aeruginosa, Haemophilus influenzae* (a, c, d, e, f serotypes and the unencapsulated strains), Hepatitis (A, C, D, E, F and G strains), *, Influenza, Staphylococcus* spp., *Staphylococcus aureus, Staphylococcus aureus* type 5, *Staphylococcus aureus* type 8, *Streptococcus* spp , *Streptococcus pneumoniae* (1, 2, 3, 4, 5,6A, 6B, 6C, 6D, 6E, 6G, 6H, 7A, 7B, 7C, 7F, 8, 9A,9L,9F,9N, 9V, 10F, 10B,10C, 10A, 11 A,11F,11B, 11C,11D,11E,12A,12B, 12F,13, 14, 15A,15C ,15B,15F,16A, 16F, 17A,17F, 18C,18F,18A,18B, 19A, 19B, 19C, 19F, 20, 20A,20B,21,22A, 22F, 23A,23B, 23F, 24A, 24B,24F , 25F, 25A,27,28F, 28A, 29, 31,32F, 32A,33A, 33C, 33D, 33E, 33F,33B, 34, 45,38,35A,35B,35C,35F,36,37,38, 39, 40,41F,41A,42,43,44,45,46,47F,47A,48), Group A *Streptococcus, Group B Streptococcus(group Ia, Ib, II, III, IV, V, Vl, VII VII, VIII, and IX.), Neisseria meningitidis, Haemophilus pneumonia, Helicobacter pylori, Chlamydia pneumoniae, Chlamydia trachomatis, Ureaplasma urealyticum, Mycoplasma pneumoniae, Streptococcus pyogenes, Streptococcus agalactiae, Streptococcus viridans, Enterococcusfaecalis, Enterococcus faecium, Enterococcus faecalis Neisseria gonorrhoeae, Bacillus anthracis, Vibrio cholerae, Pasteurella pestis, Campylobacter spp., Campylobacter jejuni, Clostridium spp., Clostridium tetani, Clostridium difficile, Mycobacterium spp., Mycobacterium tuberculosis, M. catarrhalis* , *Klebsiella pneumoniae ,Treponema spp., Borrelia spp., Borrelia burgdorferi, Leptospira spp., Hemophilus ducreyi, Corynebacterium diphtheria, Bordetella pertussis, Bordetella parapertussis, Bordetella bronchiseptica, Shigella spp., Erlichia spp., Rickettsia spp and N. meningitidis polysaccharide (A, B, C, D, W135, X, Y, Z and 29E)acellular pertussis antigen, modified adenylate cyclase, Malaria Antigen (RTS, S), anthrax, dengue, malaria, measles, mumps, rubella, BCG, Human papilloma virus, Japanese encephalitis, Dengue, Zika, Ebola, Chikungunya, Poliovirus, Rotavirus, smallpox, yellow fever, Flavivirus, Shingles, and Varicella virus antigens.*

According to a eleventh embodiment of the present disclosure, wherein the composition comprises of atleast one combination:
a) *Salmonella enterica serovar typhi* saccharide-carrier protein conjugate antigen;
b) *Neisseria meningitidis A* saccharide - carrier protein conjugate antigen;
c) *Neisseria meningitidis* C saccharide - carrier protein conjugate antigen;
d) *Neisseria meningitidis Y* saccharide - carrier protein conjugate antigen;
e) *Neisseria meningitidis W* -135 saccharide - carrier protein conjugate antigen;
f) *Neisseria meningitidis X* saccharide - carrier protein conjugate antigen;
or
a) *Salmonella enterica serovar typhi* saccharide-carrier protein conjugate antigen;
b) *Salmonella enterica serovar Paratyphi A* saccharide- carrier protein conjugate antigen;
c) *Neisseria meningitidis A* saccharide - carrier protein conjugate antigen;
d) *Neisseria meningitidis* C saccharide - carrier protein conjugate antigen;
e) *Neisseria meningitidis Y* saccharide - carrier protein conjugate antigen;
f) *Neisseria meningitidis W* -135 saccharide - carrier protein conjugate antigen;
g) *Neisseria meningitidis* X saccharide - carrier protein conjugate antigen;
or
a) *Salmonella enterica serovar typhi* saccharide-carrier protein conjugate antigen;
b) *Salmonella enterica serovar Paratyphi A* saccharide- carrier protein conjugate antigen;
c) *Salmonella enterica serovar typhimurium* saccharide-carrier protein conjugate antigen;
d) *Neisseria meningitidis A* saccharide - carrier protein conjugate antigen;
e) *Neisseria meningitidis* C saccharide - carrier protein conjugate antigen;
f) *Neisseria meningitidis Y* saccharide - carrier protein conjugate antigen;
g) *Neisseria meningitidis* W -135 saccharide - carrier protein conjugate antigen;
h) *Neisseria meningitidis* X saccharide - carrier protein conjugate antigen;
or
a) *Salmonella enterica serovar typhi* saccharide-carrier protein conjugate antigen;
b) *Salmonella enterica serovar Paratyphi A* saccharide- carrier protein conjugate antigen;
c) *Salmonella enterica serovar typhimurium* saccharide-carrier protein conjugate antigen;
d) *Salmonella enterica serovar enteritidis* saccharide-carrier protein conjugate antigen;
e) *Neisseria meningitidis A* saccharide - carrier protein conjugate antigen;
f) *Neisseria meningitidis* C saccharide - carrier protein conjugate antigen;
g) *Neisseria meningitidis Y* saccharide - carrier protein conjugate antigen;
h) *Neisseria meningitidis* W -135 saccharide - carrier protein conjugate antigen;
i) *Neisseria meningitidis X* saccharide - carrier protein conjugate antigen;
or
a) *Salmonella enterica serovar typhimurium* saccharide-carrier protein conjugate antigen;
b) *Salmonella enterica serovar enteritidis* saccharide-carrier protein conjugate antigen;
c) *Neisseria meningitidis A* saccharide - carrier protein conjugate antigen;
d) *Neisseria meningitidis* C saccharide - carrier protein conjugate antigen;
e) *Neisseria meningitidis Y* saccharide - carrier protein conjugate antigen;
f) *Neisseria meningitidis W* -135 saccharide - carrier protein conjugate antigen;
g) *Neisseria meningitidis* X saccharide - carrier protein conjugate antigen

a) *Salmonella enterica serovar typhi* saccharide-carrier protein conjugate antigen;
b) *Neisseria meningitidis A* saccharide - carrier protein conjugate antigen;
c) *Neisseria meningitidis* C saccharide - carrier protein conjugate antigen;
d) *Neisseria meningitidis Y* saccharide - carrier protein conjugate antigen;
e) *Neisseria meningitidis W* -135 saccharide - carrier protein conjugate antigen
or
a) *Salmonella enterica serovar typhi* saccharide-carrier protein conjugate antigen;
b) *Salmonella enterica serovar Paratyphi A* saccharide- carrier protein conjugate antigen;
c) *Neisseria meningitidis A* saccharide - carrier protein conjugate antigen;
d) *Neisseria meningitidis* C saccharide - carrier protein conjugate antigen;
e) *Neisseria meningitidis Y* saccharide - carrier protein conjugate antigen;
f) *Neisseria meningitidis W* -135 saccharide - carrier protein conjugate antigen
or
a) *Salmonella enterica serovar typhimurium* saccharide-carrier protein conjugate antigen;
b) *Salmonella enterica serovar enteritidis* saccharide-carrier protein conjugate antigen;
c) *Neisseria meningitidis A* saccharide - carrier protein conjugate antigen;
d) *Neisseria meningitidis* C saccharide - carrier protein conjugate antigen;
e) *Neisseria meningitidis Y* saccharide - carrier protein conjugate antigen;
f) *Neisseria meningitidis W* -135 saccharide - carrier protein conjugate antigen;
or
a) *Salmonella enterica serovar typhi* saccharide-carrier protein conjugate antigen;
b) *Neisseria meningitidis A* saccharide - carrier protein conjugate antigen;
c) *Neisseria meningitidis* C saccharide - carrier protein conjugate antigen;
or
a) *Salmonella enterica serovar typhi* saccharide-carrier protein conjugate antigen;
b) *Salmonella enterica serovar Paratyphi A* saccharide- carrier protein conjugate antigen;
c) *Neisseria meningitidis A* saccharide - carrier protein conjugate antigen;
d) *Neisseria meningitidis* C saccharide - carrier protein conjugate antigen;
or
a) *Salmonella enterica serovar typhimurium* saccharide-carrier protein conjugate antigen;
b) *Salmonella enterica serovar enteritidis* saccharide-carrier protein conjugate antigen;
c) *Neisseria meningitidis A* saccharide - carrier protein conjugate antigen;
d) *Neisseria meningitidis* C saccharide - carrier protein conjugate antigen;

Yet preferably 0.5 ml of the composition comprises of *Salmonella enterica serovar typhi* ViPs-TT conjugate antigen in a dose range of 1.25 - 50 µg;
Yet preferably 0.5 ml of the composition comprises of *Salmonella enterica serovar Paratyphi A* OSP-CP conjugate antigen in a dose range of 1.25 - 50 µg; wherein the CP is either TT or DT or CRM197
Yet preferably 0.5 ml of the composition comprises of *Salmonella enterica serovar typhimurium* saccharide-CP conjugate antigen in a dose range of 1.25 - 50 µg; wherein the CP is either TT or DT or CRM197
Yet preferably 0.5 ml of the composition comprises of *Salmonella enterica serovar enteritidis* saccharide-CP conjugate antigen in a dose range of 1.25 - 50 µg; wherein the CP is either TT or DT or CRM197
Yet preferably 0.5 ml of the composition comprises of *Neisseria meningitidis A* saccharide - TT conjugate antigen in a dose of 5 µg
Yet preferably 0.5 ml of the composition comprises of *Neisseria meningitidis C* saccharide - CRM197 conjugate antigen in a dose of 5 µg
Yet preferably 0.5 ml of the composition comprises of *Neisseria meningitidis Y* saccharide - CRM197 conjugate antigen in a dose of 5 µg
Yet preferably 0.5 ml of the composition comprises of *Neisseria meningitidis W* saccharide - CRM197 conjugate antigen in a dose of 5 µg
Yet preferably 0.5 ml of the composition comprises of *Neisseria meningitidis X* saccharide - TT conjugate antigen in a dose of 5 µg

According to a twelfth embodiment of the present disclosure, wherein the immunogenic composition comprises of atleast one combination:
a) *Salmonella enterica serovar typhi* saccharide-carrier protein conjugate antigen;
b) an inactivated *polio virus* (IPV) antigen, selected from Salk or Sabin strain
c) a diphtheria toxoid, (D) antigen
d) a tetanus toxoid, (T) antigen
e) a whole cell pertussis, (wP) antigen or acellular pertussis, (aP);
f) a hepatitis B virus surface antigen, (HBsAg) and
g) a *Haemophilus influenzae* type b antigen, (Hib);
or
a) *Salmonella enterica serovar typhi* saccharide-carrier protein conjugate antigen;
b) an inactivated *polio virus* (IPV) antigen, selected from Salk or Sabin strain
c) a rotavirus antigen;
d) a diphtheria toxoid, (D) antigen
e) a tetanus toxoid, (T) antigen
f) a whole cell pertussis, (wP) antigen or acellular pertussis, (aP);
g) a hepatitis B virus surface antigen, (HBsAg) and
h) a *Haemophilus influenzae* type b antigen, (Hib);
or
a) *Salmonella enterica serovar typhi* saccharide-carrier protein conjugate antigen;
b) *Salmonella enterica serovar Paratyphi A* saccharide- carrier protein conjugate antigen;
c) an inactivated *polio virus* (IPV) antigen, selected from Salk or Sabin strain
d) a diphtheria toxoid, (D) antigen
e) a tetanus toxoid, (T) antigen
f) a whole cell pertussis, (wP) antigen or acellular pertussis, (aP);
g) a hepatitis B virus surface antigen, (HBsAg) and
h) a *Haemophilus influenzae* type b antigen, (Hib);
or
a) *Salmonella enterica serovar typhi* saccharide-carrier protein conjugate antigen;
b) *Salmonella enterica serovar Paratyphi A* saccharide- carrier protein conjugate antigen;
c) a rotavirus antigen;
d) an inactivated *polio virus* (IPV) antigen, selected from Salk or Sabin strain
e) a diphtheria toxoid, (D) antigen
f) a tetanus toxoid, (T) antigen
g) a whole cell pertussis, (wP) antigen or acellular pertussis, (aP);
h) a hepatitis B virus surface antigen, (HBsAg) and
i) a *Haemophilus influenzae* type b antigen, (Hib);
or
a) *Salmonella enterica serovar typhi* saccharide-carrier protein conjugate antigen;
b) *Salmonella enterica serovar Paratyphi A* saccharide- carrier protein conjugate antigen;
c) *Salmonella enterica serovar typhimurium* saccharide-carrier protein conjugate antigen;
d) an inactivated *polio virus* (IPV) antigen, selected from Salk or Sabin strain
e) a diphtheria toxoid, (D) antigen
f) a tetanus toxoid, (T) antigen
g) a whole cell pertussis, (wP) antigen or acellular pertussis, (aP);
h) a hepatitis B virus surface antigen, (HBsAg) and
i) a *Haemophilus influenzae* type b antigen, (Hib);
or
a) *Salmonella enterica serovar typhi* saccharide-carrier protein conjugate antigen;
b) *Salmonella enterica serovar Paratyphi A* saccharide- carrier protein conjugate antigen;
c) *Salmonella enterica serovar typhimurium* saccharide-carrier protein conjugate antigen;
d) a rotavirus antigen;
e) an inactivated *polio virus* (IPV) antigen, selected from Salk or Sabin strain
f) a diphtheria toxoid, (D) antigen
g) a tetanus toxoid, (T) antigen
h) a whole cell pertussis, (wP) antigen or acellular pertussis, (aP);
i) a hepatitis B virus surface antigen, (HBsAg) and
j) a *Haemophilus influenzae* type b antigen, (Hib);
or
a) *Salmonella enterica serovar typhi* saccharide-carrier protein conjugate antigen;
b) *Salmonella enterica serovar Paratyphi A* saccharide- carrier protein conjugate antigen;
c) *Salmonella enterica serovar typhimurium* saccharide-carrier protein conjugate antigen;
d) *Salmonella enterica serovar enteritidis* saccharide-carrier protein conjugate antigen;
e) an inactivated *polio virus* (IPV) antigen, selected from Salk or Sabin strain
f) a diphtheria toxoid, (D) antigen
g) a tetanus toxoid, (T) antigen
h) a whole cell pertussis, (wP) antigen or acellular pertussis, (aP);
i) a hepatitis B virus surface antigen, (HBsAg) and
j) a *Haemophilus influenzae* type b antigen, (Hib);
or
a) *Salmonella enterica serovar typhi* saccharide-carrier protein conjugate antigen;
b) *Salmonella enterica serovar Paratyphi A* saccharide- carrier protein conjugate antigen;
c) *Salmonella enterica serovar typhimurium* saccharide-carrier protein conjugate antigen;
d) *Salmonella enterica serovar enteritidis* saccharide-carrier protein conjugate antigen;
e) a rotavirus antigen;
f) an inactivated *polio virus* (IPV) antigen, selected from Salk or Sabin strain
g) a diphtheria toxoid, (D) antigen
h) a tetanus toxoid, (T) antigen
i) a whole cell pertussis, (wP) antigen or acellular pertussis, (aP);
j) a hepatitis B virus surface antigen, (HBsAg) and
k) a *Haemophilus influenzae* type b antigen, (Hib);

Yet preferably 0.5 ml of the composition comprises of *Salmonella enterica serovar typhi* ViPs-TT conjugate antigen in a dose range of 1.25 - 50 µg;
Yet preferably 0.5 ml of the composition comprises of *Salmonella enterica serovar Paratyphi A* OSP-CP conjugate antigen in a dose range of 1.25 - 50 µg; wherein the CP is either TT or DT or CRM197
Yet preferably 0.5 ml of the composition comprises of *Salmonella enterica serovar typhimurium* saccharide-CP conjugate antigen in a dose range of 1.25 - 50 µg; wherein the CP is either TT or DT or CRM197
Yet preferably 0.5 ml of the composition comprises of *Salmonella enterica serovar enteritidis* saccharide-CP conjugate antigen in a dose range of 1.25 - 50 µg; wherein the CP is either TT or DT or CRM197
Yet preferably the composition comprises of diphtheria toxoid, (D) antigen in an amount of 1 to 50 Lf per 0.5 ml
Yet preferably the composition comprises of a tetanus toxoid, (T) in an amount of 1 to 30 Lf per 0.5 ml
Yet preferably the composition comprises of a whole cell pertussis, (wP) antigen in an amount of 1 to 50 IOU per 0.5 ml or acellular pertussis, (aP) antigen comprising one or more of modified adenylate cyclase, Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25µg; per 0.5ml;
Yet preferably the composition comprises of a hepatitis B virus surface antigen, (HBsAg) in an amount of 1 to 20 µg per 0.5 ml;
Yet preferably the composition comprises of a *Haemophilus influenzae* type b antigen, (Hib) in an amount of 1 to 20 µg per 0.5 ml;
Yet preferably the composition comprises of an inactivated rotavirus antigen selected from CDC-9, CDC-66 or any other inactivated rotavirus strains present in an amount in the range of 1 to 50 µg per 0.5 ml;
Yet preferably the composition comprises of an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;

According to a thirteenth embodiment of the present disclosure, wherein the immunogenic composition comprises of atleast one combination:
a) *Salmonella enterica serovar typhi* saccharide-carrier protein conjugate antigen;
b) a rotavirus antigen;
c) a diarrheogenic *Escherichia coli* spp. (enterotoxigenic and enterohemorragic) antigen;
d) a *Shigella* spp. antigen;
e) a *Campylobacter jejuni* antigen;
f) a *Vibrio cholerae* antigen;
or
a) *Salmonella enterica serovar typhi* saccharide-carrier protein conjugate antigen;
b) *Salmonella enterica serovar Paratyphi A* saccharide- carrier protein conjugate antigen;
c) a rotavirus antigen;
d) a diarrheogenic *Escherichia coli* spp. (enterotoxigenic and enterohemorragic) antigen;
e) a *Shigella* spp. antigen;
f) a *Campylobacter jejuni* antigen;
g) a *Vibrio cholerae* antigen;
or
a) *Salmonella enterica serovar typhi* saccharide-carrier protein conjugate antigen;
b) *Salmonella enterica serovar Paratyphi A* saccharide- carrier protein conjugate antigen;
c) a rotavirus antigen;
d) a diarrheogenic *Escherichia coli* spp. (enterotoxigenic and enterohemorragic) antigen;
e) a *Shigella* spp. antigen;
f) a *Campylobacter jejuni* antigen
or
a) *Salmonella enterica serovar typhi* saccharide-carrier protein conjugate antigen;
b) *Salmonella enterica serovar Paratyphi A* saccharide- carrier protein conjugate antigen;
c) a rotavirus antigen;
d) a diarrheogenic *Escherichia coli* spp. (enterotoxigenic and enterohemorragic) antigen;
e) a *Shigella* spp. antigen;
or
a) *Salmonella enterica serovar typhi* saccharide-carrier protein conjugate antigen;
b) *Salmonella enterica serovar Paratyphi A* saccharide- carrier protein conjugate antigen;
c) a rotavirus antigen;
d) a *Shigella* spp. antigen;
or
a) *Salmonella enterica serovar typhimurium* saccharide-carrier protein conjugate antigen;
b) *Salmonella enterica serovar enteritidis* saccharide-carrier protein conjugate antigen;
c) a rotavirus antigen;
d) a diarrheogenic *Escherichia coli* spp. (enterotoxigenic and enterohemorragic) antigen;
e) a *Shigella* spp. antigen;
f) a *Campylobacter jejuni* antigen;
g) a *Vibrio cholerae* antigen;
or
a) *Salmonella enterica serovar typhi* saccharide-carrier protein conjugate antigen;
b) *Salmonella enterica serovar Paratyphi A* saccharide- carrier protein conjugate antigen;
c) *Salmonella enterica serovar typhimurium* saccharide-carrier protein conjugate antigen;
d) a rotavirus antigen;
e) a diarrheogenic *Escherichia coli* spp. (enterotoxigenic and enterohemorragic) antigen;
f) a *Shigella* spp. antigen;
g) a *Campylobacter jejuni* antigen;
h) a *Vibrio cholerae* antigen;
or
a) *Salmonella enterica serovar typhi* saccharide-carrier protein conjugate antigen;
b) *Salmonella enterica serovar Paratyphi A* saccharide- carrier protein conjugate antigen;
c) *Salmonella enterica serovar typhimurium* saccharide-carrier protein conjugate antigen;
d) *Salmonella enterica serovar enteritidis* saccharide-carrier protein conjugate antigen;
e) a rotavirus antigen;
f) a diarrheogenic *Escherichia coli* spp. (enterotoxigenic and enterohemorragic) antigen;
g) a *Shigella* spp. antigen;
h) a *Campylobacter jejuni* antigen;
i) a *Vibrio cholerae* antigen;

Yet preferably 0.5 ml of the composition comprises of *Salmonella enterica serovar typhi* ViPs-TT conjugate antigen in a dose range of 1.25 - 50 µg;
Yet preferably 0.5 ml of the composition comprises of *Salmonella enterica serovar Paratyphi A* OSP-CP conjugate antigen in a dose range of 1.25 - 50 µg; wherein the CP is either TT or DT or CRM197
Yet preferably 0.5 ml of the composition comprises of *Salmonella enterica serovar typhimurium* saccharide-CP conjugate antigen in a dose range of 1.25 - 50 µg; wherein the CP is either TT or DT or CRM197
Yet preferably 0.5 ml of the composition comprises of *Salmonella enterica serovar enteritidis* saccharide-CP conjugate antigen in a dose range of 1.25 - 50 µg; wherein the CP is either TT or DT or CRM197
Yet preferably the composition comprises of an inactivated rotavirus antigen selected from CDC-9, CDC-66 or any other inactivated rotavirus strains present in an amount in the range of 1 to 50 µg per 0.5 ml;

According to one aspect of the embodiment, the immunogenic composition may additionally comprise of a buffering agent selected from the group consisting of carbonate, phosphate, acetate, HEPES, Succinate, Histidine, TRIS, borate, citrate, lactate, gluconate and tartrate, as well as more complex organic buffering agents including a phosphate buffering agent that contains sodium phosphate and/or potassium phosphate in a ratio selected to achieve the desired pH. In another example, the buffering agent contains Tris (hydroxymethyl) aminomethane, or "Tris", formulated to achieve the desired pH. Yet in another example, the buffering agent could be the minimum essential medium with Hanks salts. Other buffers, such as HEPES, piperazine-N, N'-bis (PIPES), and 2-ethanesulfonic acid (MES) are also envisaged by the present disclosure. The buffer aids in stabilizing the immunogenic composition of the present disclosure. The amount of the buffer may be in the range of 0.1 mM to 100 mM, preferably selected from 5mM, 6mM, 7mM, 22 mM, 23mM, 24mM, 25mM, 26 mM, 27 mM, 28 mM, 29 mM and 30 mM. The amount of the buffer may be in the range of 0.1 mg - 2.0 mg.

Citrate buffer may be prepared by dissolving citric acid monohydrate (CAM) in the range of 1.05 to 2.63 mg and trisodium citrate dehydrate (TCD) in the range of 1.47-3.68 mg
Preferably the immunogenic composition may additionally comprise of TRIS or Citrate buffer or Histidine buffer or Succinate Buffer in the range of 0.1 mg - 2.0 mg.

Preferably the immunogenic composition may additionally comprise of TRIS Buffer in the range of 0.61 mg - 1.52 mg.

Preferably the immunogenic composition may additionally comprise of Citrate buffer in the range of 10 mM to 25 mM.

Preferably the immunogenic composition may additionally comprise of Histidine buffer in the range of 0.78 to 1.94 mg.

Preferably the immunogenic composition may additionally comprise of Succinate Buffer in the range of 0.59 to 1.48 mg.

Yet another aspect of the embodiment, the immunogenic composition may additionally comprise of pharmaceutically acceptable excipients selected from the group consisting of sugars, surfactants, polymers, salts, aminoacids or pH modifiers.

Examples of Surfactants may include ionic and non-ionic surfactants such as polysorbate 20, polysorbate 40, polysorbate 60, polysorbate 65, polysorbate 80, polysorbate 85, nonylphenoxypolyethanol, t-Octylphenoxypolyethoxyethanol, oxtoxynol 40, nonoxynol-9, triethanolamine, triethanolamine polypeptide oleate, polyoxyethylene-660 hydroxystearate, polyoxyethylene- 35 ricinoleate, soy lecithin and a poloxamer.

Preferably the immunogenic composition may comprise of polysorbate 20 as pharmaceutically acceptable excipients.

Examples of the polymers may include dextran, carboxymethylcellulose, hyaluronic acid, cyclodextrin, etc.

Examples of the salts may include NaCl, KCl, KH₂PO₄, Na₂HPO₄.2H₂O, CaCl₂, MgCl₂, etc. Preferably, the salt may be NaCl. Typically the amount of the salt may be in the range of 100 mM to 200 mM.

Preferably the immunogenic composition may comprise of Sodium chloride in the range of 1 - 10 mg.

Examples of the aminoacids as excipient selected from the group of L-Histidine, Lysine, Isoleucine, Methionine, Glycine, Aspartic acid. Tricine, arginine, leucine, glutamine, alanine, peptide, hydrolysed protein or protein such as serum albumin.

Preferably the immunogenic composition may comprise of Histidine.

Examples of the sugars as excipient selected from the group of sucrose, mannitol, trehalose, mannose, raffinose, lactitol, lactobionic acid, glucose, maltulose, iso- maltulose, maltose, lactose sorbitol, dextrose, fructose, glycerol, or a combination thereof.

Preferably the immunogenic composition may additionally comprise of Sucrose.

Examples of the polymers as excipient selected from the group of dextran, carboxymethylcellulose, hyaluronic acid, cyclodextrin.

Yet preferably the single dose composition is free of preservative.

Yet preferably the multi-dose immunogenic composition may additionally comprise of preservative selected from the group consisting of 2-phenoxyethanol, Benzethonium chloride (Phemerol), Phenol, m-cresol, Thiomersal, Formaldehyde, paraben esters (e.g. methyl-, ethyl-, propyl- or butyl- paraben), benzalkonium chloride, benzyl alcohol, chlorobutanol, p-chlor-m-cresol, or benzyl alcohol or a combination thereof. A vaccine composition may include material for a single immunization, or may include material for multiple immunizations (i.e. a 'multidose' kit). The inclusion of a preservative is preferred in multidose arrangements. As an alternative (or in addition) to including a preservative in multidose compositions, the compositions may be contained in a container having an aseptic adaptor for removal of material. Preferably, the preservative may be 2-phenoxyethanol in the range of 0.1 mg to 50 mg; more preferably 1 - 10 mg.

Yet another aspect of the embodiment, the immunogenic composition may additionally comprise of auxiliary substances such as wetting or emulsifying agents, diluent pH buffering agents, gelling or viscosity enhancing additives, preservatives, flavoring agents, colors, and the like, depending upon the route of administration and the preparation desired.

Yet preferable aspect of the embodiment, the immunogenic composition may additionally comprise of water for injection as diluent.

Yet another aspect of the embodiment, the immunogenic composition may additionally comprise of an adjuvant selected from the group of aluminum salt, aluminum hydroxide, aluminum phosphate, aluminum hydroxyphosphate, and potassium aluminum sulfate.

Yet another aspect of the embodiment, the immunogenic composition may additionally comprise of an immunostimulatory component selected from the group consisting of an oil and water emulsion, MF-59, a liposome, a lipopolysaccharide, a saponin, lipid A, lipid A derivatives, Monophosphoryl lipid A, 3-deacylated monophosphoryl lipid A, AS01, AS03, an oligonucleotide, an oligonucleotide comprising at least one unmethylated CpG and/or a liposome, Freund's adjuvant, Freund's complete adjuvant, Freund's incomplete adjuvant, polymers, co-polymers such as polyoxyethylene-polyoxypropylene copolymers, including block co-polymers, polymer p 1005, CRL-8300 adjuvant, muramyl dipeptide, TLR-4 agonists, flagellin, flagellins derived from gram negative bacteria, TLR-5 agonists, fragments of flagellins capable of binding to TLR-5 receptors, Alpha-C-galactosylceramide, Chitosan, Interleukin-2, QS-21, ISCOMS, squalene mixtures (SAF-1), Quil A, cholera toxin B subunit, polyphosphazene and derivatives, mycobacterium cell wall preparations, mycolic acid derivatives, non-ionic block copolymer surfactants, OMV, fHbp, saponin combination with sterols and lipids.

Yet another aspect of the embodiment, the immunogenic composition may be fully liquid. Suitable forms of liquid preparation may include solutions, suspensions, emulsions, syrups, isotonic aqueous solutions, viscous compositions and elixirs that are buffered to a selected pH.

Yet preferably the immunogenic composition may be fully liquid, may be stable at 2-8°C, 25°C and 40°C for over a period of six months and free polysaccharide after 6 months not more than 7.5% for 180-220 kDa polysaccharide and free polysaccharide after 6 months not more than 10.5% for 388/80/45 kDa polysaccharide.

The immunogenic composition of the present disclosure may be in the form of transdermal preparations including lotions, gels, sprays, ointments or other suitable techniques. If nasal or respiratory (mucosal) administration is desired (e.g., aerosol inhalation or insufflation), compositions can be in a form and dispensed by a squeeze spray dispenser, pump dispenser or aerosol dispenser. Aerosols are usually under pressure by means of a hydrocarbon. Pump dispensers can preferably dispense a metered dose or a dose having a particular particle size. When in the form of solutions, suspensions and gels, in some embodiments, the immunogenic compositions contain a major amount of water (preferably purified water) in addition to the active ingredient(s).Yet alternative aspect of the embodiment, the immunogenic composition could be lyophilized or freeze dried composition.

As used herein the terms "Freeze-drying" or "lyophilize" or "lyophilization" involves lyophilization and refers to the process by which a suspension/solution is frozen, after which the water is removed by sublimation at low pressure. As used herein, the term "sublimation" refers to a change in the physical properties of a composition, wherein the composition changes directly from a solid state to a gaseous state without becoming a liquid.

Accordingly, the lyophilized immunogenic composition may be stable at 2-8 deg C from 12 to 36 months; at 25 deg C from 2 to 6 months; at 37 deg C from 1 week to 4 weeks, at 42 deg C for 2-7 days, and at 55 deg C for 2-7 days.

According to one aspect of the embodiment, the method for reconstituting a lyophilized immunogenic composition may comprise the step of reconstituting the lyophilized immunogenic composition with an aqueous solution optionally saline or water for injection (WFI) wherein, the final pH of the immunogenic composition after reconstitution is in the range of pH 6.0 to pH 8.0; more preferably in the range of pH 7.0 to pH 8.0; still more preferably in the range of pH 7.2 to pH 7.9; and most preferably in the range of pH 7.5 to pH 7.9.

According to a ninth embodiment of the present disclosure, the immunogenic composition may be formulated for use in a method for reducing the onset of or preventing a health condition comprising *Salmonella* serovar strains *S. typhi; S. paratyphi A; S. typhimurium* and *S. enteritidis* infection involving administration of an immunologically effective amount of the immunogenic composition to a human subject via parenteral or subcutaneous or intradermal, intramuscular or intraperitoneal or intravenous administration or injectable administration or sustained release from implants or administration by eye drops or nasal or rectal or buccal or vaginal, peroral or intragastric or mucosal or perlinqual, alveolar or gingival or olfactory or respiratory mucosa administration or any other routes of immunization.

According to the preferred aspect of the embodiment, the immunogenic composition may be administered to a human subject via intramuscular route or subcutaneous.

According to the preferred aspect of the embodiment, an immunologically-effective amount of the immunogenic composition comprising the polysaccharide - protein conjugate for vaccination against *Salmonella* serovar strains *S. typhi; S. paratyphi A; S. typhimurium* and *S. enteritidis* infection bacterial infection could be from about 1 µg/0.5ml of the Polysaccharide conjugate of *Salmonella* serovar strains *S. typhi; S. paratyphi A; S. typhimurium* or *S. enteritidis* or less to about 100 µg/0.5ml of the Polysaccharide conjugate of *Salmonella* serovar strains *S. typhi; S. paratyphi A; S. typhimurium* or *S. enteritidis* or more. In some other aspects, it could be from about 2, 3, 4, 5, 6, 7, 8, 9, 10, 15, 20, 25, 30, 35, 40, 45 or 50 µg to about 55, 60, 65, 70, 75, 80, 85, 90, or 95 µg per 0.5ml single dose. In some embodiments, the immunologically-effective amount for vaccination against *Salmonella* serovar strains *S. typhi; S. paratyphi A; S. typhimurium* and *S. enteritidis* infection bacterial infection is from 1 µg/0.5ml to 50 µg/0.5ml; more preferably *Salmonella enterica serovar typhi* saccharide-carrier protein conjugate antigen; *Salmonella enterica serovar Paratyphi A* saccharide- carrier protein conjugate antigen; *Salmonella enterica serovar typhimurium* saccharide-carrier protein conjugate antigen; *Salmonella enterica serovar enteritidis* saccharide-carrier protein conjugate antigen; is present in a dose range of about 5 ug/0.5ml to about 30 ug/0.5ml; yet more preferably *Salmonella enterica serovar typhi* saccharide-carrier protein conjugate antigen; *Salmonella enterica serovar Paratyphi A* saccharide- carrier protein conjugate antigen; *Salmonella enterica serovar typhimurium* saccharide-carrier protein conjugate antigen; *Salmonella enterica serovar enteritidis* saccharide-carrier protein conjugate antigen; is present in a dose of about 25 ug/0.5ml

According to tenth embodiment of the present disclosure, the immunogenic composition may be administered intramuscularly or subcutaneous in a dose effective for the production of neutralizing antibody and protection. The vaccines are administered in a manner compatible with the dosage formulation, and in such amount as will be prophylactically and/or therapeutically effective against Typhoidal and NTS infection. The immunogenic composition of the present disclosure can be administered as primary prophylactic agents in elders, adolescents, adults or children at the risk of infection, or can be used as secondary agents for treating infected patients. For example, the immunogenic composition as disclosed herein can be used in elders, adolescents, adults or children less than 2 years of age or more than 2 years of age at risk of *Salmonella* serovar strains *S. typhi; S. paratyphi A; S. typhimurium* and *S. enteritidis* infection, or can be used as secondary agents for treating *Salmonella* serovar strains *S. typhi; S. paratyphi A; S. typhimurium* and *S. enteritidis* infected patients.

Yet alternatively the vaccines for NTS may be administered in infants between the ages of two and four months old, before peak incidence occurs around the age of 12 months. In addition, vaccine implementation would likely also include populations infected with HIV, as they are at heightened risk of infection with NTS. In developed countries, NTS vaccines may target the elderly who experience very high case-fatality rates (up to 50 percent). It has been proposed that, in children, programmatic field implementation would integrate directly with existing Expanded Programme on Immunization schedules, perhaps at 6, 10, and 14 weeks.

More preferably the immunogenic composition may be administered intramuscularly or subcutaneous in a dosage volume of about 0.5ml or 1ml.

According to eleventh embodiment of the present disclosure, the immunogenic composition could be formulated as single dose vials or multidose vials (2 Dose or 5 Dose or 10 Dose vials) or multidose kit or as pre-filled syringes wherein the said immunogenic composition may be given in a single dose schedule, or preferably a multiple dose schedule in which a primary course of vaccination is followed by 1-3 separate doses given at subsequent time intervals after 1-3 years if needed. The dosage regimen will also, at least in part, be determined on the need of a booster dose required to confer protective immunity.

Yet preferably the immunogenic composition may be formulated for administration to a human subject elders, adolescents, adults or children less than 2 years of age or more than 2 years of age according to a one dose or two dose regimens or 3 dose regimens consisting of a first dose and/or a second dose to be administered between 3 months to 2 years after the first dose and/or a third dose to be administered between 3 months to 2 years after the second dose.

According to eleventh embodiment of the present disclosure, the immunogenic composition may be administered concomitantly with other drugs or any other vaccine.

Yet according to one aspect of eleventh embodiment, wherein a single dose vaccine kit may comprise of:
a first container containing a lyophilized (freeze-dried) immunogenic composition:
   a) *Neisseria meningitidis A* saccharide - TT conjugate antigen 5 µg per 0.5 ml;
   b) *Neisseria meningitidis C* saccharide - CRM197 conjugate antigen 5 µg per 0.5 ml;
   c) *Neisseria meningitidis Y* saccharide - CRM197 conjugate antigen 5 µg per 0.5 ml;
   d) *Neisseria meningitidis W* -135 saccharide - CRM197 conjugate antigen 5 µg per 0.5 ml;
   e) *Neisseria meningitidis X* saccharide - TT conjugate antigen 5 µg per 0.5 ml;
   f) Sucrose 1-12 mg per 0.5 ml;
   g) Sodium citrate (Dihydrate) 0.1- 2 mg per 0.5 ml;
   h) Tris Buffer 0.05 - 0.5 mg per 0.5 ml;
   and
a second container containing a liquid composition for the reconstitution of the lyophilized (freeze-dried) immunogenic composition comprising:
   a) *Salmonella enterica serovar typhi* ViPs-TT conjugate antigen 1.25 - 50 µg per 0.5 ml;
   b) Sodium chloride 1 - 10 mg per 0.5 ml;
   c) Water for Injection (WFI) q.s.;
wherein there is no antigenic interference of ViPs-TT with *Neisseria meningitidis* antigens, for prophylaxis against typhoid caused by Salmonella typhi and *Neisseria meningitidis* antigens wherein the said vaccine formulation is sufficient to elicit the required T- dependent immune response against S. typhi including in children below 2 years of age, adolescent adult, and elders through only one injection to comprise a complete vaccination schedule.

Yet according to second aspect of eleventh embodiment, wherein a single dose vaccine kit may comprise of:
a first container containing a lyophilized (freeze-dried) immunogenic composition:
   a) *Neisseria meningitidis A* saccharide - TT conjugate antigen 5 µg per 0.5 ml;
   b) *Neisseria meningitidis C* saccharide - CRM197 conjugate antigen 5 µg per 0.5 ml;
   c) *Neisseria meningitidis Y* saccharide - CRM197 conjugate antigen 5 µg per 0.5 ml;
   d) *Neisseria meningitidis W -135* saccharide - CRM197 conjugate antigen 5 µg per 0.5 ml;
   e) *Neisseria meningitidis X* saccharide - TT conjugate antigen 5 µg per 0.5 ml;
   f) Sucrose 1-12 mg per 0.5 ml;
   g) Sodium citrate (Dihydrate) 0.1- 2 mg per 0.5 ml;
   h) Tris Buffer 0.05 - 0.5 mg per 0.5 ml;
   and
a second container containing a liquid composition for the reconstitution of the lyophilized (freeze-dried) immunogenic composition comprising:
   a) *Salmonella enterica serovar typhi* ViPs-TT conjugate antigen 1.25 - 50 µg per 0.5 ml;
   b) *Salmonella enterica serovar paratyphi A* OSP - CP conjugate antigen 1.25 - 50 µg per 0.5 ml; wherein the CP is either TT or DT or CRM197;
   c) Sodium chloride 1 - 10 mg per 0.5 ml;
   d) Water for Injection (WFI) q.s.;
wherein there is no antigenic interference of ViPs-TT; OSP antigen with *Neisseria meningitidis* antigens, for prophylaxis against typhoid and paratyphoid caused by Salmonella typhi, S. paratyphi and *Neisseria meningitidis* antigens wherein the said vaccine formulation is sufficient to elicit the required T- dependent immune response against S. typhi and paratyphi including in children below 2 years of age, adolescent adult, and elders through only one injection to comprise a complete vaccination schedule.

Yet according to third aspect of eleventh embodiment, wherein a single dose vaccine kit may comprise of:
a first container containing a fully liquid hexavalent immunogenic composition:
   a) an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;
   b) a diphtheria toxoid, (D) antigen in an amount of 1 to 50 Lf per 0.5 ml;
   c) a tetanus toxoid, (T) antigen in an amount of 1 to 30 Lf per 0.5 ml;
   d) a whole cell pertussis, (wP) antigen in an amount of 1 to 50 IOU per 0.5 ml or acellular pertussis, (aP) antigen comprising one or more of modified adenylate cyclase selected from Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25µg; per 0.5ml;
   e) a hepatitis B virus surface antigen, (HBsAg) in an amount of 1 to 20 µg per 0.5 ml;
   f) a *Haemophilus influenzae* type b antigen, (Hib) in an amount of 1 to 20 µg per 0.5 ml;
   and
a second container containing a fully liquid immunogenic composition comprising:
   a) *Salmonella enterica serovar typhi* ViPs-TT conjugate antigen 1.25 - 50 µg per 0.5 ml;
   b) Sodium chloride 1 - 10 mg per 0.5 ml; and/or
   c) Tris Buffer or Citrate buffer or Histidine buffer or Succinate Buffer 0.1 mg - 1.6 mg per 0.5 ml; and/or
   d) Polysorbate selected from polysorbate 20, polysorbate 40, polysorbate 60, polysorbate 65, polysorbate 80, polysorbate 85, nonylphenoxypolyethoxethanol, octylphenoxypolyethoxethanol, oxtoxynol 40, nonoxynol- 9, triethanolamine, triethanolamine polypeptide oleate, polyoxyethylene- 660 hydroxystearate, polyoxyethylene- 35 ricinoleate, soy lecithin and a poloxamer in an amount of 25-500 µg per 0.5 ml; and/or
   e) 2-Phenoxyethanol 1-10 mg per 0.5 ml; and/or
   f) Water for Injection (WFI) q.s.
wherein there is no antigenic interference of ViPs-TT with Hexavalent immunogenic composition, for prophylaxis against typhoid caused by Salmonella typhi and Hexavalent antigens wherein the said vaccine formulation is sufficient to elicit the required T- dependent immune response against S. typhi including in children below 2 years of age, adolescent adult, and elders through only one injection to comprise a complete vaccination schedule.

Yet according to fourth aspect of eleventh embodiment, wherein a single dose vaccine kit may comprise of:
a first container containing a fully liquid hexavalent immunogenic composition:
   a) an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;
   b) a diphtheria toxoid, (D) antigen in an amount of 1 to 50 Lf per 0.5 ml;
   c) a tetanus toxoid, (T) antigen in an amount of 1 to 30 Lf per 0.5 ml;
   d) a whole cell pertussis, (wP) antigen in an amount of 1 to 50 IOU per 0.5 ml or acellular pertussis, (aP) antigen comprising one or more of modified adenylate cyclase selected from Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25pg; per 0.5ml;
   e) a hepatitis B virus surface antigen, (HBsAg) in an amount of 1 to 20 µg per 0.5 ml;
   f) a *Haemophilus influenzae* type b antigen, (Hib) in an amount of 1 to 20 µg per 0.5 ml;
   and
a second container containing a fully liquid immunogenic composition comprising:
   a) *Salmonella enterica serovar typhi* ViPs-TT conjugate antigen 1.25 - 50 µg per 0.5ml;
   b) *Salmonella enterica serovar paratyphi A* OSP - CP conjugate antigen 1.25 - 50 µg per 0.5ml; wherein the CP is either TT or DT or CRM197;
   c) Sodium chloride 1 - 10 mg; and/or
   d) Tris Buffer or Citrate buffer or Histidine buffer or Succinate Buffer 0.1 mg - 1.6 mg per 0.5ml; and/or
   g) Polysorbate selected from polysorbate 20, polysorbate 40, polysorbate 60, polysorbate 65, polysorbate 80, polysorbate 85, nonylphenoxypolyethoxethanol, octylphenoxypolyethoxethanol, oxtoxynol 40, nonoxynol- 9, triethanolamine, triethanolamine polypeptide oleate, polyoxyethylene- 660 hydroxystearate, polyoxyethylene- 35 ricinoleate, soy lecithin and a poloxamer; in an amount of 25-500 µg per 0.5 ml; and/or
   h) 2-Phenoxyethanol 1-10 mg per 0.5ml; and/or
   e) Water for Injection (WFI) q.s.
wherein there is no antigenic interference of ViPs-TT; OSP antigen with *Hexavalent* antigens, for prophylaxis against typhoid and paratyphoid caused by Salmonella typhi, S. paratyphi and *Hexavalent* antigens wherein the said vaccine formulation is sufficient to elicit the required T- dependent immune response against S. typhi and paratyphi including in children below 2 years of age, adolescent adult, and elders through only one injection to comprise a complete vaccination schedule.

Yet according to fifth aspect of eleventh embodiment, wherein a single dose vaccine kit may comprise of:
a first container containing a fully liquid heptavalent immunogenic composition:
   a) an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;
   b) an inactivated rotavirus antigen selected from CDC-9, CDC-66 or any other inactivated rotavirus strains present in an amount in the range of 1 to 50 µg per 0.5 ml;
   c) a diphtheria toxoid, (D) antigen in an amount of 1 to 50 Lf per 0.5 ml;
   d) a tetanus toxoid, (T) antigen in an amount of 1 to 30 Lf per 0.5 ml;
   e) a whole cell pertussis, (wP) antigen in an amount of 1 to 50 IOU per 0.5 ml or acellular pertussis, (aP) antigen comprising one or more of modified adenylate cyclase selected from Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25µg; per 0.5ml;
   f) a hepatitis B virus surface antigen, (HBsAg) in an amount of 1 to 20 µg per 0.5 ml;
   g) a *Haemophilus influenzae* type b antigen, (Hib) in an amount of 1 to 20 µg per 0.5 ml;
   and
a second container containing a liquid composition for the reconstitution of the lyophilized (freeze-dried) immunogenic composition comprising:
   a) *Salmonella enterica serovar typhi* ViPs-TT conjugate antigen 1.25 - 50 µg per 0.5 ml;
   b) Sodium chloride 1 - 10 mg per 0.5 ml;
   c) Water for Injection (WFI) q.s.;
wherein there is no antigenic interference of ViPs-TT with Heptavalent immunogenic composition, for prophylaxis against typhoid caused by Salmonella typhi and Heptavalent antigens wherein the said vaccine formulation is sufficient to elicit the required T- dependent immune response against S. typhi including in children below 2 years of age, adolescent adult, and elders through only one injection to comprise a complete vaccination schedule.

Yet according to sixth aspect of eleventh embodiment, wherein a single dose vaccine kit may comprise of:
a first container containing a fully liquid heptavalent immunogenic composition:
   a) an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;
   b) an inactivated rotavirus antigen selected from CDC-9, CDC-66 or any other inactivated rotavirus strains present in an amount in the range of 1 to 50 µg per 0.5 ml;
   c) a diphtheria toxoid, (D) antigen in an amount of 1 to 50 Lf per 0.5 ml;
   d) a tetanus toxoid, (T) antigen in an amount of 1 to 30 Lf per 0.5 ml;
   e) a whole cell pertussis, (wP) antigen in an amount of 1 to 50 IOU per 0.5 ml or acellular pertussis, (aP) antigen comprising one or more of modified adenylate cyclase selected from Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25µg; per 0.5ml;
   f) a hepatitis B virus surface antigen, (HBsAg) in an amount of 1 to 20 µg per 0.5 ml;
   g) a *Haemophilus influenzae* type b antigen, (Hib) in an amount of 1 to 20 µg per 0.5 ml;
   and
a second container containing a fully liquid immunogenic composition comprising:
   a) *Salmonella enterica serovar typhi* ViPs-TT conjugate antigen 1.25 - 50 µg per 0.5 ml;
   b) *Salmonella enterica serovar paratyphi A* OSP - CP conjugate antigen 1.25 - 50 µg per 0.5 ml; wherein the CP is either TT or DT or CRM197;
   c) Sodium chloride 1 - 10 mg per 0.5 ml; and/or
   d) Tris Buffer or Citrate buffer or Histidine buffer or Succinate Buffer 0.1 mg - 1.6 mg per 0.5 ml; and/or
   g) Polysorbate selected from polysorbate 20, polysorbate 40, polysorbate 60, polysorbate 65, polysorbate 80, polysorbate 85, nonylphenoxypolyethoxethanol, octylphenoxypolyethoxethanol, oxtoxynol 40, nonoxynol- 9, triethanolamine, triethanolamine polypeptide oleate, polyoxyethylene- 660 hydroxystearate, polyoxyethylene- 35 ricinoleate, soy lecithin and a poloxamer in an amount of 25-500 µg per 0.5 ml; and/or
   h) 2-Phenoxyethanol 1-10 mg per 0.5 ml; and/or
   e) Water for Injection (WFI) q.s.
wherein there is no antigenic interference of ViPs-TT; OSP antigen with *Heptavalent* antigens, for prophylaxis against typhoid and paratyphoid caused by Salmonella typhi, S. paratyphi and *Heptavalent* antigens wherein the said vaccine formulation is sufficient to elicit the required T- dependent immune response against S. typhi and paratyphi including in children below 2 years of age, adolescent adult, and elders through only one injection to comprise a complete vaccination schedule.

*Salmonella* serovar strains *S. typhi; S. paratyphi A; S. typhimurium* and S. *enteritidis* used for developing the immunogenic composition of the present disclosure may include: *Salmonella* enterica serovar *typhi* TY2 strain with "tviB" gene specific for Vi polysaccharide (Identified by Geneombio Technologies Private Limited, Pune and isolated from stool sample of typhoid confirmed patient at Villoo Poonawalla Memorial Hospital, Pune); S. *typhi:* ATCC 19430; C6524 (NICED, Kolkata, India); *S. paratyphi A:* ATCC 9150 procured from Chromachemie Laboratory Private Limited, Bangalore, CMCC50073, CMCC50973; *S. enteritidis:* ATCC 4931; ATCC 13076; *S. enteritidis* R11; *S. enteritidis* D24359; *S. enteritidis* 618; *S. enteritidis* 502; *S. enteritidis* IV3453219;S. *typhimurium: S. typhimurium* 2192; ATCC 14208; *S. typhimurium* 2189; *S. typhimurium* D23580; ATCC 19585; ATCC 700408; (LT2/SL134 (ST19)); *S.typhimurium* 177(ST19) CDC 6516-60; ATCC 700720. Further any attenuated *Salmonella* serovar strain (*S. typhi, S. paratyphi A, S. enteritidis* and *S. typhimurium)* may be used for the preparation of the immunogenic composition of the present disclosure.

*Salmonella* enterica serovar *typhi* deposited at NCMR-NCCS; Pune, an International Depositary Authority having assigned Accession No. MCC 0193; Strain designation- PDL-1.

Other embodiments disclosed herein also encompasses vaccine kit comprising a first container containing a lyophilized (freeze-dried) immunogenic composition and a second container containing an aqueous solution optionally saline or WFI (water for injection) for the reconstitution of the lyophilized (freeze-dried) immunogenic composition.

The foregoing description of the specific embodiments fully reveals the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein has been described in terms of preferred embodiments, those skilled in the art will recognize that the embodiments herein can be practiced with modification within the spirit and scope of the embodiments as described herein.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The use of the expression "one or more" or "at least one" suggests the use of one or more elements or ingredients or quantities, as the use may be in the embodiment of the invention to achieve one or more of the desired objects or results.

Any discussion of documents, acts, materials, devices, articles or the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

The numerical values given for various physical parameters, dimensions and quantities are only approximate values and it is envisaged that the values higher than the numerical value assigned to the physical parameters, dimensions and quantities fall within the scope of the invention unless there is a statement in the specification to the contrary.

While considerable emphasis has been placed herein on the specific features of the preferred embodiment, it will be appreciated that many additional features can be added and that many changes can be made in the preferred embodiment without departing from the principles of the disclosure. These and other changes in the preferred embodiment of the disclosure will be apparent to those skilled in the art from the disclosure herein, whereby it is to be distinctly understood that the foregoing descriptive matter is to be interpreted merely as illustration of the disclosure and not as a limitation.

### TECHNICAL ADVANTAGES:

1. The present disclosure provides monovalent and multivalent multivalent polysaccharide - protein conjugate vaccine comprising polysaccharide derived from *Salmonella* serovar strains *S. typhi; S. paratyphi A; S. typhimurium* and *S. enteritidis in any combination thereof* effective to confer protection or treatment of infections against *Salmonella* serovar strains *S. typhi; S. paratyphi A; S. typhimurium* and *S. enteritidis* or to prevent, ameliorate, or delay the onset or progression of the clinical manifestations thereof.
2. Improved upstream, downstream and conjugation process
3. Upstream fermentation media is devoid of casein digest/Tryptone, casamino acids.
4. Use of i) combination of antifoam , soya peptone and yeast extract ii) particular DO 36-39% and iii) osmolality 400 - 600 mOsmol/kg thereby resulting in improvement in harvest stage polysaccharide yield of >50%
5. Improved purification method with low endotoxin <10 EU/µg, low protein <1% and low nucleic acid content <2% wherein purification process is i) devoid of Sodium deoxycholate (DOC) being an animal-origin product, even its residual presence in final product may lead to non-acceptance of product by regulatory agencies and certain religious communities ii) devoid of Triton X iii) devoid of ammonium sulphate iv) devoid of any Adsorbents (hydroxylapatite, calcium phosphate or apatite)
6. Improved conjugation efficiency (> 60%), improved conjugate yield (≥50%) and stability of conjugate wherein i) Ps:Pr: EDAC ratio is 1:1:2 ii) stability indicating free polysaccharide is < 5% (preferably < 3%) and free protein is < 5% (preferably < 4%)
7. Improved immunogenicity for conjugate attributed to i) Vi polysaccharide - protein conjugate having a peculiar size between 1200 kDa to 1600 kDa. and ii) Polysaccharide used for conjugation has average molecular weight between 150 and 300 kDa (preferably between 150 and 250 kDa)
8. The immunogenic composition is stable at 2-8°C, 25°C and 40°C for over a period of six months and free polysaccharide after 6 months not more than 7.5% for 180-220 kDa polysaccharide and free polysaccharide after 6 months not more than 10.5% for 388/80/45 kDa polysaccharide.
9. The mice groups injected with bivalent (typhoid and paratyphoid) SIIPL vaccine such as a) SIIPL Vi PS-TT + SIIPL O-SP A DT, b) SIIPL Vi PS-TT + SIIPL O-SP A TT and c) SIIPL Vi PS-TT + SIIPL O-SP A CRM Vi TT exhibited greater than 4-fold higher induction of IgG (as compared to mice administered with unconjugated Vi PS or unconjugated SIIPL O-SP A) thus indicating the immunogenic potential of bivalent SIIPL vaccine containing combination of Vi TT and SIIPL O-SP A (DT/TT/CRM).
10. Minimum components involved in the vaccine composition.

### EXAMPLES

The following examples are included to demonstrate preferred embodiments of the invention. It should be appreciated by those of skill in the art that the compositions and techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1:

### A) STRAINS:

*Salmonella* serovar strains *S. typhi; S. paratyphi A; S. typhimurium* and *S. enteritidis* used for developing the immunogenic composition of the present disclosure may include: *Salmonella* enterica serovar *typhi* TY2 strain with "tviB" gene specific for Vi polysaccharide (Identified by Geneombio Technologies Private Limited, Pune and isolated from stool sample of typhoid confirmed patient at Villoo Poonawalla Memorial Hospital, Pune); *Salmonella* enterica serovar *typhi* deposited at NCMR-NCCS; Pune, an International Depositary Authority having assigned Accession No. MCC 0193; Strain designation- PDL-1, *S. typhi:* ATCC 19430; C6524 (NICED, Kolkata, India); *S. paratyphi A:* ATCC 9150 procured from Chromachemie Laboratory Private Limited, Bangalore, CMCC50073, CMCC50973; *S. enteritidis:* ATCC 4931; ATCC 13076; *S. enteritidis* R11; *S. enteritidis* D24359; *S. enteritidis* 618; *S. enteritidis* 502; *S. enteritidis* IV3453219;S. *typhimurium: S. typhimurium* 2192; ATCC 14208; S. *typhimurium* 2189; *S. typhimurium* D23580; ATCC 19585; ATCC 700408; (LT2/SL134 (ST19)); *S.typhimurium* 177(ST19) CDC 6516-60; ATCC 700720. Further any attenuated *Salmonella* serovar strain (*S. typhi, S. paratyphi A, S. enteritidis* and *S. typhimurium)* may be used for the preparation of the immunogenic composition of the present disclosure.

WO2018037365, WO2019016654 and WO2020075184 are being incorporated with reference to the preparation of diphtheria toxoid (DT), tetanus toxoid (TT), inactivated whole cell pertussis, (wP), acellular pertussis, (aP), hepatitis B virus surface antigen (HBsAg), Haemophilus influenzae type b antigen (Hib) and inactivated poliovirus (standard dose and dose reduced poliovirus).

WO2018037365 is being incorporated with reference to the preparation of inactivated poliovirus and inactivated rotavirus.

WO2013114268 is being incorporated with reference to the preparation of meningococcal polysaccharide antigens from serotypes A, C, W, X and Y.

### B) The method of obtaining the polysaccharide derived from Salmonella serovar strains S. typhi; S. paratyphi A; S. typhimurium and S. enteritidis, by fed-batch process comprise of the following steps: (UPSTREAM FERMENTATION)

### 1) Inoculation and harvesting of S1 stage:

0.5 mL culture from cell bank vial is diluted to 5 mL with medium and loopful culture from this is streaked on SCDA plate and incubated at 36±0.5°C for 28 to 32 Hrs.

### 2) Inoculation and harvesting of S2 stage:

10 well isolated colonies from the S1 stage plate are inoculate into the conical flask containing 40 mL medium. Incubate the flask at 36±0.5°C and 150±10 RPM until Optical Density (OD) of the culture reaches between 2.5 to 3.5 range.

### 3) Inoculation and harvesting of S3 stage:

When Optical Density (OD) of the S2 stage culture reaches between 2.5 to 3.5 range, Expand the 40ml culture to 800 ml and distribute 160 ml content in each five 1L flasks. Incubate the flasks at 36±0.5°C and 150±10 rpm until OD of the culture reaches between 2.5 to 3.5 range.

### 4) Inoculation and harvesting of S4 stage (20L Fermenter):

When OD of the S3 stage culture reaches between 2.5 to 3.5 range, pool the culture of all five flasks in seed bottle assembly and inoculate in 20L fermenter. Continue the fermentation using following parameters,

### Fermentation process parameters:

| **Table 1: Fermentation process parameters** | |
|---|---|
| **Parameter** | **Set Point & Range** |
| DO (%) | 37 (30 to 90) |
| Agitation (RPM) | 150 to 500 |
| Air (nl/min) | 2.0 to 10 |
| Oxygen (nl/min) | 0.0 to 15 |
| pH | 7.0 ±0.4 |
| TEMP (°C) | 36.0 ± 2.0 |

Start the Feed media from 3rd hour on wards and increase in proportion with OD. Continue the fermentation for 13 to 15 hrs.

### 5) S4 Stage Formalin Inactivation:

After S4 stage is completed, add formaldehyde solution to the fermenter making the final conc. of 0.5 to 1.0% and incubate the culture at 36°C ± 2°C for 8 to 10 hrs.

### 6) Cell Separation by Centrifugation:

After Inactivation step is completed, clarify the broth by centrifugation and collect the supernatant.

Carryout centrifugation using following parameters,

| **Table 2: Centrifugation parameters** | |
|---|---|
| **Parameter** | **Set value** |
| Temp set point | 2 - 8 °C |
| RPM | 7000 - 8000 |
| Centrifugation time | 40 to 60 min |

### 7) Clarification by Depth Filtration:

After centrifugation is over, supernatant is filtered using depth filter.

### 8) 0.2µ Filtration:

After the depth filtration, the filtrate is subjected to 0.2µ filtration.

### B1. Media optimization for growth of Salmonella typhi S. paratyphi A; S. typhimurium and S. enteritidis.

Following experiments were carried out for Media optimization and batch parameters;
Experiment No.: 01 - Frantz media was selected for the experiment as it is the widely used for growth of Gram negative organisms and evaluated for the growth of Salmonella Typhi. Frantz media was used for development of seed and fermentation. Glucose as carbon source was composed in Feed media.

| **Table 3: Components of Fermentation media for Experiment No.: 01** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |

| **Table 4: Components of Feed media for Experiment No.: 01** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |

Seed was developed in disposable flasks (125ml and 500ml) and inoculated in 2L fermenter. After inoculation, fermentation was carried out in fed-batch mode. Feed was added in fermenter to support the growth of Salmonella Typhi organism. Batch was operated at 36°C temperature, 7.00 pH, 25% dissolved oxygen (DO) and 150 to 500RPM agitation (in cascade mode to maintain DO). Antifoam was added intermittently to control foaming during the fermentation.

### Observations:

| **Table 5: OD₅₉₀ values in Experiment No.: 01** | |
|---|---|
| **Culture Age (Hrs)** | **OD₅₉₀** |
| 0 | 0.0 |
| 1 | 0.12 |
| 2 | 0.295 |
| 3 | 0.49 |
| 4 | 1.43 |
| 5 | 4.33 |
| 6 | 8.73 |
| 7 | 9.24 |
| 8 | 9.7 |
| 9 | 9.68 |
| 10 | 9.7 |
| 11 | 9.57 |
| 12 | 9.3 |
| 13 | 9.2 |
| 14 | 9.5 |

**Conclusion:** Frantz media was found to support the growth of Salmonella typhi organism to limited extent. Further optimization of media was required.

Experiment No.: 02 - Yeast extract was added to the fermentation and feed media to support the growth of cells.

| **Table 6: Components of Fermentation media for Experiment No.: 02** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |

| **Table 7: Components of Feed media for Experiment No.: 02** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |

### Procedure:

Seed was developed in disposable flasks (125ml and 500ml) and inoculated in 2L fermenter. After inoculation, fermentation was carried out in fed-batch mode. Feed were added in fermenter to support the growth of Salmonella Typhi organism. Batch was operated at 36°C temperature, 7.00 pH, 25% dissolved oxygen (DO) and 150 to 500RPM agitation (in cascade mode to maintain DO). Antifoam was added intermittently to control foaming during the fermentation.

### Observations:

| **Table 8: OD₅₉₀ values in Experiment No.: 02** | |
|---|---|
| **Culture Age (Hrs)** | **OD₅₉₀** |
| 0 | 0.0 |
| 1 | 0.12 |
| 2 | 0.295 |
| 3 | 0.49 |
| 4 | 1.43 |
| 5 | 4.33 |
| 6 | 8.73 |
| 7 | 9.24 |
| 8 | 9.7 |
| 9 | 9.68 |
| 10 | 9.7 |
| 11 | 10.1 |
| 12 | 10.6 |
| 13 | 11.2 |
| 14 | 11.8 |

Conclusion: Addition of yeast extract was found to support the growth of *Salmonella typhi* organism to limited extent. Further optimization of media was required.

Experiment No.: 03 - Soya peptone was added to the fermentation and feed media to support the growth of cells.

| **Table 9: Components of Fermentation media for Experiment No.: 03** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |
| Soya peptone | 9 |

| **Table 10: Components of Feed media for Experiment No.: 03** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |
| Soya peptone | 50 |

### Procedure:

Seed was developed in disposable flasks (125ml and 500ml) and inoculated in 2L fermenter. After inoculation, fermentation was carried out in fed-batch mode. Feed was added in fermenter to support the growth of *Salmonella Typhi* organism. Batch was operated at 36°C temperature, 7.00 pH, 25% dissolved oxygen (DO) and 150 to 500RPM agitation (in cascade mode to maintain DO). Antifoam was added intermittently to control foaming during the fermentation.

### Observations:

| **Table 11: OD₅₉₀ values in Experiment No.: 03** | |
|---|---|
| **Culture Age (Hrs)** | **OD₅₉₀** |
| 0 | 0.0 |
| 1 | 0.12 |
| 2 | 0.295 |
| 3 | 0.49 |
| 4 | 1.43 |
| 5 | 4.33 |
| 6 | 8.73 |
| 7 | 9.24 |
| 8 | 9.7 |
| 9 | 9.68 |
| 10 | 9.8 |
| 11 | 10.5 |
| 12 | 11.8 |
| 13 | 12.6 |
| 14 | 13.2 |

Conclusion: Addition of Soya peptone was found to support the growth of *Salmonella typhi* organism. Optimizations of batch parameters for manufacturing were required.

Experiment No.: 04 - Antifoam Ctobe was replaced by antifoam Struktol for improved cell growth.

| **Table 12:Components of Fermentation media for Experiment No.: 04** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |
| Soya peptone | 9 |

| **Table 13: Components of Feed media for Experiment No.: 04** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |
| Soya peptone | 50 |

### Procedure:

Seed was developed in disposable flasks (125ml and 500ml) and inoculated in 2L fermenter. After inoculation, fermentation was carried out in fed-batch mode. Feed was added in fermenter to support the growth of *Salmonella Typhi* organism. Batch was operated at 36°C temperature, 7.00 pH, 25% dissolved oxygen (DO) and 150 to 500RPM agitation (in cascade mode to maintain DO). Antifoam was added intermittently to control foaming during the fermentation.

### Observations:

| **Table 14: OD₅₉₀ values in Experiment No.: 04** | |
|---|---|
| **Culture Age (Hrs)** | **OD₅₉₀** |
| 0 | 0.0 |
| 1 | 0.12 |
| 2 | 0.295 |
| 3 | 0.49 |
| 4 | 1.43 |
| 5 | 4.33 |
| 6 | 8.73 |
| 7 | 9.24 |
| 8 | 10.6 |
| 9 | 11.4 |
| 10 | 12.9 |
| 11 | 13.2 |
| 12 | 13.8 |
| 13 | 14.4 |
| 14 | 15.2 |

Conclusion: Replacement of antifoam C with antifoam Struktol was found to improve the growth of *Salmonella typhi* organism.

Experiment No.: 05 - Growth pattern of *Salmonella typhi* at following fermentation parameters at 20 L scale batch was studied. For batches of *Salmonella typhi,* suitable parameters were defined. Dissolved oxygen and Osmolality were controlled around experimental set points.

| **Table 15: Experimental set points for Experiment No.: 05** | |
|---|---|
| **Parameters** | **Experimental Set Points** |
| pH | 7.0 |
| Temperature (°C) | 36 |
| Dissolved Oxygen (%) | 30 |
| Agitation (rpm) | 150 to 500 |
| Osmolality (mOsmol/kg) | 300 |

| **Table 16:Components of Fermentation media for Experiment No.: 05** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |
| Soya peptone | 9 |

| **Table 17: Components of Feed media for Experiment No.: 05** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |
| Soya peptone | 50 |

**Procedure:** Seed was developed in disposable flasks (250ml and 1L) and inoculated in 20L fermenter. After inoculation, fermentation was carried out in fed-batch mode. Feed was added in fermenter to support the growth of *Salmonella Typhi* organism. Batch was operated at 36°C temperature, 7.00 pH and 150 to 500RPM agitation (in cascade mode to maintain DO). Antifoam was added intermittently to control foaming during the fermentation.

### Observations:

| **Table 18: OD₅₉₀ values in Experiment No.: 05** | |
|---|---|
| **Culture Age (Hrs)** | **OD₅₉₀** |
| 0 | 0.20 |
| 1 | 0.45 |
| 2 | 0.96 |
| 3 | 1.87 |
| 4 | 3.2 |
| 5 | 4.33 |
| 6 | 8.73 |
| 7 | 9.24 |
| 8 | 9.7 |
| 9 | 11.2 |
| 10 | 14.1 |
| 11 | 16.2 |
| 12 | 17.3 |
| 13 | 17.9 |
| 14 | 18.5 |

Conclusion: From the observations, it was concluded that the optimization of parameters mentioned in the table of experimental set points of this experiment were required for growth of *Salmonella typhi* organism.

Experiment No.: 06 - Growth pattern of *Salmonella typhi* at following fermentation parameters at 20L scale batch was studied. For batches of *Salmonella typhi,* suitable parameters were defined. Dissolved oxygen and Osmolality were controlled around experimental set points.

| **Table 19: Experimental set points for Experiment No.: 06** | |
|---|---|
| **Parameters** | **Experimental Set Points** |
| pH | 7.0 |
| Temperature (°C) | 36 |
| Dissolved Oxygen (%) | 40 |
| Agitation (rpm) | 150 to 500 |
| Osmolality (mOsmol/kg) | 400 |

| **Table 20: Components of Fermentation media for Experiment No.: 06** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |
| Soya peptone | 9 |

| **Table 21: Components of Feed media for Experiment No.: 06** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |
| Soya peptone | 50 |

**Procedure:** Seed was developed in disposable flasks (250ml and 1L) and inoculated in 20L fermenter. After inoculation, fermentation was carried out in fed-batch mode. Feed was added in fermenter to support the growth of *Salmonella Typhi* organism. Batch was operated at 36°C temperature, 7.00 pH and 150 to 500RPM agitation (in cascade mode to maintain DO). Antifoam was added intermittently to control foaming during the fermentation.

### Observations:

| **Table 22: OD₅₉₀ values in Experiment No.: 06** | |
|---|---|
| **Culture Age (Hrs)** | **OD₅₉₀** |
| 0 | 0.20 |
| 1 | 0.45 |
| 2 | 0.96 |
| 3 | 1.87 |
| 4 | 3.2 |
| 5 | 4.33 |
| 6 | 8.73 |
| 7 | 11.8 |
| 8 | 15.2 |
| 9 | 17.8 |
| 10 | 19.5 |
| 11 | 20.5 |
| 12 | 21.1 |
| 13 | 21.9 |
| 14 | 22.8 |

Conclusion: From the observations, it was concluded that the optimization of parameters mentioned in the table of experimental set points of this experiment were required for growth of *Salmonella typhi* organism.

Experiment No.: 07 - Growth pattern of *Salmonella typhi* at following fermentation parameters at 20 L scale batch was studied. For batches of *Salmonella typhi,* suitable parameters were defined. Dissolved oxygen and Osmolality were controlled around experimental set points.

| **Table 23: Experimental set points for Experiment No.: 07** | |
|---|---|
| **Parameters** | **Experimental Set Points** |
| pH | 7.0 |
| Temperature (°C) | 36 |
| Dissolved Oxygen (%) | 37 |
| Agitation (rpm) | 150 to 500 |
| Osmolality (mOsmol/kg) | 700 |

| **Table 24: Components of Fermentation media for Experiment No.: 07** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |
| Soya peptone | 9 |

| **Table 25: Components of Feed media for Experiment No.: 07** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |
| Soya peptone | 50 |

**Procedure:** Seed was developed in disposable flasks (250ml and 1L) and inoculated in 20L fermenter. After inoculation, fermentation was carried out in fed-batch mode. Feed was added in fermenter to support the growth of *Salmonella Typhi* organism. Batch was operated at 36°C temperature, 7.00 pH and 150 to 500RPM agitation (in cascade mode to maintain DO). Antifoam was added intermittently to control foaming during the fermentation.

### Observations:

| **Table 26: OD₅₉₀ values in Experiment No.: 07** | |
|---|---|
| **Culture Age (Hrs)** | **OD₅₉₀** |
| 0 | 0.21 |
| 1 | 0.49 |
| 2 | 0.29 |
| 3 | 1.43 |
| 4 | 2.81 |
| 5 | 4.73 |
| 6 | 7.1 |
| 7 | 9.2 |
| 8 | 12.4 |
| 9 | 16.1 |
| 10 | 19.2 |
| 11 | 23.4 |
| 12 | 26.3 |
| 13 | 28.2 |
| 14 | 28.4 |

Conclusion: From the observations, it was concluded that the optimization of parameters mentioned in the table of experimental set points of this experiment were required for growth of *Salmonella typhi* organism.

Experiment No.: 08 - Growth pattern of *Salmonella typhi* at following fermentation parameters at 20 L scale batch was studied. For batches of *Salmonella typhi,* suitable parameters were defined. Dissolved oxygen and Osmolality were controlled around experimental set points.

| **Table 27: Experimental set points for Experiment No.: 08** | |
|---|---|
| **Parameters** | **Experimental Set Points** |
| pH | 7.0 |
| Temperature (°C) | 36 |
| Dissolved Oxygen (%) | 40 |
| Agitation (rpm) | 150 to 500 |
| Osmolality (mOsmol/kg) | 700 |

| **Table 28: Components of Fermentation media for Experiment No.: 08** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |
| Soya peptone | 9 |

| **Table 29: Components of Feed media for Experiment No.: 08** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |
| Soya peptone | 50 |

**Procedure:** Seed was developed in disposable flasks (250ml and 1L) and inoculated in 20L fermenter. After inoculation, fermentation was carried out in fed-batch mode. Feed was added in fermenter to support the growth of *Salmonella Typhi* organism. Batch was operated at 36°C temperature, 7.00 pH and 150 to 500RPM agitation (in cascade mode to maintain DO). Antifoam was added intermittently to control foaming during the fermentation.

### Observations:

| **Table 30: OD₅₉₀ values in Experiment No.: 08** | |
|---|---|
| **Culture Age (Hrs)** | **OD₅₉₀** |
| 0 | 0.20 |
| 1 | 0.66 |
| 2 | 1.62 |
| 3 | 3.2 |
| 4 | 5.3 |
| 5 | 8.1 |
| 6 | 11.2 |
| 7 | 16.3 |
| 8 | 19.2 |
| 9 | 23.8 |
| 10 | 27.4 |
| 11 | 28.8 |
| 12 | 30.1 |
| 13 | 31.2 |
| 14 | 32.4 |

Conclusion: From the observations, it was concluded that the optimization of parameters mentioned in the table of experimental set points of this experiment were required for growth of *Salmonella typhi* organism.

Experiment No.: 09 - Growth pattern of *Salmonella typhi* at following fermentation parameters at 20 L scale batch was studied. For batches of *Salmonella typhi,* suitable parameters were defined. Dissolved oxygen and Osmolality were controlled around experimental set points.

| **Table 31: Experimental set points for Experiment No.: 09** | |
|---|---|
| **Parameters** | **Experimental Set Points** |
| pH | 7.0 |
| Temperature (°C) | 36 |
| Dissolved Oxygen (%) | 40 |
| Agitation (rpm) | 150 to 500 |
| Osmolality (mOsmol/kg) | 500 |

| **Table 32: Components of Fermentation media for Experiment No.: 09** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |
| Soya peptone | 9 |

| **Table 33: Components of Feed media for Experiment No.: 09** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |
| Soya peptone | 50 |

**Procedure:** Seed was developed in disposable flasks (250ml and 1L) and inoculated in 20L fermenter. After inoculation, fermentation was carried out in fed-batch mode. Feed was added in fermenter to support the growth of *Salmonella Typhi* organism. Batch was operated at 36°C temperature, 7.00 pHand 150 to 500RPM agitation (in cascade mode to maintain DO). Antifoam was added intermittently to control foaming during the fermentation.

### Observations:

| **Table 34: OD₅₉₀ values in Experiment No.: 09** | |
|---|---|
| **Culture Age (Hrs)** | **OD₅₉₀** |
| 0 | 0.22 |
| 1 | 0.65 |
| 2 | 2.14 |
| 3 | 4.2 |
| 4 | 6.5 |
| 5 | 11.3 |
| 6 | 14.2 |
| 7 | 18.6 |
| 8 | 26.5 |
| 9 | 29.4 |
| 10 | 32.3 |
| 11 | 36.2 |
| 12 | 37.2 |
| 13 | 38.1 |
| 14 | 38.5 |

Conclusion: From the observations, it was concluded that the optimization of parameters mentioned in the table of experimental set points of this experiment were required for growth of *Salmonella typhi* organism.

**Experiment No.: 10** - Growth pattern of *Salmonella typhi* at following fermentation parameters at 20 L scale batch was studied. For batches of *Salmonella typhi,* suitable parameters were defined. Dissolved oxygen and Osmolality were controlled around experimental set points.

| **Table 35: Experimental set points for Experiment No.: 10** | |
|---|---|
| **Parameters** | **Experimental Set Points** |
| pH | 7.0 |
| Temperature (°C) | 36 |
| Dissolved Oxygen (%) | 37 |
| Agitation (rpm) | 150 to 500 |
| Osmolality (mOsmol/kg) | 500 |

| **Table 36:Components of Fermentation media for Experiment No.: 10** | |
|---|---|
| **Material** | **Qty. (g/L)** |
| D- Glucose monohydrate | 4 |
| Sodium di-hydrogen phosphate monohydrate | 4.1 |
| Di- sodium hydrogen phosphate heptahydrate | 18.8 |
| Magnesium sulfate heptahydrate | 2.5 |
| Yeast extract | 14 |
| Soya peptone | 9 |

| **Table 37: Components of Feed media for Experiment No.: 10** | |
|---|---|
| **Material** | **Qty. (g/L)** |
| D- Glucose monohydrate | 100 |
| Sodium di-hydrogen phosphate monohydrate | 1.025 |
| Di- sodium hydrogen phosphate heptahydrate | 0.94 |
| Magnesium sulfate heptahydrate | 0.625 |
| Yeast extract | 50 |
| Soya peptone | 50 |

**Procedure:** Seed was developed in disposable flasks (250ml and 1L) and inoculated in 20L fermenter. After inoculation, fermentation was carried out in fed-batch mode. Feed was added in fermenter to support the growth of *Salmonella Typhi* organism. Batch was operated at 36°C temperature, 7.00 pH and 150 to 500RPM agitation (in cascade mode to maintain DO). Antifoam was added intermittently to control foaming during the fermentation.

### Observations:

| **Table 38: OD₅₉₀ values in Experiment No.: 10** | |
|---|---|
| **Culture Age (Hrs)** | **OD₅₉₀** |
| 0 | 0.21 |
| 1 | 0.87 |
| 2 | 2.75 |
| 3 | 5.3 |
| 4 | 8.30 |
| 5 | 14.12 |
| 6 | 18.5 |
| 7 | 24.6 |
| 8 | 30 |
| 9 | 33.3 |
| 10 | 38.6 |
| 11 | 42.4 |
| 12 | 43.1 |
| 13 | 43.7 |
| 14 | 44 |

**Conclusion:**

| **Table 39: USP** - **Harvest Yield (in percentage or other unit) obtained for each of the experiments 1 to 10** | | |
|---|---|---|
| **S.No.** | **Description** | **Harvest Yield (mg/L)** |
| 1 | Experiment-1 | 142 mg/L |
| 2 | Experiment-2 | 180 mg/L |
| 3 | Experiment-3 | 210 mg/L |
| 4 | Experiment-4 | 452 mg/L |
| 5 | Experiment-5 | 463 mg/L |
| 6 | Experiment-6 | 480 mg/L |
| 7 | Experiment-7 | 492 mg/L |
| 8 | Experiment-8 | 510 mg/L |
| 9 | Experiment-9 | 537 mg/L |
| 10 | Experiment-10 | 600 mg/L |

Fed-batch mode of cultivation to obtain a high yield harvest of polysaccharide derived from Salmonella serovar strains S. typhi; S. paratyphi A; S. typhimurium and S. enteritidis, by fed-batch process involved the use of combination of an antifoam agent J673 STRUKTOL, soya peptone Difco^{™} Select Phytone^{™} UF at a range of 40 to 70 g/L and yeast extract Difco^{™} Yeast Extract, UF at a range of 40 to 70 g/L during cultivation results in improved harvest yield (100-700 mg/L), and the fermentation parameters comprises of pH maintained in the range of 6.7 to 7.1, temperature maintained in the range of 34.0- 38.0°C, dissolved oxygen level maintained between 36- 39%, Agitation (rpm) maintained between 150 to 500 and osmolality 400 - 600 mOsmol/kg.

### Example 2

### C) The method of purifying the polysaccharide derived from Salmonella serovar strains S. typhi; S. paratyphi A; S. typhimurium and S. enteritidis (DOWNSTREAM PURIFICATION)

### C1) ViPs fermentation harvest subjected to following downstream purification steps to obtain desired quality of Vi-polysaccharide (ViPs):

n) Clarification of a bacterial capsular polysaccharide harvest by direct flow filtration (DFF) through at least one membrane having a pore size of about 0.2 micrometers;
o) concentration by tangential flow ultrafiltration (TFF) and buffer exchange (20mM Tris buffer pH-7.5 containing 5mM EDTA) by diafiltration (DF) using a membrane having 10 0 kDa molecular weight cut off (MWCO);
p) treatment with SDS(20%SDS stock input), ethylene diamine tetra-acetic acid (EDTA) (4 to lOmM) and Sodium acetate (5% to 10%) for denaturation of proteins, nucleic acids and lipopolysaccharide;
q) Ethanol precipitation (40% to 70%);
r) Centrifugation and filtration by direct flow filtration (DFF) through at least one clarification filter having a pore size of about 0.2 µM;
s) treatment with 2M Potassium chloride (KCl) for removal of excess detergent followed by Centrifugation and filtration by direct flow filtration (DFF) through at least one clarification filter having a pore size of about 0.2 µM;
t) concentration by tangential flow filtration (TFF) and buffer exchange by diafiltration (DF) using a membrane having 30kDa molecular weight cut off (MWCO);
u) Selective precipitation of PS by CTAB (12% CTAB stock input);
v) centrifugation and filtration by direct flow filtration (DFF) through at least one clarification filter having a pore size of about 0.45 micrometers to about 0.2 micrometers;
w) Removal of protein and nucleic acid impurities by washing pellet with 60% ethanol (50% to 70%) in presence of 1M NaCl (0.1M to 2M);
x) selective precipitation of polysaccharide by utilizing 60% ethanol (<75% OR>95% );
y) dissolving polysaccharide in WFI or 1M NaCl and subjecting to concentration by tangential flow filtration (TFF) and buffer exchange by diafiltration (DF) using a membrane having 30kDa molecular weight cut off (MWCO); and
z) Sterile Filtration through at least one sterile filter having a pore size of about 0.2 micrometers under sterile conditions and stored at ≤ -20°C

### C2) Salmonella paratyphi O-specific polysaccharide (OSP) of Lipopolysaccharide (LPS) fermentation harvest subjected to following downstream purification steps to obtain desired quality of O-specific polysaccharide from Salmonella Paratyphi A lipopolysaccharide (LPS):

r) Centrifugation at 7000 rpm for 30 min at 4°C, Collected cell pellet (∼1Kg) and supernatant and Cell pellet suspended in 15 L of 1M NaCl and stirred for 1hr at Room temp.
s) concentration by tangential flow ultrafiltration (TFF) and buffer exchange by diafiltration (DF) using 0.45µm Prostak cassette and membrane having 30kDa molecular weight cut off (MWCO);
t) Acid hydrolysis of LPS with 1% Acetic acid (pH~2.8 - 3.0) at 90°C temperature for 180 min
u) The mixture cooled to Room Temperature (RT) & Removal of impurity precipitation by Centrifugation at 7000 rpm for 45 min at 25°C
v) Neutralization Collected the supernatant in glass bottle and neutralized to pH -7.0 with liquor ammonia
w) Clarification by direct flow filtration (DFF) through at least one membrane having a pore size of about 0.45 and 0.2 micrometers;
x) Added Sodium deoxycholate stock solution to get final concentration of 1% and incubated for 30 min under stirring at 30°C temperature, Adjusted pH to 2.0 with Acetic acid and incubated for 15 min under stirring at 30°C
y) Centrifugation at 7000 rpm for 45 min at 25°C
z) Direct flow filtration (DFF) through at least one membrane having a pore size of about 0.45 and 0.2 micrometers;
aa) concentration by tangential flow ultrafiltration (TFF) and buffer exchange by diafiltration (DF) using a membrane having 10 kDa molecular weight cut off (MWCO);
bb) Sterile Filtration through at least one sterile filters having a pore size of about 0.2 micrometers under sterile conditions to obtain Purified O-Specific Polysaccharide (OSP).

| **Table 40: Analytical Methods used for testing at various stages** | | | | | |
|---|---|---|---|---|---|
| **S.No** . | **Test** | **Test method** | **Units** | **References** | **Acceptance criteria** |
| 1 | Vi Polysacchari de content | HPAEC-PAD | mg/mL | WHO TRS 987 | Actual value |
| 2 | Identity | NMR spectroscopy | NA | NA | Confirm the identity /Compara ble to NIBSC Vi Polysaccharide Standard. |
| 3 | Protein impurity | Lowry method | mg/g | IP/BP/Ph Eur 0250/WHO/In-house | Shall contain less than 10.0 mg of Protein per gram of PS |
| 4 | Nucleic acid impurity | UV spectroscopy (Absorbance at 260nm) | mg/g | IP/BP/Ph Eur 0250/WHO/In-house | Shall contain less than 20.0 mg of Nucleic acid per gram of PS |
| 5 | O acetyl content (Lyophilized) | Hestrin's method | mmol/g | IP/BP/Ph Eur 0250/WHO/In-house | Shall be ≥ 2.0 mmol/g polysaccharide |
| 6 | Moisture content | Thermogravimet ry | Percentag e (%) | IP/BP/EP/WHO/I n house | Actual value |
| 7 | Molecular size distribution | SEC-HPLC | Percentag e (%) | IP/BP/EP/WHO/I n-house | At least 50% of polysaccharide shall be eluted before a KD of 0.3 |
| 8 | Endotoxin content | Kinetic turbidometric assay using Limulus amebolysate (LAL) | EU/µg | IP/BP/Ph Eur 0250/WHO/In-house | Less than 100 EU of Endotoxin per µg of PS |
| 9 | pH | Potentiometric | Number | - | Actual value |
| 10 | Bioburden | -- | CFU/mL | IP/BP/WHO/In-house | 100 cfu / 10 mL of PS |
| 11 | Total PS Content | HPAEC-PAD | mg/mL | WHO TRS 987 | Actual value |
| 12 | Average Molecular Weight (AMW) | SE-HPLC | kDa | WHO TRS 987 | Actual value |
| 13 | Identity | NMR | NA | In-house | Comparable to published data/NIBSC STD |
| 14 | Bacterial and mycotic Sterility | Suitable | NA | WHO TRS 987 | Shall be comply |
| 15 | Vi strength | HPAEC-PAD | µg/mL | WHO TRS 987 | 25 µg 10.5 mL |
| 16 | Free Polysaccharid e | HPAEC-PAD-DOC | Percentag e (%) | WHO TRS 987/In-house | < 40% |
| 17 | O-Acetylation | Hestrin | mmol/g | WHO TRS 987/In-house | > 2.0 mmol/g of Vi |
| 18 | Molecular Size Distribution (MSD) | SE-HPLC | Percentag e (%) | WHO TRS 987/In-house | For information (but larger than Vi) K_{D} to be decided |
| 19 | Endotoxin or Pyrogen Content | Kinetic turbidometric assay using Limulus amebolysate (LAL) and Animal Test | EU/µg | WHO TRS 987 | <100 E.U./dose Agreed by NRA |
| 20 | Adjuvant Content and degree of adsorption | Suitable | mg/ml | WHO TRS 987 | Actual value |
| 21 | Preservative Content | Instrument /Suitable Method | mg/ml | WHO TRS 987/In-house | Shall be comply |
| 22 | General Safety (Innocuity) | Animal Test | NA | WHO TRS 987/In-house | Agreed by NRA |
| 23 | pH | Potentiometric | Number | In-house | 6.0 to 8.0 |
| 24 | Osmolality | Osmometer | mOsmol/k g | WHO TRS 987/In-house | Agreed by NRA |

### Results and Interpretation:

| **Table 41: Purified Vi polysaccharide tested by IPQC** | | | | |
|---|---|---|---|---|
| **TEST** | **SPECIFICATION** | **Batch 1** | **Batch 2** | **Batch 3** |
| **Polysaccharide concentration** | Actualvalue (mg/mL) | 3.762 | 3.52 | 3.306 |
| **Identity test by NMR** | Should complies | Complies | Complies | Complies |
| **O-acetyl content** | Not less than 2.0 mmol/g polysaccharide | 2.395 | 2.943 | 3.336 |
| **Protein impurity** | Not more than 1% by weight of PS | 0.797 | 0.568 | 0.907 |
| **Nucleic acid impurity** | Not more than 2% by weight of PS | 0.984 | 1.136 | 1.180 |
| **Endotoxin content** | Not more than 150EU of endotoxin per µg of PS | 16.39 | 52.37 | 45.33 |
| **Molecular size distribution (MSD)** | At least 50% of PS is eluted before a distribution coefficient (K_{D}) of 0.25 is reached. | 93.38 | 94.35 | 94.47 |
| **Free formaldehyde (Residual)** | Not more than 0.02% w/v. | 0.00 | 0.00 | 0.00 |
| **Residual Ethanol** | Not more than 5,000 ppm. | Not detected | Not detected | Not detected |
| **Bioburden** | NMT 100 cfu/10mL of PS | 0 cfu/10mL of PS | 0 cfu/10mL of PS | 0 cfu/10mL of PS |

Improved method of Vi PS purification has clear advantages in terms of both, ease of operation as well as several other advantages such as,
> Improved Polysaccharide purification (in terms of recovery, O-acetyl, endotoxin, protein, nucleic acid content, polydispersity, viscosity) when Sodium acetate (6%) used Vs Sodium acetate (2%)
   **When Sodium acetate (6%) used - Values for**
   DSP % Recovery - 50%
   Ps Viscosity - Data not available
   Ps Polydispersity - Data not available
   O-acetyl - 2.9 mmol/gm of PS
   **When Sodium acetate** (2%) **used** - **Values for**
   DSP % Recovery - 40%
   Ps Viscosity - Data not available
   Ps Polydispersity - Data not available
   O-acetyl - 2.6 mmol/gm of PS
   Sodium acetate reacts with nucleic acids. It breaks up into Na+ and (CH3COO)-. The positively charged sodium ion neutralizes negatively charged PO3- of the nucleic acids; thus helps in precipitation of nucleic acids. So higher concentration i.e. 6% of sodium acetate effectively precipitates host cell impurities like nucleic acids.
> Improved Polysaccharide purification (in terms of recovery, O-acetyl, endotoxin, protein, nucleic acid content, polydispersity, viscosity) CTAB (3%) Vs CTAB (0.5, 1%). CTAB is an amine based cationic quaternary surfactant. It interact with anionic polysaccharide (ionic interaction) and then decreases their solubility hence it precipitates Polysaccharide from the solution. So higher concentration of CTAB (2%) always precipitates higher amounts polysaccharide which gives higher yields and removal of protein impurity is an added advantage.
> Improved Polysaccharide purification (in terms of recovery, O-acetyl, endotoxin, protein, nucleic acid content, polydispersity, viscosity) for current process without DOC Vs DOC based process.
   Advantage of Improved method without DOC process
   1. Regulatory issue: DOC is an animal origin component and is non HALAL compliant. It is manufactured & supplied by a single vendor throughout the world, whereas SDS is a synthetic detergent, HALAL certified and with several suppliers available globally.
   2. Functional issue: DOC breaks down the endotoxins without effecting the chemical composition and once the detergent is removed it can regains its biological activity, whereas SDS owing to its amphipathic nature and higher aggregation number denatures and solubilizes the proteins well and also disrupts endotoxins irreversibly to its monomeric units.
➢ The new improved method of Vi PS purification developed without using Sodium deoxycholate (DOC) which is an animal origin component or Phenol. Improved method based on the reverse purification of polysaccharides in which host cell impurities like proteins, nucleic acids and lipo-polysaccharides will be removed at initial steps by using Sodium acetate (6%), Sodium dodecyl sulfate (2%) and Ethanol (40%) and concentration and diafiltration using 30kDa cut off membranes at different steps, then polysaccharide precipitated by cationic detergent CTAB and performed polishing purification steps to achieve higher yields of Purified Vi Polysaccharide which is meeting WHO specifications.
➢ The purification process results in significant recovery of about 40% to 65% with the desired O-acetyl levels (greater than 2.0 mmol/g polysaccharide), purified Vi polysaccharide yield in the range of 400 to 4000 mg/L, average molecular weight was found to be in the range of 40 to 400 kDa, contains less than 1% proteins/peptides, less than 2% nucleic acids, less than 100 EU of endotoxins per µg of polysaccharide (PS), Molecular size distribution (greater than 50% of PS is eluted before a distribution coefficient (KD) of 0.25 is reached)
➢ The O-specific polysaccharide (OSP) purification process results in significant reduction of endotoxin (< 100EU of endotoxin per µg of PS), protein (< 1%) and nucleic acid (< 2%) impurities, higher recovery of capsular polysaccharide suitably in the range of 40% to 65%, with the desired O-acetyl levels (> 2.0 mmol/g polysaccharide), Molecular size distribution (>50% of PS is eluted before a distribution coefficient (KD) of 0.25 is reached) and average molecular weight of the purified O-specific polysaccharide (OSP) was found to be in the range of 40 to 200 kDa.

### Example 3:

### D) The method of conjugating the polysaccharide derived from Salmonella serovar strains S. typhi; S. paratyphi A; S. typhimurium and S. enteritidis to carrier protein.

The carrier protein used for conjugation with *Salmonella* typhi Vi polysaccharide is tetanus toxoid. The polysaccharides derived from *S. paratyphi A, S. typhimurium* and *S. enteritidis* individually conjugated to a carrier protein selected from tetanus toxoid (TT), diphtheria toxoid (DT) or CRM197.

In accordance with the present disclosure, CRM197 is procured from Recombinant Strain CS463-003 (MB 101) of *Pseudomonas fluorescens* from Pfenex USA.

In accordance with the present disclosure, TT is procured from Clostridium Tetani (Harvard No 49205) obtained from Central research Institute (CRI), National Control Authority, Kasauli, Himachal Pradesh, India. Central research Institute (CRI) procured this strain from NVI, Netherland.

In accordance with the present disclosure, DT is produced cured from cultures of Corynebacterium diphtheriae Park-Williams Number 8 strain, the strain is obtained from Central research Institute (CRI), National Control Authority, Kasauli, Himachal Pradesh, India. Central research Institute (CRI) procured this strain from Wellcome Research Laboratories.

### D1) Preparation of Monovalent Salmonella typhi conjugate using Carbodiimide Chemistry:

### Step 1: Concentration and Derivatization of TT

### Procedure:

a) GFC purified TT (Monomeric Content More than 90%) was concentrated using 10kDa membrane to achieve NLT 12mg/ml Protein concentration (Lowry's assay.)
b) Derivatization ratio was used as given below and calculated the required quantities accordingly.
   Pr:ADH:EDC as 1:6.0:1
   Derivatization Scale: 13gm
c) The Concentrated TT (in 0.9% NaCl), 1M MES pH 6.0, ADH solution (dissolved in 0.1M MES pH 6.0) and EDC solution (dissolved in 0.1M MES pH 6.0) were added sequentially and made final required volume by addition of 0.1M MES buffer pH 6.0. The final Pr conc. in reaction was -4.5mg/ml.
d) The reaction mixture was stirred and allowed to continue about 1 hr at pH 5.90. Then derivatization reaction was quenched by adjusting pH above 7.5 using 0.1M Phosphate buffer containing EDTA pH 8.0.
e) Quenched reaction mixture was diafiltered using 10kDa cut Off membrane against lOmM Phosphate buffer using 22volumes followed by 0.1M MES buffer pH 6.0 at least 12 volumes to remove unreacted moieties and other residuals.
f) The derivatized sample was analyzed for total Pr conc. by Lowry's assay and Extent of derivatization value by colorimetric (TNBS) assay.

### Results:

The Derivatized Pr Conc. by Lowry's assay: 15mg/ml
The Extent of derivatization value (DOA): 19
Percentage recovery: ~75 %

### Step 2: Conjugation of Vi Ps to Derivatization Of TT

### Procedure:

Vi PS-Derivatized TT conjugation performed using Carbodiimide chemistry.

Following parameters of PS and Prwere used for Conjugation.
a. The ratio for conjugation reaction was used Ps:Pr:EDC::1:0.9:1.75 (+ 0.5 for Pr and EDC) Conjugation scale 10gm.
b. The required batch volume of PS was added into pre-sterilize glass bottle.
c. 1M MES buffer pH 6.0 (stock buffer) was added into the measured volume of PS to achieve 0.1M MES conc.
d. The mixture was stirred at ∼100rpm for homogeneous mixing.
e. Then measured volume of Derivatized TT was added into Ps containing bottle.
f. After addition of TT immediatelyfreshly prepared EDC (dissolved in 0.1M MES Buffer pH 6.0) was added into above reaction.
g. pH was observed and recorded (pH 6.0+0.5)
h. The sample was analyzed at different time intervals using SE-HPLC. The conjugation conversion percentage and Protein consumption was monitored.
i. The reaction was quenched after 1.45 hrs by raising pH at 7.5 using 0.1M Phosphate buffer containing EDTA, when <90% protein consumed.
j. The quenched reaction mixture was stored at 2-8°C till further use.

### Step 3: Purification of Quenched Vi-TT Conjugate

Purification of quenched conjugate were performed by two methods carried out to remove unreacted Ps, Unreacted Pr, other residuals using following parameters;
**1. Ultrafiltration method:**
Following parameters used for diafiltration;
Diafiltration Scale: 8.5gm
Ps conc. during D/F: ~2mg/ml
Membrane Cut Off: 300kDa
Membrane Make: Pall
Membrane Area: 2.5m²
Buffer A Used: lOmM PBS least 25volumes
Buffer B Used: 0.9% NaCl 30 volumes.
Final volume: 6.5Lts
Purified Conjugate was filtered using 0.2µM filter.
**2. Gel filtration Chromatography:**
Quenched conjugate about 1.5gm was concentrated on 0.1m2 300kDa cut off membrane ~ 2-4mg/ml. Conjugate purification performed to remove unbound PS, unbound TT and residual EDC using Gel filtration chromatography.
Two separate GFC run were performed with same parameters for purification. Following Chromatographic conditions used for Purification.

| **Table 42: Chromatographic conditions used for Purification of Conjugates** | | |
|---|---|---|
| **Sr. No** | **Parameters** | **Details** |
| 1 | Chromatography used | Gel filtration |
| 2 | Column used | BPG Series |
| 3 | Resin Used | Toyopearl 'HW'65F |
| 4 | Elution buffer | 0.17M NaCl |
| 5 | Operating Linear flow rate | 30cm/hr |
| 6 | Operating Volumetric flow rate | 40ml/min |
| 7 | Sample loading | 3.5% of total bed volume |
| 8 | Fraction collection | 1 min |

1 Total 27 Fractions collected were(100ml each) analyzed on SE-HPLC and pooled together on the basis of chromatographic profile.
2 Fractions were analyzed for Ps content by HPAD method, Pr content by Lowry's method, % free Ps by DOC-HPAD method.
3 Based on analysis Fractions were pooled (1 to 23) and filtered using 0.22µM filter.
4 Sample was analyzed for Ps content, Pr content and free Ps analysis and endotoxin, O-Acetyl content, sterility, pH value and Endotoxin analysis.

### RESULTS AND INTERPRETATION:

| **Table 43: IPQC evaluation of Vi-TT Conjugate Purified** byUltra-filtration **and GFC** | | |
|---|---|---|
| **Test** | **Vi-TT Conjugate Purified by Ultra-filtration** | **Vi-TT Conjugate Purified GFC** |
| Ps Content | 1.43mg/ml | 1.36 mg/ml |
| Pr Content | 1.04 mg/ml | 1.20mg/ml |
| PS/Pr ratio | 1.37 | 1.13 |
| Recovery % | 60 | 52 |
| pH | 6.67 | 5.63 |
| Endotoxin | 0.35Eu/µg | 0.46 Eu/µg |
| | 505.89Eu/ml | 619.61Eu/ml |

**Comparative Conjugation process data-** Improved conjugation efficiency, improved conjugate yield and stability (Free Ps, Free Pr) of SIIPL typhoid conjugate wherein Ps:Pr: EDAC ratio is 1:1:2 Vs Other Ps:Pr: EDAC ratios

**Improved conjugation efficiency:** Conjugation Conversion Percentage observed more than 90%.

**Improved conjugate yield:** The purification performed using different methods i.e Ultrafiltration method and Gel filtration method with variable conjugation purification scale and recovery found 40 to 87%.

**Ratio used 1:1:2** vs 1:1.5:1.2, 1:0.9:2, 1:0.9:1.75, 1:0.8:1.8:, 1:0.7:1.8, 1:0.9:0.6, 1:0.9:1, and 1:0.7:1.5 for Vi-TT Conjugation and it was found that lower protein with 1.5 EDC can give optimum conjugation conversion (%).

It was found that Vi Ps with variable sizes can be conjugated with ADH activated TT.

The used different ratio like Ps:Pr:EDC::1:0.7:1.8, 1:0.8:1.8,1:0.9:2 and 1:1:2 at lower pH at different purification techniques results in more than 0.5 PS/Pr ratio, lower free ps with good recovery of Conjugates.

### D2) Preparation of Monovalent Salmonella paratyphi conjugates:

Two types of conjugation chemistries (cyanylation and carbodiimide chemistry) were applied for the conjugation of the Paratyphi OSP to Carrier protein Diphtheria Toxoid (DT), CRM197 and Tetanus Toxoid (TT):
**1) Cyanylation chemistry based conjugation of the Paratyphi OSP to Carrier protein Diphtheria Toxoid (DT), CRM197 and Tetanus Toxoid (TT)**
   A) Derivatization of Diphtheria Toxoid (DT), (Addition of Linker ADH) using carbodiimide chemistry and conjugation with Concentrated OSP using cyanylation chemistry
   B) Derivatization of CRM197, Tetanus Toxoid (TT) (Addition of Linker ADH) using carbodiimide chemistry and conjugation with Concentrated OSP using cyanylation chemistry
   C) Derivatization of Tetanus Toxoid (TT) (Addition of Linker ADH) using carbodiimide chemistry and conjugation with Concentrated OSP using cyanylation chemistry

### 1A) Preparation of S. Paratyphi Conjugates using Diphtheria Toxoid as carrier protein. 1) Protein derivatization:

High Monomeric Diphtheria toxoid (DT) was concentrated to (10-20mg/ml) on a 10kDa membrane and analysed for protein content

| **Table 44: The protein content measured by BCA (Bicinchoninic acid) assay.** | |
|---|---|
| **Test Sample** | **Total Protein mg/ml** |
| Concentrated DT | 12.27 |

To the concentrated DT freshly prepared 1M MES Buffer was added and Adipic acid dihydrazide (ADH) (dissolved 75-100mg/ml in 100mM MES buffer pH:5.8) in the 1:10 by weight ratio and EDAC (1-ethyl-3-(3-dimethyl-aminopropyl) carbodiimide) (dissolved 30-40 mg/ml in 100mM MES Buffer pH:5.8) in the 1:1 by weight ratio. The reaction was continued at 5.8 pH for about 1Hrs and then the reaction mixture was diafiltered on 10 kDa TFF in 50mM Borate buffer pH 9.0 to remove residuals and unreacted components. The final sample was analysed for protein content and degree of derivatization

| **Table 45: Degree of Derivatization by TNBS assay** | | |
|---|---|---|
| **Test Sample** | **Total Protein mg/ml** | **DOD** |
| ADH Derivatized DT | 40 | 16.40 |

### 2) Conjugation of S. Paratyphi A polysaccharide (OSP) and ADH Derivatized DT:

### Two Experiments were performed by change in 1-cyano- 4-pyrrolidinopyridinium tetrafluoroborate (CPPT) (CPIP) ratio

**Expt No:1** - The OSP was concentrated on 10 kDa membrane to achieve a concentration of (10-15mg/ml).

| **Table 46: The Polysaccharide content was analysed by Anthrone assay** | |
|---|---|
| **Test Sample** | **Total PS mg/ml** |
| Concentrated OSP | 13.48 |

To the Concentrated PS 0.9% NaCl was added and Freshly prepared CPIP solution (114mg/ml in acetonitrile) was added into polysaccharide in the 1:1.3 by weight ratio. The pH was shifted to 9.5 immediately with 2.5M NaOH and held for up to 3 min. Then protein was added in 1:0.8 by weight ratio **PS: PR: CPIP as 1:0.8:1.3**

Shodex columns SB-804 HQ and SB-805 HQ were used sequentially with PBS as mobile phase at 1 ml/min flow rate tomonitored for protein conversion. The reaction was quenched after 3-4hours by adding 2M Glycine 10 times to that of PS by weight.

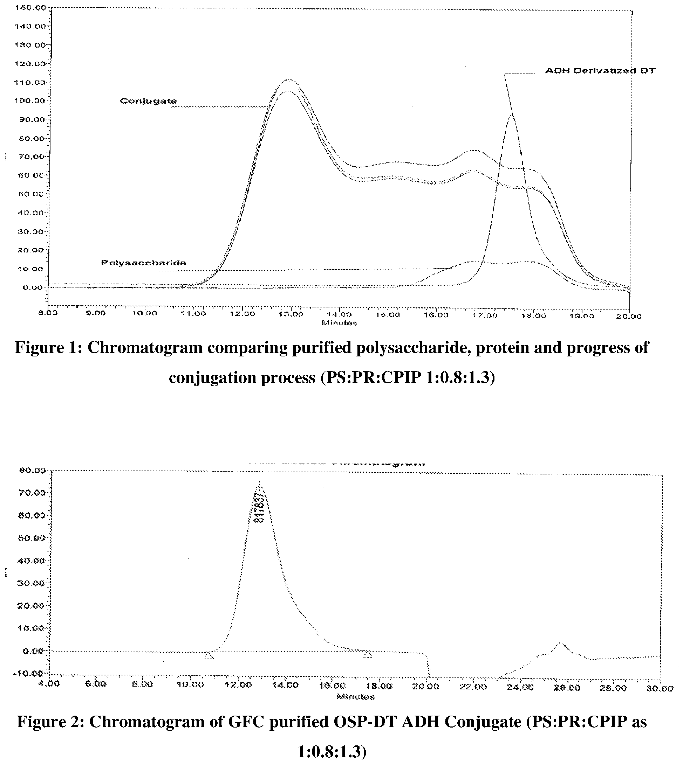

### Refer Figure 1: Comparison of Polysaccharide, Protein and Conjugate (PS: PR: CPIP 1:0.8:1.3)

**3) Conjugate Purification by GFC:** A Column XK16/70 was made ready using GFC resin (Tyopearl HW65F) with a bed height of 40cm. The column was packed at a flow rate of 100cm/hr and allowed to settle and 1M NaCl 1% of the column volume was passed to assess the integrity of the packed column. The column was equilibrated using 0.9% NaCl at 30cm/hr and the conjugate was loaded on the column and fractions collected at 1min interval. **Fractions 2-9** were pooled according to the profile on HPLC. The final pooled fractions were filtered and sent for analysis

| **Table 47: Evaluation of Purified OSP-DT ADH Conjugate (PS:PR:CPIP as 1:0.8:1.3)** | | | |
|---|---|---|---|
| **Test Sample** | **TPS (mg/ml)** | **TPr (mg/ml)** | **Ratio (Ps/pr)** |
| OSP-DT ADH Conjugate 1 | 0.215 | 0.210 | 1.02 |

### Refer Figure 2: Chromatogram of GFC purified OSP-DT ADH Conjugate (PS:PR:CPIP as 1:0.8:1.3)

### Expt No: 2 -

To the Concentrated PS 0.9% NaCl was added and Freshly prepared CPIP solution (114mg/ml in acetonitrile) was added into polysaccharide in the 1:1.1 by weight ratio. The pH was shifted to 9.5 immediately with 2.5M NaOH and held for up to 3 min. Then protein was added in 1:0.8 by weight ratio PS:PR:CPIP as **1:0.8:1.1.**

Shodex columns SB-804 HQ and SB-805 HQ were used sequentially with PBS as mobile phase at 1 ml/min flow rate to monitored for protein conversion. The reaction was quenched after 3-4hours by adding 2M Glycine 10 times to that of PS by weight.

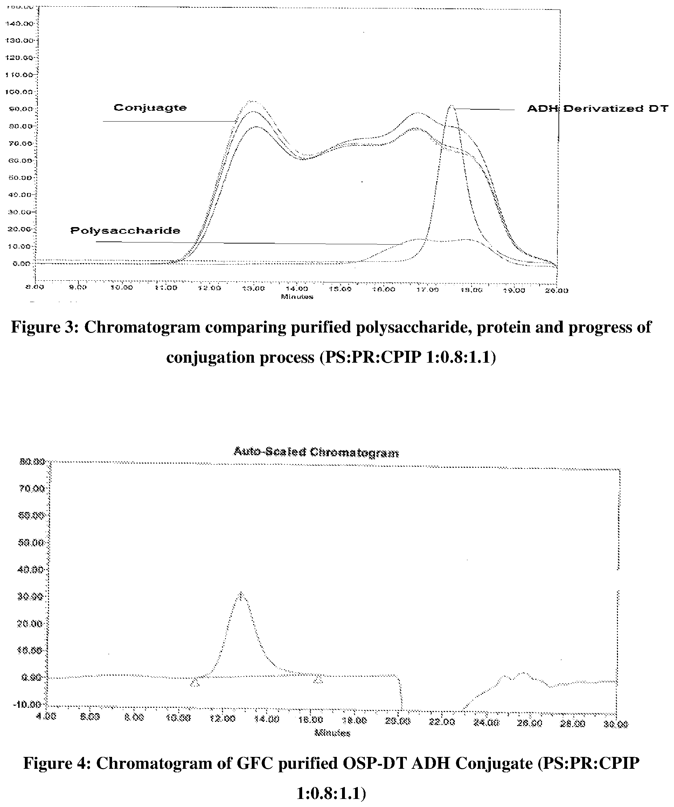

### Refer Figure 3: Comparison of Polysaccharide, Protein and Conjugate (PS:PR:CPIP 1:0.8:1.1)

**Conjugate Purification by GFC:** A Column XK16/70 was made ready using GFC resin (Tyoperal HW65F) with a bed height of 40cm. The column was packed at a flow rate of 100cm/hr and allowed to settle and 1M NaCL 1% of the column volume was passed to assess the integrity of the packed column. The column was equilibrated using 0.9% NaCL at 30cm/hr and the conjugate was loaded on the column and fractions were collected at 1min interval. **Fractions 2-8** were pooled according to the profile on HPLC. The final pooled fractions were filtered and sent for analysis

| **Table 48: Evaluation of Purified OSP-DT ADH Conjugate(PS:PR:CPIP 1:0.8:1.1)** | | | |
|---|---|---|---|
| **Test Sample** | **TPS (mg/ml)** | **TPr (mg/ml)** | **Ratio (Ps/pr)** |
| OSP-DT ADH Conjugate 2 | 0.113 | 0.100 | 1.13 |

### Refer Figure 4: Chromatogram of GFC purified OSP-DT ADH Conjugate (PS:PR:CPIP 1:0.8:1.1)

**Conclusion:** Conjugation of Paratyphi A PS using ADH derivatized DT was found successful and the PS/PR ratio was found satisfactory.

### 1B. Preparation of S.Paratyphi Conjugates using CRM197 as carrier protein

### 1) Protein derivatization: Cross reacting mutant (CRM197) received from Production department (SIIPL) was taken for derivatization

| **Table 49: The protein content measured by BCA (Bicinchoninic acid) assay.** | |
|---|---|
| **Test Sample** | **Total Protein mg/ml** |
| Concentrated CRM197 | 32 |

To the concentrated CRM197 freshly prepared 1M MES Buffer was added and Adipic acid dihydrazide (ADH) (dissolved 75-100mg/ml in 100mM MES buffer pH:6.5) in the 1:3.5 by weight ratio and EDAC (1-ethyl-3-(3-dimethyl-aminopropyl) carbodiimide) (dissolved 30-40 mg/ml in 100mM MES Buffer pH:6.5) in the 1:0.25 by weight ratio. 5% Tween 80 was added. The final reaction volume was made up using 100mM MES Buffer to achieve a final concentration of 3-4mg/ml the reaction was continued at 6.5 pH for about 3Hrs and then the reaction mixture was diafiltered on 10 kDa TFF in 50mM Borate buffer and 0.005% Tween 80 pH 9.0 to remove residuals and unreacted components. The final sample was analysed for protein content and degree of derivatization

**Brief description of assay or reference:** The protein content was measured by BCA (Bicinchoninic acid) assay.

| **Table 50: Degree of Derivatization by TNBS assay** | | |
|---|---|---|
| **Test Sample** | **Total Protein mg/ml** | **DOD** |
| ADH Derivatized CRM197 | 32 | 6.74 |

### 2) Conjugation of S.Paratyphi A polysaccharide (OSP) and ADH Derivatized CRM197:

The OSP received from DSP was concentrated on 10 kDa membrane to achieve a concentration of (10-15mg/ml).

| **Table 51: The Polysaccharide content analysed by Anthrone assay** | |
|---|---|
| **Test Sample** | **Total PS mg/ml** |
| Concentrated OSP | 11.7 |

To the Concentrated PS 0.9% NaCl was added and Freshly prepared CPIP solution (114mg/ml in acetonitrile) was added into polysaccharide in the 1:1.3 by weight ratio. The pH was shifted to 9.5 immediately with 2.5M NaOH and held for up to 3 min. Then protein was added in 1:1 by weight ratio PS:PR:CPIP as 1:1:1.3

Shodex columns SB-804 HQ and SB-805 HQ were used sequentially with PBS as mobile phase at 1 ml/min flow rate to monitored for protein conversion. The reaction was quenched after 3-4hours by adding 2M Glycine 10 times to that of PS by weight.

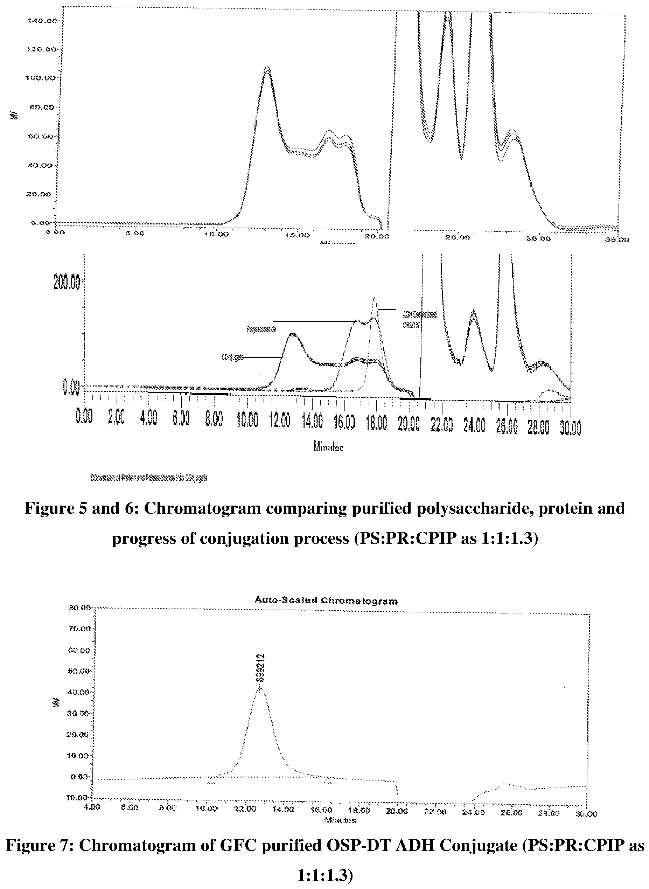

### Refer Figure 5 and 6: Comparison of Polysaccharide, Protein and Conjugate (PS:PR:CPIP as 1:1:1.3)

**3) Conjugate Purification by GFC:** A Column XK16/70 was made ready using GFC resin (Tyoperal HW65F) with a bed height of 40cm. The column was packed at a flow rate of 100cm/hr and allowed to settle and 1M NaCL 1% of the column volume was passed to assess the integrity of the packed column. The column was equilibrated using 0.9% NaCL at 30cm/hr and the conjugate was loaded on the column and fractions collected at 1min interval, Fractions 2-7 were pooled according to the profile on HPLC. The final pooled fractions were filtered and sent for analysis

| **Table 52: Evaluation of Purified OSP-DT ADH Conjugate(PS:PR:CPIP 1:1:1.3)** | | | |
|---|---|---|---|
| **Test Sample** | **TPS (mg/ml)** | **TPr (mg/ml)** | **Ratio (Ps/pr)** |
| OSP-CRM ADH Conjugate | 0.150 | 0.220 | 0.681 |

### Refer Figure 7: Chromatogram of GFC purified OSP-DT ADH Conjugate (PS:PR:CPIP as 1:1:1.3)

**Conclusion:** Conjugation of Paratyphi A PS using ADH derivatized CRM197 was found successful and the PS/PR ratio was found satisfactory.

### 1C) Preparation of S.Paratyphi Conjugates using Tetanus Toxoid as carrier protein:

### 1) Protein derivatization: High Monomeric Tetanus toxoid (TT) received from Production department (SIIPL) was concentrated to (15-20mg/ml) on a 30 kDa membrane and analysed for protein content

| **Table 53: The protein content measured by Lowry assay** | |
|---|---|
| **Test Sample** | **Total Protein mg/ml** |
| Concentrated TT | 16.29 |

The concentrated TT freshly prepared 1M MES Buffer was added and Adipic acid dihydrazide (ADH) (dissolved 75-100mg/ml in 100mM MES buffer pH:6.0)in the 1:10 by weight ratio and EDAC (1-ethyl-3-(3-dimethyl-aminopropyl) carbodiimide) (dissolved 30-40 mg/ml in 100mM MES BufferpH:6.0) in the 1:1 by weight ratio. The reaction was continued at 6.0 pH for about 1Hrs and then the reaction mixture was diafiltered on 30 kDa TFF in lOmM Phosphate buffer pH 7.2 to remove residuals and unreacted components. The final sample was analysed for protein content and degree of derivatization

**Brief description of assay or reference:** The protein content was measured by Lowry assay.

| **Table 54: Degree of Derivatization by TNBS assay** | | |
|---|---|---|
| **Test Sample** | **Total Protein mg/ml** | **DOD** |
| ADH Derivatized TT | 16.29 | 12.9 |

### 2) Conjugation of S.Paratyphi A polysaccharide(OSP) and ADH Derivatized TT:

The OSP received from DSP Team was concentrated on 10 kDa membrane to achieve a concentration of (10-13mg/ml).

| **Table 55:The Polysaccharide content analysed by Anthrone assay** | |
|---|---|
| **Test Sample** | **Total PS mg/ml** |
| Concentrated OSP | 13.20 |

### Two Experiments were performed by change in 1-cyano- 4-pyrrolidinopyridinium tetrafluoroborate (CPPT) (CPIP) ratio

### Expt No:1

To the Concentrated PS 0.9% NaCl was added and freshly prepared CPIP solution (114mg/ml in acetonitrile) was added into polysaccharide in the 1:1.25 by weight ratio. The pH was shifted to 9.5 immediately with 2.5M NaOH and held for up to 3 min. Then protein was added in 1:0.8 by weight ratio PS:PR:CPIP as**1:0.8:1.25**

Shodex columns SB-804 HQ and SB-805 HQ were used sequentially with PBS as mobile phase at 1 ml/min flow rate tomonitored for protein conversion. The reaction was quenched after 3-4hours by adding 2M Glycine 10 times to that of PS by weight.

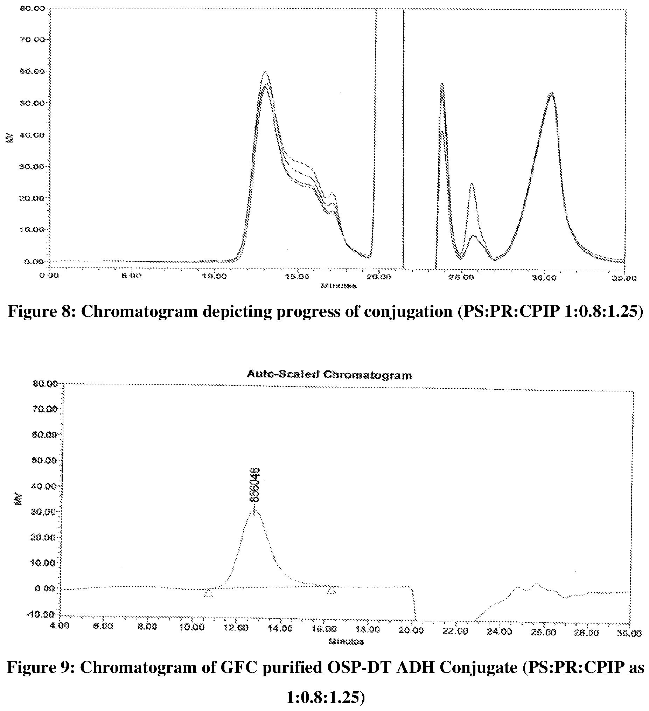

### Refer Figure 8: Comparison of Polysaccharide, Protein and Conjugate (PS:PR:CPIP 1:0.8:1.25)

**3) Conjugate Purification by Ultrafiltration:** Quenched conjugate was purified by diafiltration 300 kDa TFF membrane in lOmM PBS pH 7.2 followed by lOmM Tris Buffer pH7.2 The final concentrated sample was sent for analysis.

| **Table 56: Evaluation of Purified OSP-DT ADH Conjugate (PS:PR:CPIP as 1:0.8:1.25)** | | | |
|---|---|---|---|
| **Test Sample** | **TPS (mg/ml)** | **TPr (mg/ml)** | **Ratio(Ps/pr)** |
| OSP-TT ADH Conjugate | 0.098 | 0.120 | 0.816 |

### Refer Figure 9: Chromatogram of GFC purified OSP-DT ADH Conjugate

### (PS:PR:CPIP as 1:0.8:1.25)

### Expt No: 2

To the Concentrated PS 0.9% NaCl was added and Freshly prepared CPIP solution (114mg/ml in acetonitrile) was added into polysaccharide in the 1:1.3 by weight ratio. The pH was shifted to 9.5 immediately with 2.5M NaOH and held for up to 3 min. Then protein was added in 1:0.9 by weight ratio PS:PR:CPIP1:0.9:1.3

Shodex columns SB-804 HQ and SB-805 HQ were used sequentially with PBS as mobile phase at 1 ml/min flow rate tomonitored for protein conversion. The reaction was quenched after 3-4hours by adding 2M Glycine 10 times to that of PS by weight.

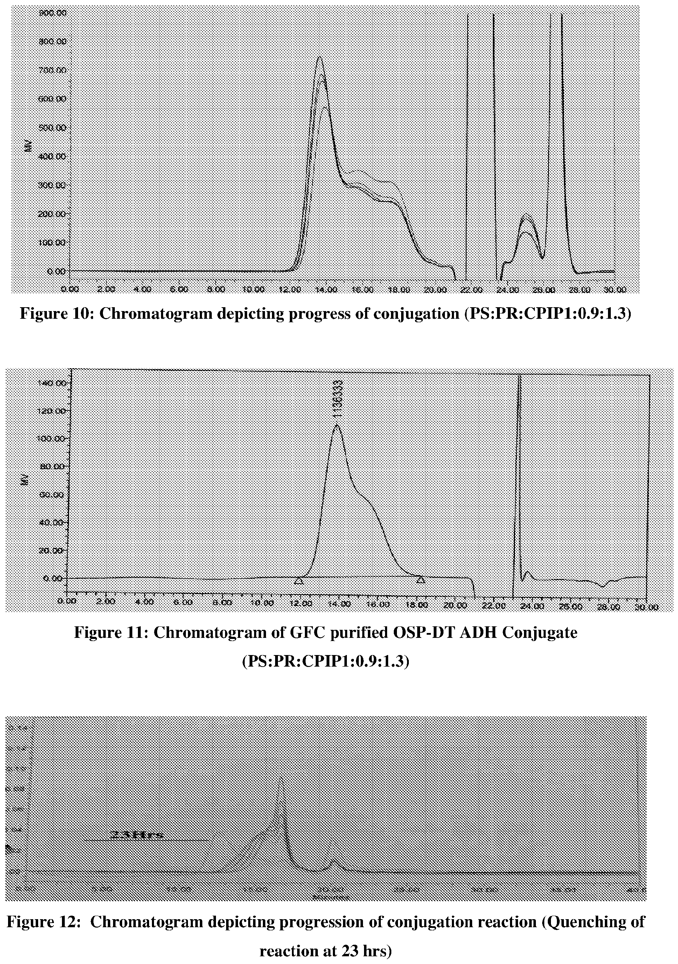

### Refer Figure 10: Comparison of Polysaccharide, Protein and Conjugate

### (PS:PR:CPIP1:0.9:1.3)

3) Conjugate Purification by Ultrafiltration: Quenched conjugate was purified by diafiltration 300 kDa TFF membrane in lOmM PBS pH7.2 followed by 10mM Tris Buffer pH7.2 The final concentrated sample was sent for analysis.

| **Table 57: Evaluation of Purified OSP-DT ADH Conjugate(PS:PR:CPIP1:0.9:1.3)** | | | |
|---|---|---|---|
| **Test Sample** | **TPS (mg/ml)** | **TPr (mg/ml)** | **Ratio(Ps/pr)** |
| OSP-TT ADH Conjugate | 0.025 | 0.035 | 0.715 |

### Refer Figure 11: Chromatogram of GFC purified OSP-DT ADH Conjugate (PS:PR:CPIP1:0.9:1.3)

Conclusion: Conjugation of Paratyphi A PS using ADH derivatized TT was found successful, two different types of diafiltration strategy were applied and both the process gave satisfactory PS/Pr ratio.

### 2) Carbodiimide chemistry based conjugation of the Paratyphi OSP to Carrier protein Diphtheria Toxoid (DT), CRM197 and Tetanus Toxoid (TT)

A) Derivatization of Concentrated OSP (Addition of Linker ADH) using cyanylation chemistry and conjugation with Tetanus Toxoid (TT) using carbodiimide chemistry.
B) Derivatization of Concentrated OSP (Addition of Linker ADH) using cyanylation chemistry and conjugation with Diphtheria Toxoid (DT) using carbodiimide chemistry.
C) Derivatization of Concentrated OSP (Addition of Linker ADH) using cyanylation chemistry and conjugation with CRM197 using carbodiimide chemistry.

### 2A) Preparation of S.Paratyphi Conjugates using Tetanus Toxoid as carrier protein.

**1) Polysaccharide derivatization:** The OSP received from DSP Team was concentrated on 10 kDa membrane to achieve a concentration of (10-13mg/ml).

| **Table 58:The Polysaccharide content analysed by Anthrone assay** | |
|---|---|
| **Test Sample** | **Total PS mg/ml** |
| Concentrated OSP | 13.20 |

To the Concentrated PS 0.9% NaCl was added and freshly prepared CPIP solution (114mg/ml in acetonitrile) was added into polysaccharide in the 1:0.7 by weight ratio. The pH was shifted to 9.5 immediately with 2.5M NaOH and held for up to 3 min. Then Adipic acid dihydrazide (ADH) (dissolved 75-100mg/ml in 0.5M Sodium bicarbonate buffer pH: 8.0) in the 1:10 by weight ratio PS: ADH: CPIP as 1:10:0.7 The reaction was continued at 9.5 pH for about 2Hrs, quenched using 2M Glycine 10 and then the reaction mixture was diafiltered on 10 kDa TFF in 100mM MES buffer pH 6.0 to remove residuals and unreacted components. The final sample was analysed for polysaccharide content

| **Table 59:The Polysaccharide content analysed by Anthrone assay** | |
|---|---|
| **Test Sample** | **PS Content (mg/ml)** |
| PS Content | 20.7 |

**2) Protein Preparation:** High Monomeric Tetanus toxoid (TT) received from Production department (SIIPL) was concentrated to (10-15mg/ml) on a 30 kDa membrane and analysed for protein content
**Brief description of assay or reference:** The protein content was measured by Lowry assay.

| **Table 60: The protein content measured by Lowry assay** | |
|---|---|
| **Test Sample** | **Total Protein mg/ml** |
| Concentrated TT | 15.3 |

**3) Conjugation of ADH Derivatized S.Paratyphi A polysaccharide (OSP) and Concentrated TT:** Two experiments were performed at different temperatures to check the Pr conversion.

### Expt No :1

To the ADH derivatized PS, protein was added in 1:0.9 by weight and freshly prepared EDAC (1-ethyl-3-(3-dimethyl-aminopropyl) carbodiimide) (dissolved 30-40 mg/ml in 100mM MES Buffer) in the 1:0.86 by weight ratio. The reaction was continued at 6.0 pH **temperature 6°C ratio PS:PR:EDC as 1:0.9:0.86**

Shodex columns SB-804 HQ and SB-805 HQ were used sequentially with PBS as mobile phase at 1 ml/min flow rate tomonitored for protein conversion. The reaction was quenched after **23 hours** by adding 100mM Phosphate buffer containing EDTA.

### Refer Figure 12: Chromatogram depicting progression of conjugation reaction (Quenching of reaction at 23 hrs)

**4) Conjugate Purification by Ultrafiltration:** Quenched conjugate was purified by diafiltration 300 kDa TFF membrane in lOmM PBS pH7.2 followed by 10mM Tris Buffer pH7.2 The final concentrated sample was sent for analysis.

| **Table 61: Evaluation of Purified OSP ADH-TT Conjugate(PS:PR:EDC 1:0.9:0.86)** | | | |
|---|---|---|---|
| **Test Sample** | **TPS (mg/ml)** | **TPr (mg/ml)** | **Ratio (Ps/pr)** |
| ADH OSP-TT Conjugate | 0.013 | 0.200 | 0.06 |

### Expt No : 2

To the ADH derivatized PS, protein was added in 1:0.9 by weight and freshly prepared EDAC (1-ethyl-3-(3-dimethyl-aminopropyl) carbodiimide) (dissolved 30-40 mg/ml in 100mM MES Buffer) in the 1:0.86 by weight ratio. The reaction was continued at 6.0 pH, **temperature 10°C ratio PS:PR:EDC as 1:0.9:0.86**

Shodex columns SB-804 HQ and SB-805 HQ were used sequentially with PBS as mobile phase at 1 ml/min flow rate tomonitored for protein conversion. The reaction was quenched after **4 hours** by adding 100mM Phosphate buffer containing EDTA.

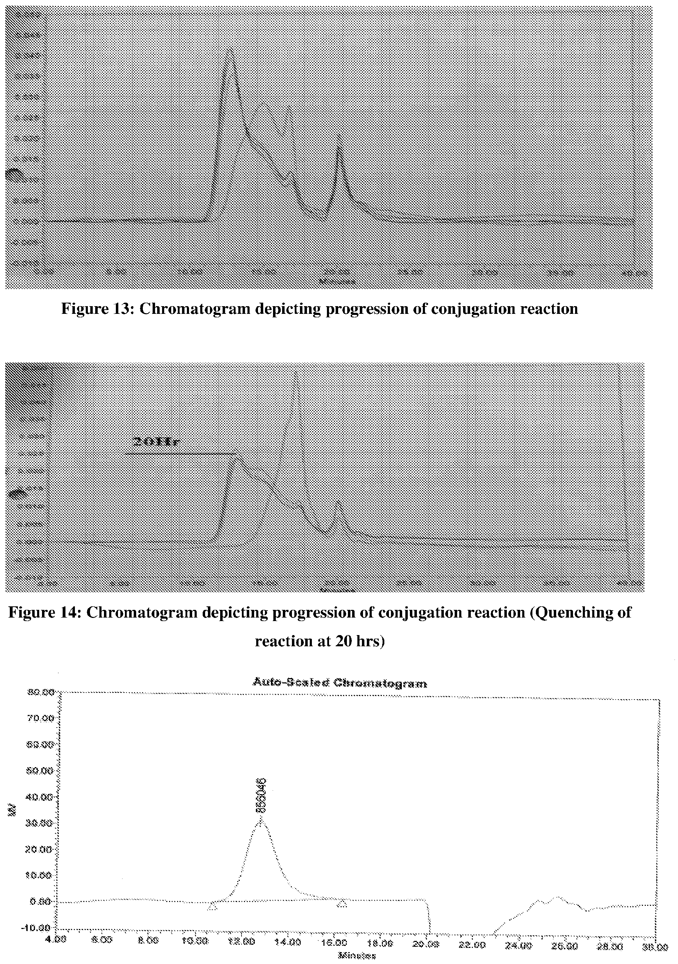

### Refer Figure 13: Chromatogram depicting progression of conjugation reaction

**4) Conjugate Purification by GFC:** A Column XK16/70 was made ready using GFC resin (Tyoperal HW65F) with a bed height of 40cm. The column was packed at a flow rate of 100cm/hr and allowed to settle and 1M NaCl 1% of the column volume was passed to assess the integrity of the packed column. The column was equilibrated using 0.9% NaCl at 30cm/hr and the conjugate was loaded on the column and fractions collected at 1min interval, Fractions 2-10 were pooled according to the profile on HPLC. The final pooled fractions were filtered and sent for analysis

| **Table 62: Evaluation of Purified ADH OSP-TT Conjugate** | | | |
|---|---|---|---|
| **Test Sample** | **TPS (mg/ml)** | **TPr (mg/ml)** | **Ratio (Ps/pr)** |
| ADH OSP-TT Conjugate | 0.017 | 0.270 | 0.06 |

**Conclusion:** Using the reverse conjugation chemistry (Activating PS), Conjugation of ADH derivatized Paratyphi A PS and concentrated TT was found successful, by increase in temperature from 6 to 10°C. Conjugation rate of reaction was increased, PS/Pr ratio was very low in both the reactions.

### 2B) Preparation of S.Paratyphi Conjugates using Diptheria Toxoid as carrier protein.

**1) Polysaccharide derivatization:** The OSP received from DSP Team was concentrated on 10 kDa membrane to achieve a concentration of (10-13mg/ml).

| **Table 63:The Polysaccharide content analysed by Anthrone assay** | |
|---|---|
| **Test Sample** | **Total PS mg/ml** |
| Concentrated OSP | 10.09 |

To the Concentrated PS 0.9% NaCl was added and Freshly prepared CPIP solution (0.114mg/ml in acetonitrile) was added into polysaccharide in the 1:0.7 by weight ratio. The pH was shifted to 9.5 immediately with 2.5M NaOH and held for up to 3 min. Then Adipic acid dihydrazide (ADH) (dissolved 75-100mg/ml in 0.5M Sodium bicarbonate buffer pH:8.0) in the 1:10 by weight ratio PS:ADH:CPIP as 1:10:0.7 The reaction was continued at 9.5 pH for about 2Hrs,quenched using 2M Glycine 10 and then the reaction mixture was diafiltered on 10 kDa TFF in 100mM MES buffer pH 6.0 to remove residuals and unreacted components. The final sample was analysed for polysaccharide content

| **Table 64:The Polysaccharide content analysed by Anthrone assay** | |
|---|---|
| **Test Sample** | **PS Content (mg/ml)** |
| PS Content | 18.20 |

**2) Protein Preparation:** High Monomeric Diptheria toxoid (DT) received from Production department (SIIPL) was concentrated to (10-20mg/ml) on a 10 kDa membrane and analysed for protein content
**Brief description of** assay **or reference:** The protein content was measured by Lowry assay.

| **Table 65: The protein content measured by Lowry assay** | |
|---|---|
| **Test Sample** | **Total Protein mg/ml** |
| Concentrated DT | 16.09 |

**3) Conjugation of ADH Derivatized S.Paratyphi A polysaccharide(OSP) and Concentrated DT:** Same conditions of TT conjugate were applied to DT to check the Pr Conversion.

### Expt No :1

To the ADH derivatized PS, protein was added in 1:0.8 by weight and freshly prepared EDAC (1-ethyl-3-(3-dimethyl-aminopropyl) carbodiimide) (dissolved 30-40 mg/ml in 100mM MES Buffer) in the 1:0.86 by weight ratio. The reaction was continued at 6.0 pH

### temperature 6°C ratio PS:PR:EDC as 1:0.8:0.86

Shodex columns SB-804 HQ and SB-805 HQ were used sequentially with PBS as mobile phase at 1 ml/min flow rate tomonitored for protein conversion. The reaction was quenched after 20 hours by adding 100mM Phosphate buffer containing EDTA.

### Refer Figure 14: Chromatogram depicting progression of conjugation reaction

### (Quenching of reaction at 20 hrs)

4) Conjugate Purification by GFC: A Column XK16/70 was made ready using GFC resin (Tyoperal HW65F) with a bed height of 40cm. The column was packed at a flow rate of 100cm/hr and allowed to settle and 1M NaCl 1% of the column volume was passed to assess the integrity of the packed column. The column was equilibrated using 0.9% NaCl at 30cm/hr and the conjugate was loaded on the column and fractions collected at lmin interval, Fractions 2-12 were pooled according to the profile on HPLC. The final pooled fractions were filtered and sent for analysis

| **Table 66: Evaluation of Purified OSP ADH-DT Conjugate(PS:PR:EDC 1:0.9:0.86)** | | | |
|---|---|---|---|
| **Test Sample** | **TPS (mg/ml)** | **TPr (mg/ml)** | **Ratio (Ps/pr)** |
| ADH OSP-DT Conjugate | 0.020 | 0.300 | 0.060 |

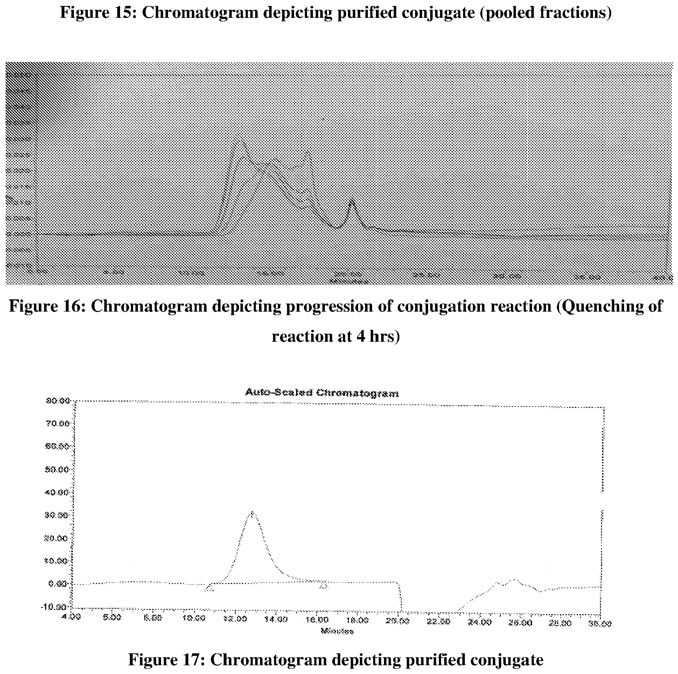

### Refer Figure 15: Chromatogram depicting purified conjugate (pooled fractions)

### Expt No :2

To the ADH derivatized PS, protein was added in 1:1.0 by weight and freshly prepared EDAC (1-ethyl-3-(3-dimethyl-aminopropyl) carbodiimide) (dissolved 30-40 mg/ml in 100mM MES Buffer) in the 1:0.9 by weight ratio. The reaction was continued at 6.0 pH, **temperature 10°C ratio PS:PR:EDC as 1:1.0:0.9**

Shodex columns SB-804 HQ and SB-805 HQ were used sequentially with PBS as mobile phase at 1 ml/min flow rate tomonitored for protein conversion. The reaction was quenched after **4 hours** by adding 100mM Phosphate buffer containing EDTA.

### Refer Figure 16: Chromatogram depicting progression of conjugation reaction

### (Quenching of reaction at 4 hrs)

4) Conjugate Purification by Ultrafiltration: Quenched conjugate was purified by diafiltration 300 kDa TFF membrane in lOmM PBS pH 7.2 followed by lOmM Tris Buffer pH 7.2 The final concentrated sample was sent for analysis.

### Refer Figure 17: Chromatogram depicting purified conjugate

| **Table 67: Evaluation of Purified ADH OSP-DT Conjugate** | | | |
|---|---|---|---|
| **Test Sample** | **TPS (mg/ml)** | **TPr (mg/ml)** | **Ratio (Ps/pr)** |
| ADH OSP-DT Conjugate | 0.019 | 0.230 | 0.08 |

**Conclusion:** The Conjugation of ADH derivatized Paratyphi A PS using Carbodiimide chemistry was found successful using concentrated DT. Rate of conjugation reaction was increased by increasing the temperature but PS/Pr ratio was low in both experiments

### D3) The method of conjugating the polysaccharide derived from Salmonella serovar strains S. typhimurium and S. enteritidis to carrier protein selected from tetanus toxoid (TT), diphtheria toxoid (DT) or CRM197.

### 1) Cyanylation chemistry based conjugation of the S. typhimurium and S. enteritidis polysaccharide to Carrier protein Diphtheria Toxoid (DT), CRM197 and Tetanus Toxoid (TT)

A) Derivatization of Diphtheria Toxoid (DT), (Addition of Linker ADH) using carbodiimide chemistry and conjugation with Concentrated S. *typhimurium and S. enteritidis* polysaccharide using cyanylation chemistry
B) Derivatization of CRM197, (Addition of Linker ADH) using carbodiimide chemistry and conjugation with Concentrated S. *typhimurium and S. enteritidis* using cyanylation chemistry
C) Derivatization of Tetanus Toxoid (TT) (Addition of Linker ADH) using carbodiimide chemistry and conjugation with Concentrated S. *typhimurium and S. enteritidis* using cyanylation chemistry
D) Cyanylation chemistry (CPPT) based conjugation of the Paratyphi OSP to Carrier protein Diphtheria Toxoid (DT), CRM197 and Tetanus Toxoid (TT) without derivatization of Polysaccharide or Carrier protein.

### Procedure followed:

### A) Preparation of S. typhimurium and S. enteritidis polysaccharide conjugates using Diphtheria Toxoid (DT) as carrier protein.

### 1) Protein derivatization:

High Monomeric Diphtheria toxoid (DT) was concentrated to (10-20mg/ml) on a 10 kDa membrane and analysed for protein content
To the concentrated DT freshly prepared 1M MES Buffer was added and Adipic acid dihydrazide (ADH) (dissolved 75-100mg/ml in 100mM MES buffer pH: 5.8) in the 1:10 by weight ratio and EDAC (1-ethyl-3-(3-dimethyl-aminopropyl) carbodiimide) (dissolved 30-40 mg/ml in 100mM MES Buffer pH: 5.8) in the 1:1 by weight ratio. The reaction was continued at 5.8 pH for about 1Hrs and then the reaction mixture was diafiltered on 10 kDa TFF in 50mM Borate buffer pH 9.0 to remove residuals and unreacted components. The final sample was analysed for protein content and degree of derivatization

### 2) Conjugation of S. typhimurium and S. enteritidis polysaccharide (PS) and ADH Derivatized DT:

### Two Experiments were performed by change in 1-cyano- 4-pyrrolidinopyridinium tetrafluoroborate (CPPT) (CPIP) ratio

**Expt No:1** - The S. typhimurium and S. enteritidis polysaccharide was concentrated on 10 kDa membrane to achieve a concentration of (10-15mg/ml).

To the Concentrated PS 0.9% NaCl was added and Freshly prepared CPIP solution(114mg/ml in acetonitrile) was added into polysaccharide in the 1:1.3 by weight ratio. The pH was shifted to 9.5 immediately with 2.5M NaOH and held for up to 3 min. Then protein was added in 1:0.8 by weight ratio **PS:PR:CPIP** as **1:0.8:1.3**

Shodex columns SB-804 HQ and SB-805 HQ were used sequentially with PBS as mobile phase at 1 ml/min flow rate to monitored for protein conversion. The reaction was quenched after 3-4hours by adding 2M Glycine 10 times to that of PS by weight.

3) Conjugate Purification by GFC: A Column XK16/70 was made ready using GFC resin (Tyopearl HW65F) with a bed height of 40cm. The column was packed at a flow rate of 100cm/hr and allowed to settle and 1M NaCl 1% of the column volume was passed to assess the integrity of the packed column. The column was equilibrated using 0.9% NaCl at 30cm/hr and the conjugate was loaded on the column and fractions collected at lmin interval. The final pooled fractions were filtered and sent for analysis

### Expt No: 2 -

To the Concentrated PS 0.9% NaCl was added and Freshly prepared CPIP solution (114mg/ml in acetonitrile) was added into polysaccharide in the 1:1.1 by weight ratio. The pH was shifted to 9.5 immediately with 2.5M NaOH and held for up to 3 min. Then protein was added in 1:0.8 by weight ratio PS:PR:CPIP as**1:0.8:1.1.**

Shodex columns SB-804 HQ and SB-805 HQ were used sequentially with PBS as mobile phase at 1 ml/min flow rate to monitored for protein conversion. The reaction was quenched after 3-4hours by adding 2M Glycine 10 times to that of PS by weight.

Conjugate Purification by GFC: A Column XK16/70 was made ready using GFC resin (Tyoperal HW65F) with a bed height of 40cm. The column was packed at a flow rate of 100cm/hr and allowed to settle and 1M NaCL 1% of the column volume was passed to assess the integrity of the packed column. The column was equilibrated using 0.9% NaCL at 30cm/hr and the conjugate was loaded on the column and fractions were collected at lmin interval. The final pooled fractions were filtered and sent for analysis

**Conclusion:** Conjugation of S. typhimurium and S. enteritidis polysaccharide using ADH derivatized DT was found successful and the PS/PR ratio was found satisfactory.

### B. Preparation of S. typhimurium and S. enteritidis polysaccharide Conjugates using CRM197 as carrier protein

### 1) Protein derivatization: Cross reacting mutant (CRM197) received from Production department (SIIPL) was taken for derivatization

To the concentrated CRM197 freshly prepared 1M MES Buffer was added and Adipic acid dihydrazide (ADH) (dissolved 75-100mg/ml in 100mM MES buffer pH:6.5) in the 1:3.5 by weight ratio and EDAC (1-ethyl-3-(3-dimethyl-aminopropyl) carbodiimide) (dissolved 30-40 mg/ml in 100mM MES Buffer pH:6.5) in the 1:0.25 by weight ratio. 5% Tween 80 was added. The final reaction volume was made up using 100mM MES Buffer to achieve a final concentration of 3-4mg/ml the reaction was continued at 6.5 pH for about 3Hrs and then the reaction mixture was diafiltered on 10 kDa TFF in 50mM Borate buffer and 0.005% Tween 80 pH 9.0 to remove residuals and unreacted components. The final sample was analysed for protein content and degree of derivatization

### 2) Conjugation of S. typhimurium and S. enteritidis polysaccharide (PS) and ADH Derivatized CRM197:

The PS received from DSP was concentrated on 10 kDa membrane to achieve a concentration of (10-15mg/ml).

To the Concentrated PS 0.9% NaCl was added and Freshly prepared CPIP solution (114mg/ml in acetonitrile) was added into polysaccharide in the 1:1.3 by weight ratio. The pH was shifted to 9.5 immediately with 2.5M NaOH and held for up to 3 min. Then protein was added in 1:1 by weight ratio PS:PR:CPIP as 1:1:1.3

Shodex columns SB-804 HQ and SB-805 HQ were used sequentially with PBS as mobile phase at 1 ml/min flow rate to monitored for protein conversion. The reaction was quenched after 3-4hours by adding 2M Glycine 10 times to that of PS by weight.

**3) Conjugate Purification by GFC:** A Column XK16/70 was made ready using GFC resin (Tyoperal HW65F) with a bed height of 40cm. The column was packed at a flow rate of 100cm/hr and allowed to settle and 1M NaCL 1% of the column volume was passed to assess the integrity of the packed column. The column was equilibrated using 0.9% NaCL at 30cm/hr and the conjugate was loaded on the column and fractions collected at lmin interval, The final pooled fractions were filtered and sent for analysis.

Conclusion: Conjugation of S. typhimurium and S. enteritidis PS using ADH derivatized CRM197 was found successful and the PS/PR ratio was found satisfactory.

### C) Preparation of S.Paratyphi Conjugates using Tetanus Toxoid as carrier protein:

**1) Protein derivatization:** High Monomeric Tetanus toxoid (TT) received from Production department (SIIPL) was concentrated to (15-20mg/ml) on a 30 kDa membrane and analysed for protein content. The concentrated TT freshly prepared 1M MES Buffer was added and Adipic acid dihydrazide (ADH) (dissolved 75-100mg/ml in 100mM MES buffer pH:6.0)in the 1:10 by weight ratio and EDAC (1-ethyl-3-(3-dimethyl-aminopropyl) carbodiimide) (dissolved 30-40 mg/ml in 100mM MES BufferpH:6.0) in the 1:1 by weight ratio.The reaction was continued at 6.0 pH for about 1Hrs and then the reaction mixture was diafiltered on 30 kDa TFF in lOmM Phosphate buffer pH 7.2 to remove residuals and unreacted components. The final sample was analysed for protein content and degree of derivatization
**2) Conjugation of S. typhimurium and S. enteritidis polysaccharide (PS) and ADH Derivatized TT:**
   The OSP received from DSP Team was concentrated on 10 kDa membrane to achieve a concentration of (10-13mg/ml).

### Two Experiments were performed by change in 1-cyano- 4-pyrrolidinopyridinium tetrafluoroborate (CPPT) (CPIP) ratio

### Expt No:1

To the Concentrated PS 0.9% NaCl was added and freshly prepared CPIP solution (114mg/ml in acetonitrile) was added into polysaccharide in the 1:1.25 by weight ratio. The pH was shifted to 9.5 immediately with 2.5M NaOH and held for up to 3 min. Then protein was added in 1:0.8 by weight ratio PS:PR:CPIP **as1:0.8:1.25**

Shodex columns SB-804 HQ and SB-805 HQ were used sequentially with PBS as mobile phase at 1 ml/min flow rate tomonitored for protein conversion. The reaction was quenched after 3-4hours by adding 2M Glycine 10 times to that of PS by weight.

**3) Conjugate Purification by Ultrafiltration:** Quenched conjugate was purified by diafiltration 300 kDa TFF membrane in lOmM PBS pH7.2 followed by lOmM Tris Buffer pH7.2 The final concentrated sample was sent for analysis.

### Expt No: 2

To the Concentrated PS 0.9% NaCl was added and Freshly prepared CPIP solution (114mg/ml in acetonitrile) was added into polysaccharide in the 1:1.3 by weight ratio. The pH was shifted to 9.5 immediately with 2.5M NaOH and held for up to 3 min. Then protein was added in 1:0.9 by weight ratio PS:PR:CPIP **1:0.9:1.3**

Shodex columns SB-804 HQ and SB-805 HQ were used sequentially with PBS as mobile phase at 1 ml/min flow rate tomonitored for protein conversion. The reaction was quenched after 3-4hours by adding 2M Glycine 10 times to that of PS by weight.

**3) Conjugate Purification by Ultrafiltration:** Quenched conjugate was purified by diafiltration 300 kDa TFF membrane in lOmM PBS pH7.2 followed by lOmM Tris Buffer pH7.2 The final concentrated sample was sent for analysis.

### Conclusion: Conjugation of S. typhimurium and S. enteritidis polysaccharide (PS) using ADH derivatized TT was found successful, two different types of diafiltration strategy were applied and both the process gave satisfactory PS/Pr ratio.

### 2) Carbodiimide chemistry based conjugation of the S. typhimurium and S. enteritidis polysaccharide to Carrier protein Diphtheria Toxoid (DT), CRM197 and Tetanus Toxoid (TT)

A) Derivatization of Concentrated S. *typhimurium and S. enteritidis* polysaccharide (Addition of Linker ADH) using cyanylation chemistry and conjugation with Tetanus Toxoid (TT) using carbodiimide chemistry.
B) Derivatization of Concentrated S. *typhimurium and S. enteritidis* polysaccharide (Addition of Linker ADH) using cyanylation chemistry and conjugation with Diphtheria Toxoid (DT) using carbodiimide chemistry.
C) Derivatization of Concentrated S. *typhimurium and S. enteritidis* polysaccharide (Addition of Linker ADH) using cyanylation chemistry and conjugation with CRM197 using carbodiimide chemistry.
D) Carbodiimide chemistry (EDAC) based conjugation of the Paratyphi OSP to Carrier protein Diphtheria Toxoid (DT), CRM197 and Tetanus Toxoid (TT) without derivatization of Polysaccharide or Carrier protein.

### Procedure followed:

**1) Polysaccharide (PS) derivatization:** The S. *typhimurium and S. enteritidis* polysaccharide received was concentrated on 10 kDa membrane to achieve a concentration of (10-13mg/ml). To the Concentrated PS 0.9% NaCl was added and Freshly prepared CPIP solution (114mg/ml in acetonitrile) was added into polysaccharide in the 1:0.5 to 1: 2 (1:0.7) by weight ratio. The pH was shifted to 9.5 immediately with 2.5M NaOH and held for up to 3 min. Then Adipic acid dihydrazide (ADH) (dissolved 75-100mg/ml in 0.5M Sodium bicarbonate buffer pH:8.0) in the 1:10 by weight ratio PS:ADH:CPIP as 1:2:0.7 to 1:10:0.7 The reaction was continued at 9.5 pH for about 2Hrs,quenched using 2M Glycine 10 and then the reaction mixture was diafiltered on 10 kDa TFF in 100mM MES buffer pH 6.0 to remove residuals and unreacted components. The final sample was analysed for polysaccharide content
**2) Protein Preparation:**
   **2A) Protein Preparation:** High Monomeric Tetanus toxoid (TT) received from Production department (SIIPL) was concentrated to (10-15mg/ml) on a 30 kDa membrane and analysed for protein content
   **2B) Protein Preparation:** High Monomeric Diptheria toxoid (DT) received from Production department (SIIPL) was concentrated to (10-20mg/ml) on a 10 kDa membrane and analysed for protein content
   **2C) Protein Preparation:** High Monomeric CRM197 received from Production department (SIIPL) was concentrated to (10-20mg/ml) on a 10 kDa membrane and analysed for protein content
**3) Conjugation**
   **3A) Conjugation of ADH Derivatized S. *typhimurium and S. enteritidis* polysaccharide and Concentrated TT:**
      To the ADH derivatized PS, protein was added in 1:0.9 by weight and freshly prepared EDAC (1-ethyl-3-(3-dimethyl-aminopropyl) carbodiimide) (dissolved 30-40 mg/ml in 100mM MES Buffer) in the 1:0.86 by weight ratio. The reaction was continued at 6.0 pH **temperature 6°C ratio PS:PR:EDC as 1:0.9:0.86**
      Shodex columns SB-804 HQ and SB-805 HQ were used sequentially with PBS as mobile phase at 1 ml/min flow rate tomonitored for protein conversion. The reaction was quenched after 23 hours by adding 100mM Phospahte buffer containing EDTA.
   **3B) Conjugation of ADH Derivatized S. *typhimurium and S. enteritidis* polysaccharide and Concentrated DT:** Same conditions of TT conjugate were applied to DT to check the Pr Conversion.
      To the ADH derivatized PS, protein was added in 1:0.8 by weight and freshly prepared EDAC (1-ethyl-3-(3-dimethyl-aminopropyl) carbodiimide) (dissolved 30-40 mg/ml in 100mM MES Buffer) in the 1:0.86 by weight ratio. The reaction was continued at 6.0 pH **temperature 6°C ratio PS:PR:EDC as 1:0.8:0.86**
      Shodex columns SB-804 HQ and SB-805 HQ were used sequentially with PBS as mobile phase at 1 ml/min flow rate tomonitored for protein conversion. The reaction was quenched after **20 hours** by adding 100mM Phosphate buffer containing EDTA.
   **3C) Conjugation of ADH Derivatized S. *typhimurium and S. enteritidis* polysaccharide and Concentrated CRM197:** Same conditions of TT and DT conjugate were applied to **CRM197** to check the Pr Conversion.
      To the ADH derivatized PS, protein was added in 1:0.8 by weight and freshly prepared EDAC (1-ethyl-3-(3-dimethyl-aminopropyl) carbodiimide) (dissolved 30-40 mg/ml in 100mM MES Buffer) in the 1:0.86 by weight ratio. The reaction was continued at 6.0 pH temperature 6°C ratio PS:PR:EDC as 1:0.8:0.86
      Shodex columns SB-804 HQ and SB-805 HQ were used sequentially with PBS as mobile phase at 1 ml/min flow rate tomonitored for protein conversion. The reaction was quenched after **20 hours** by adding 100mM Phosphate buffer containing EDTA.
**4) Conjugate Purification**
   **4A) Conjugate Purification by Ultrafiltration:** Quenched conjugate was purified by diafiltration 300 kDa TFF membrane in lOmM PBS pH7.2 followed by lOmM Tris Buffer pH7.2 The final concentrated sample was sent for analysis.
   **4B) Conjugate Purification by GFC:** A Column XK16/70 was made ready using GFC resin (Tyoperal HW65F) with a bed height of 40cm. The column was packed at a flow rate of 100cm/hr and allowed to settle and 1M NaCl 1% of the column volume was passed to assess the integrity of the packed column. The column was equilibrated using 0.9% NaCl at 30cm/hr and the conjugate was loaded on the column and fractions collected at lmin interval, Fractions 2-12 were pooled according to the profile on HPLC. The final pooled fractions were filtered and sent for analysis

**Conclusion:** Using the reverse conjugation chemistry (Activating PS), Conjugation of ADH derivatized S. typhimurium and S. enteritidis polysaccharide and concentrated TT, DT and CRM197 was found successful, by increase in temperature from 6 to 10°C. Conjugation rate of reaction was increased; PS/Pr ratio was very low in all the reactions.

The Conjugation of ADH derivatized S. typhimurium and S. enteritidis polysaccharide and concentrated TT, DT and CRM197 using Carbodiimide chemistry was found successful. Rate of conjugation reaction was increased by increasing the temperature but PS/Pr ratio was low in both experiments.

### Example 4: Immunogenic Compositions

| **Table 68A: Monovalent Immunogenic Compositions comprising *Salmonella enterica serovar typhi* ViPs conjugate antigen** | | |
|---|---|---|
| **S. No.** | **Formulation Components** | **Immunogenic composition in accordance with the present disclosure [per 0.5ml Dose]** |
| 1 | *Salmonella enterica serovar typhi* ViPs-- *TT* | 25 µg |
| | *conjugate antigen;* | (1.25 - 50 µg) |
| 2 | *Salmonella enterica serovar typhi* ViPs-- *DT* | 25 µg |
| | *conjugate antigen;* | (1.25 - 50 µg) |
| 3 | *Salmonella enterica serovar typhi* ViPs-*CRM197 conjugate antigen;* | 25 µg |
| | | (1.25 - 50 µg) |
| 4 | *Salmonella enterica serovar paratyphi A OSP* | 25 µg |
| | - *TT conjugate antigen;* | (1.25 - 50 µg) |
| 5 | *Salmonella enterica serovar paratyphi A OSP* | 25 µg |
| | *- DT conjugate antigen;* | (1.25 - 50 µg) |
| 6 | *Salmonella enterica serovar paratyphi A OSP* | 25 µg |
| | *- CRM197 conjugate antigen;* | (1.25 - 50 µg) |
| 7 | *Salmonella enterica serovar typhimurium* | 25 µg |
| | *saccharide* - *TT conjugate antigen;* | (1.25 - 50 µg) |
| 8 | *Salmonella enterica* s*erovar typhimurium* | 25 µg |
| | *saccharide* - *DT conjugate antigen;* | (1.25 - 50 µg) |
| 9 | *Salmonella enterica serovar typhimurium* | 25 µg |
| | *saccharide* - *CRM197 conjugate antigen;* | (1.25 - 50 µg) |
| 10 | *Salmonella enterica serovar enteritidis* | 25 µg |
| | *saccharide* - *TT conjugate antigen;* | (1.25 - 50 µg) |
| 11 | *Salmonella enterica serovar enteritidis* | 25 µg |
| | *saccharide* - *DT conjugate antigen;* | (1.25 - 50 µg) |
| 12 | *Salmonella enterica serovar enteritidis* | 25 µg |
| | *saccharide* - *CRM197 conjugate antigen;* | (1.25 - 50 µg) |

| | | |
|---|---|---|
| Single Dose doesn't comprise of preservative 2-Phenoxyethanol Multi-dose composition may additionally comprise of 2-Phenoxyethanol - 5 mg (1 - 10 mg) | | |

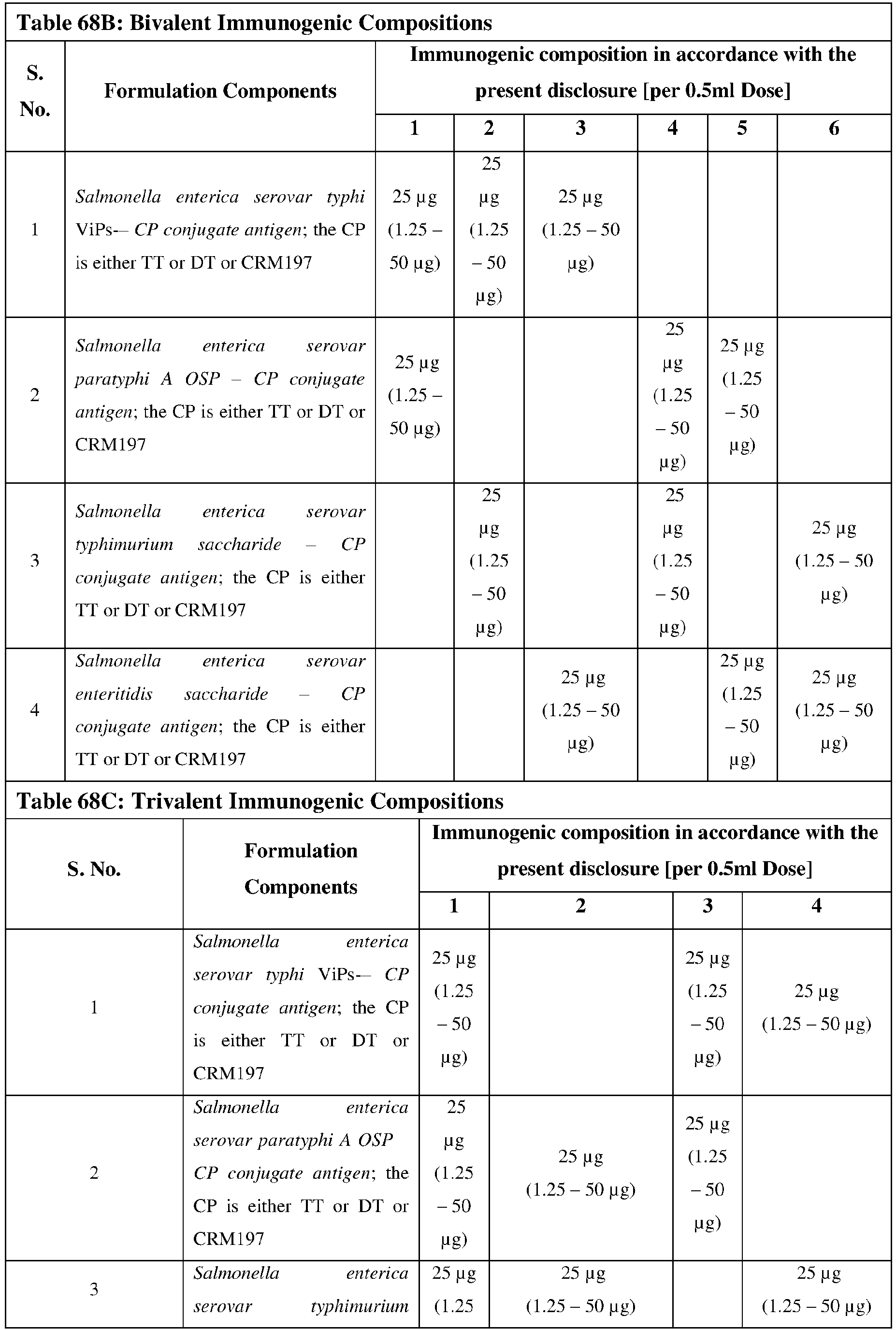

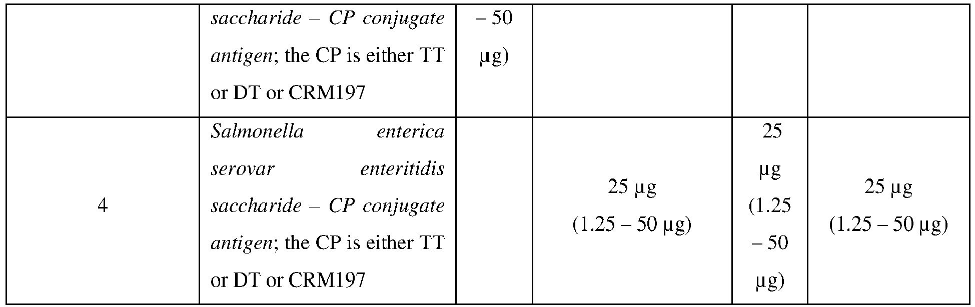

| **Table 68D: Tetravalent Immunogenic Compositions** | | |
|---|---|---|
| **S. No.** | **Formulation Components** | **Immunogenic composition in accordance with the present disclosure [per 0.5ml Dose]** |
| 1 | *Salmonella enterica serovar typhi* ViPs-- *CP conjugate antigen;* the CP is either TT or DT or CRM 197 | 25 µg |
| | | (1.25 - 50 µg) |
| 2 | *Salmonella enterica serovar paratyphi A OSP* - *CP conjugate antigen;* the CP is either TT or DT or CRM 197 | 25 µg |
| | | (1.25 - 50 µg) |
| 3 | *Salmonella enterica serovar typhimurium saccharide - CP conjugate antigen;* the CP is either TT or DT or CRM 197 | 25 µg |
| | | (1.25 - 50 µg) |
| 4 | *Salmonella enterica serovar enteritidis saccharide - CP conjugate antigen;* the CP is either TT or DT or CRM 197 | 25 µg |
| | | (1.25 - 50 µg) |

| **Table 68E: Combination of Excipient Tested in accordance with the Antigenic Component disclosed above** | | | | | | | | | | | | | | |
|---|---|---|---|---|---|---|---|---|---|---|---|---|---|---|
| **S. No.** | **Excipient** | **1** | **2** | **3** | **4** | **5** | **6** | **7** | **8** | **9** | **10** | **11** | **12** | **13** |
| 2 | Sodium chloride (140-160 mM) | 4.5 mg (1-10 mg) | 4.5 mg (1-10 mg) | 4.5 mg (1-10 mg) | 4.5 mg (1-10 mg) | 4.5 mg (1-10 mg) | 4.5 mg (1-10 mg) | 4.5 mg (1-10 mg) | 4.5 mg (1-10 mg) | 4.5 mg (1-10 mg) | 4.5 mg (1-10 mg) | 4.5 mg (1-10 mg) | 4.5 mg (1-10 mg) | 4.5 mg (1-10 mg) |
| 3 | Tris Buffer (10mM to 25mM) | - | 0.61 to 1.52 mg | 0.61 to 1.52 mg | 0.61 to 1.52 mg | | | | | | | | | |
| 4 | Citrate buffer (10mM to 25mM) | - | - | - | | CAM-1.05 to 2.63 mg TCD-1.47-3.68 mg | CAM-1.05 to 2.63 mg TCD-1.47-3.68 mg | CAM-1.05 to 2.63 mg TCD-1.47-3.68 mg | | | | | | |
| | Prepared by dissolving citric acid monohydrate (CAM) and trisodium citrate dehydrate (TCD) | | | | | | | | | | | | | |
| 5 | Histidine buffer (10mM to 25mM) | - | - | - | | | | | 0.78 to 1.94 mg | 0.78 to 1.94 mg | 0.78 to 1.94 mg | | | |
| 6 | Succinate Buffer (10mM to 25mM) | - | - | - | | | | | | | | 0.59 to 1.48 mg | 0.59 to 1.48 mg | 0.59 to 1.48 mg |
| 7 | Polysorbate-20 (0.005 to 0.1%w/v) | - | - | 25-500 µg | 25-500 µg | | 25-500 µg | 25-500 µg | | 25-500 µg | 25-500 µg | | 25-500 µg | 25-500 µg |
| 8 | Sucrose (0.5% to 5.0% w/v) | | | | 2.5 to 25 mg | | | 2.5 to 25 mg | | | 2.5 to 25 mg | | | 2.5 to 25 mg |
| 9 | Water for Injection (WFI) | q.s. | q.s. | q.s. | q.s. | q.s. | q.s. | q.s. | q.s. | q.s. | q.s. | q.s. | q.s. | q.s. |

| | | | | | | | | | | | | | | |
|---|---|---|---|---|---|---|---|---|---|---|---|---|---|---|
| √ = included in the composition | | | | | | | | | | | | | | |

| **Table 69: Combination Vaccine Compositions comprising Standard Dose and Dose reduced IPV (Salk Strain type 1 (Mahoney) or type 2 (MEF) or type 3 (Saukett)), D, T, HepB, wP, ViPs-- TT and Hib antigen IPV** | | | | | | | | | | |
|---|---|---|---|---|---|---|---|---|---|---|
| **S. No.** | **Formulation Components** | **Combination composition in accordance with the present disclosure [per 0.5ml Dose]** | | | | | | | | |
| | | **1** | **2** | **3** | **4** | **5** | **6** | **7** | | |
| 1 | Diphtheria Toxoid (D) | 22.5 Lf | 22.5 Lf | 22.5 Lf | 22.5 Lf | 22.5 Lf | 22.5 Lf | 22.5 Lf | 22.5 Lf | 22.5 Lf |
| 2 | Tetanus toxoid (T) | 7.5 Lf | 7.5 Lf | 7.5 Lf | 7.5 Lf | 7.5 Lf | 7.5 Lf | 7.5 Lf | 7.5 Lf | 7.5 Lf |
| 3 | Inactivated B. pertussis antigen (wP) | 15 IOU | 15 IOU | 15 IOU | 15 IOU | 15 IOU | 15 IOU | 15 IOU | 15 IOU | 15 IOU |
| 4 | HBs antigen | 12.5 µg | 12.5 µg | 12.5 µg | 12.5 µg | 12.5 µg | 12.5 µg | 12.5 µg | 12.5 µg | 12.5 µg |
| 5 | Hib PRP-TT conjugate antigen | 10 µg of PRP | 10 µg of PRP | 10 µg of PRP | 10 µg of PRP | 10 µg of PRP | 10 µg of PRP | 10 µg of PRP | 10 µg of PRP | 10 µg of PRP |
| 6 | Inactivated Polio Virus (IPV) | | | | | | | | | |
| | Type 1(D antigen units) | 7.5 | 8 | 5 | 10 | 10 | 10 | 10 | 4 | 40 |
| | Type 2 (D antigen units) | 16 | 2 | 2 | 2 | 2 | 2 | 2 | 0.5 | 8 |
| | Type 3(D antigen units) | 10 | 5 | 5 | 10 | 5 | 12 | 16 | 3.2 | 32 |
| 7 | Salmonella enterica serovar typhi ViPs-- TT conjugate antigen; | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) |
| 8 | Total Aluminium Content (Al³⁺) | Not more than 0.5 mg | Not more than 0.5 mg | Not more than 0.5 mg | Not more than 0.5 mg | Not more than 0.5 mg | Not more than 0.5 mg | Not more than 0.5 mg | Not more than 0.5 mg | Not more than 0.5 mg |

| **Table 70: Combination Vaccine Compositions comprising Standard Dose and Dose reduced IPV (Salk Strain type 1 (Mahoney) or type 2 (MEF) or type 3 (Saukett)), D, T, HepB, wP, ViPs-TT, OSP conjugate and Hib antigen IPV** | | | | | | | | | | |
|---|---|---|---|---|---|---|---|---|---|---|
| **S. No.** | **Formulation Components** | **Combination composition in accordance with the present disclosure [per 0.5ml Dose]** | | | | | | | | |
| | | **1** | **2** | **3** | **4** | **5** | **6** | **7** | | |
| 1 | Diphtheria Toxoid (D) | 22.5 Lf | 22.5 Lf | 22.5 Lf | 22.5 Lf | 22.5 Lf | 22.5 Lf | 22.5 Lf | 22.5 Lf | 22.5 Lf |
| 2 | Tetanus toxoid (T) | 7.5 Lf | 7.5 Lf | 7.5 Lf | 7.5 Lf | 7.5 Lf | 7.5 Lf | 7.5 Lf | 7.5 Lf | 7.5 Lf |
| 3 | Inactivated B. pertussis antigen (wP) | 15 IOU | 15 IOU | 15 IOU | 15 IOU | 15 IOU | 15 IOU | 15 IOU | 15 IOU | 15 IOU |
| 4 | HBs antigen | 12.5 µg | 12.5 µg | 12.5 µg | 12.5 µg | 12.5 µg | 12.5 µg | 12.5 µg | 12.5 µg | 12.5 µg |
| 5 | Hib PRP-TT conjugate antigen | 10 µg of PRP | 10 µg of PRP | 10 µg of PRP | 10 µg of PRP | 10 µg of PRP | 10 µg of PRP | 10 µg of PRP | 10 µg of PRP | 10 µg of PRP |
| 6 | Inactivated Polio Virus (IPV) | | | | | | | | | |
| | Type 1(D antigen units) | 7.5 | 8 | 5 | 10 | 10 | 10 | 10 | 4 | 40 |
| | Type 2 (D antigen units) | 16 | 2 | 2 | 2 | 2 | 2 | 2 | 0.5 | 8 |
| | Type 3(D antigen units) | 10 | 5 | 5 | 10 | 5 | 12 | 16 | 3.2 | 32 |
| 7 | Salmonella enterica serovar typhi ViPs-- TT conjugate antigen; | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) |
| 8 | OSP-CP Conjugate; the CP is either TT or DT or CRM 197 | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) | 25 µg (1.25 - 50 µg) |
| 9 | Total Aluminium Content (Al³⁺) | Not more than 0.5 mg | Not more than 0.5 mg | Not more than 0.5 mg | Not more than 0.5 mg | Not more than 0.5 mg | Not more than 0.5 mg | Not more than 0.5 mg | Not more than 0.5 mg | Not more than 0.5 mg |

Additionally adjusting the pH of the composition as disclosed above to about 6.0 to 7.0 with Sodium Hydroxide / Sodium Carbonate and make up the volume by adding normal saline (0.9%).

May additionally comprise of one of the preservative combination
i. 2-Phenoxyethanol in an amount of 1 to 10 mg per 0.5 ml (v/v)
ii. 2-Phenoxyethanol in an amount of 1 to 10 mg per 0.5 ml (v/v) and methylparaben in an amount of 0.1 - 1.5 mg per 0.5 ml (w/v); or
iii. 2-Phenoxyethanol in an amount of 1 to 10 mg per 0.5 ml(v/v)and propylparaben in an amount of 0.05 - 0.2 mg per 0.5 ml (w/v); or
iv. 2-Phenoxyethanol in an amount of 1 to 10 mg per 0.5 ml(v/v), methylparaben in an amount of 0.1 - 1.5 mg per 0.5 ml (w/v) and propylparaben in an amount of 0.05 - 0.2 mg per 0.5 ml (w/v).

**The Combination Vaccine Compositions comprising Dose reduced IPV, D, T, HepB, ViPs-TT, OSP conjugate, acellular pertussis, and Hib antigen may comprise of acellular pertussis antigen selected from** - Bordetella toxin in detoxified form (in particular either genetically or chemically detoxified), in particular Pertussis toxoid; Filamentous Haemagglutinin; Pertactin; or Fimbriae. Particularly Pertussis toxoid: 1 to 50 micrograms (More particularly 8µg); - Filamentous Haemagglutinin: 1 to 50 micrograms (More particularly 8µg); - Pertactin: 1 to 20 micrograms (More particularly 2.5µg); - Optionally, Fimbriae: 2 to 25 micrograms; per 0.5 ml.

| **Table 71: Combination Vaccine Compositions comprising Standard Dose and Dose reduced IPV (Sabin Strain type 1 or type 2 or type 3), D, T, HepB, wP, ViPs-- TT and Hib antigen IPV** | | | | |
|---|---|---|---|---|
| **S. No.** | **Formulation Components** | **Combination composition in accordance with the present disclosure [per 0.5ml Dose]** | | |
| | | **1** | **2** | **3** |
| 1 | Diphtheria Toxoid (D) | 22.5 Lf | 22.5 Lf | 22.5 Lf |
| 2 | Tetanus toxoid (T) | 7.5 Lf | 7.5 Lf | 7.5 Lf |
| 3 | Inactivated B. pertussis antigen (wP) | 15 IOU | 15 IOU | 15 IOU |
| 4 | HBs antigen | 12.5 µg | 12.5 µg | 12.5 µg |
| 5 | Hib PRP-TT conjugate antigen | 10 µg of PRP | 10 µg of PRP | 10 µg of PRP |
| 6 | Inactivated Polio Virus (IPV) | | | |
| | Type 1(D antigen units) | 5 | 2.5 | 7.5 |
| | Type 2 (D antigen units) | 16 | 8 | 16 |
| | Type 3(D antigen units) | 10 | 5 | 10 |
| 7 | Salmonella enterica serovar typhi ViPs-- TT conjugate antigen; | 25 µg (1.25 - 50 µg) | 25 µg (1.25 - 50 µg) | 25 µg (1.25 - 50 µg) |
| 8 | Total Aluminium Content (Al³⁺) | Not more than 0.9 mg | Not more than 0.9 mg | Not more than 0.9 mg |

| **Table 72: Combination Vaccine Compositions comprising Standard Dose and Dose reduced IPV (Sabin Strain type 1 or type 2 or type 3), D, T, HepB, wP, ViPs-TT, OSP conjugate and Hib antigen IPV** | | | | |
|---|---|---|---|---|
| **S. No.** | **Formulation Components** | **Combination composition in accordance with the present disclosure [per 0.5ml Dose]** | | |
| | | **1** | **2** | **3** |
| 1 | Diphtheria Toxoid (D) | 22.5 Lf | 22.5 Lf | 22.5 Lf |
| 2 | Tetanus toxoid (T) | 7.5 Lf | 7.5 Lf | 7.5 Lf |
| 3 | Inactivated B. pertussis antigen (wP) | 15 IOU | 15 IOU | 15 IOU |
| 4 | HBs antigen | 12.5 µg | 12.5 µg | 12.5 µg |
| 5 | Hib PRP-TT conjugate antigen | 10 µg of PRP | 10 µg of PRP | 10 µg of PRP |
| 6 | Inactivated Polio Virus (IPV) | | | |
| | Type 1(D antigen units) | 5 | 2.5 | 7.5 |
| | Type 2 (D antigen units) | 16 | 8 | 16 |
| | Type 3(D antigen units) | 10 | 5 | 10 |
| 7 | Salmonella enterica serovar typhi ViPs-- TT conjugate antigen; | 25 µg | 25 µg | 25 µg |
| | | (1.25 - 50 µg) | (1.25 - 50 µg) | (1.25 - 50 µg) |
| 8 | OSP-CP Conjugate; | 25 µg | 25 µg | 25 µg |
| | the CP is either TT or DT or CRM197 | (1.25 - 50 µg) | (1.25 - 50 µg) | (1.25 - 50 µg) |
| 9 | Total Aluminium Content (Al³⁺) | Not more than 0.9 mg | Not more than 0.9 mg | Not more than 0.9 mg |

Additionally adjusting the pH of the composition as disclosed above to about 6.0 to 7.0 with Sodium Hydroxide / Sodium Carbonate and make up the volume by adding normal saline (0.9%).

May additionally comprise of one of the preservative combination
i. 2-Phenoxyethanol in an amount of 1 to 10 mg per 0.5 ml (v/v)
ii. 2-Phenoxyethanol in an amount of 1 to 10 mg per 0.5 ml (v/v) and methylparaben in an amount of 0.1 - 1.5 mg per 0.5 ml (w/v); or
iii. 2-Phenoxyethanol in an amount of 1 to 10 mg per 0.5 ml(v/v)and propylparaben in an amount of 0.05 - 0.2 mg per 0.5 ml (w/v); or
iv. 2-Phenoxyethanol in an amount of 1 to 10 mg per 0.5 ml(v/v), methylparaben in an amount of 0.1 - 1.5 mg per 0.5 ml (w/v) and propylparaben in an amount of 0.05 - 0.2 mg per 0.5 ml (w/v).

**The Combination Vaccine Compositions comprising Dose reduced IPV, D, T, HepB, ViPs-TT, OSP Conjugate, acellular pertussis, and Hib antigen may comprise of acellular pertussis antigen selected from** - Bordetella toxin in detoxified form (in particular either genetically or chemically detoxified), in particular Pertussis toxoid; Filamentous Haemagglutinin; Pertactin; or Fimbriae. Particularly Pertussis toxoid: 1 to 50 micrograms (More particularly 8µg); - Filamentous Haemagglutinin: 1 to 50 micrograms (More particularly 8µg); - Pertactin: 1 to 20 micrograms (More particularly 2.5µg); - Optionally, Fimbriae: 2 to 25 micrograms; per 0.5 ml.

| **Table 73: Combination Vaccine Compositions comprising Standard Dose and Dose reduced IPV (Salk Strain type 1 (Mahoney) or type 2 (MEF) or type 3 (Saukett)), Inactivated Rotavirus (IRV), D, T, HepB, wP, ViPs-- TT and Hib antigen IPV** | | | | | | | | | | |
|---|---|---|---|---|---|---|---|---|---|---|
| **S. No.** | **Formulation Components** | **Combination composition in accordance with the present disclosure [per 0.5ml Dose]** | | | | | | | | |
| | | **1** | **2** | **3** | **4** | **5** | **6** | **7** | | |
| 1 | Diphtheria Toxoid (D) | 22.5 Lf | 22.5 Lf | 22.5 Lf | 22.5 Lf | 22.5 Lf | 22.5 Lf | 22.5 Lf | 22.5 Lf | 22.5 Lf |
| 2 | Tetanus toxoid (T) | 7.5 Lf | 7.5 Lf | 7.5 Lf | 7.5 Lf | 7.5 Lf | 7.5 Lf | 7.5 Lf | 7.5 Lf | 7.5 Lf |
| 3 | Inactivated B. pertussis antigen (wP) | 15 IOU | 15 IOU | 15 IOU | 15 IOU | 15 IOU | 15 IOU | 15 IOU | 15 IOU | 15 IOU |
| 4 | HBs antigen | 12.5 µg | 12.5 µg | 12.5 µg | 12.5 µg | 12.5 µg | 12.5 µg | 12.5 µg | 12.5 µg | 12.5 µg |
| 5 | Hib PRP-TT conjugate antigen | 10 µg of PRP | 10 µg of PRP | 10 µg of PRP | 10 µg of PRP | 10 µg of PRP | 10 µg of PRP | 10 µg of PRP | 10 µg of PRP | 10 µg of PRP |
| 6 | Inactivated Polio Virus (IPV) | | | | | | | | | |
| | Type 1(D antigen units) | 7.5 | 8 | 5 | 10 | 10 | 10 | 10 | 4 | 40 |
| | Type 2 (D antigen units) | 16 | 2 | 2 | 2 | 2 | 2 | 2 | 0.5 | 8 |
| | Type 3(D antigen units) | 10 | 5 | 5 | 10 | 5 | 12 | 16 | 3.2 | 32 |
| 7 | IRV | 10 µg | 10 µg | 10 µg | 10 µg | 10 µg | 10 µg | 10 µg | 10 µg | 10 µg |
| | | 1 to 50 µg | 1 to 50 µg | 1 to 50 µg | 1 to 50 µg | 1 to 50 µg | 1 to 50 µg | 1 to 50 µg | 1 to 50 µg | 1 to 50 µg |
| 8 | Salmonella enterica serovar typhi ViPs-- TT conjugate antigen; | 25 µg | 25 µg | 25 µg | 25 µg | 25 µg | 25 µg | 25 µg | 25 µg | 25 µg |
| | | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) |
| 9 | Total Aluminium Content (Al³⁺) | Not more than 0.5 mg | Not more than 0.5 mg | Not more than 0.5 mg | Not more than 0.5 mg | Not more than 0.5 mg | Not more than 0.5 mg | Not more than 0.5 mg | Not more than 0.5 mg | Not more than 0.5 mg |

| **Table 74: Combination Vaccine Compositions comprising Standard Dose and Dose reduced IPV (Salk Strain type 1 (Mahoney) or type 2 (MEF) or type 3 (Saukett)), Inactivated Rotavirus (IRV), D, T, HepB, wP, ViPs-- TT and Hib antigen IPV** | | | | | | | | | | |
|---|---|---|---|---|---|---|---|---|---|---|
| **S. No.** | **Formulation Components** | **Combination composition in accordance with the present disclosure [per 0.5ml Dose]** | | | | | | | | |
| | | **1** | **2** | **3** | **4** | **5** | **6** | **7** | | |
| 1 | Diphtheria Toxoid (D) | 22.5 Lf | 22.5 Lf | 22.5 Lf | 22.5 Lf | 22.5 Lf | 22.5 Lf | 22.5 Lf | 22.5 Lf | 22.5 Lf |
| 2 | Tetanus toxoid (T) | 7.5 Lf | 7.5 Lf | 7.5 Lf | 7.5 Lf | 7.5 Lf | 7.5 Lf | 7.5 Lf | 7.5 Lf | 7.5 Lf |
| 3 | Inactivated B. pertussis antigen (wP) | 15 IOU | 15 IOU | 15 IOU | 15 IOU | 15 IOU | 15 IOU | 15 IOU | 15 IOU | 15 IOU |
| 4 | HBs antigen | 12.5 µg | 12.5 µg | 12.5 µg | 12.5 µg | 12.5 µg | 12.5 µg | 12.5 µg | 12.5 µg | 12.5 µg |
| 5 | Hib PRP-TT conjugate antigen | 10 µg of PRP | 10 µg of PRP | 10 µg of PRP | 10 µg of PRP | 10 µg of PRP | 10 µg of PRP | 10 µg of PRP | 10 µg of PRP | 10 µg of PRP |
| 6 | Inactivated Polio Virus (IPV) | | | | | | | | | |
| | Type 1(D antigen units) | 7.5 | 8 | 5 | 10 | 10 | 10 | 10 | 4 | 40 |
| | Type 2 (D antigen units) | 16 | 2 | 2 | 2 | 2 | 2 | 2 | 0.5 | 8 |
| | Type 3(D antigen units) | 10 | 5 | 5 | 10 | 5 | 12 | 16 | 3.2 | 32 |
| 7 | IRV | 10 µg | 10 µg | 10 µg | 10 µg | 10 µg | 10 µg | 10 µg | 10 µg | 10 µg |
| | | 1 to 50 µg | 1 to 50 µg | 1 to 50 µg | 1 to 50 µg | 1 to 50 µg | 1 to 50 µg | 1 to 50 µg | 1 to 50 µg | 1 to 50 µg |
| 8 | Salmonella enterica serovar typhi ViPs-- TT conjugate antigen; | 25 µg | 25 µg | 25 µg | 25 µg | 25 µg | 25 µg | 25 µg | 25 µg | 25 µg |
| | | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) |
| 9 | OSP-CP Conjugate; | 25 µg | 25 µg | 25 µg | 25 µg | 25 µg | 25 µg | 25 µg | 25 µg | 25 µg |
| | the CP is either TT or DT or CRM 197 | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) | (1.25 - 50 µg) |
| 10 | Total Aluminium Content (Al³⁺) | Not more than 0.5 mg | Not more than 0.5 mg | Not more than 0.5 mg | Not more than 0.5 mg | Not more than 0.5 mg | Not more than 0.5 mg | Not more than 0.5 mg | Not more than 0.5 mg | Not more than 0.5 mg |

Additionally adjusting the pH of the composition as disclosed above to about 6.0 to 7.0 with Sodium Hydroxide / Sodium Carbonate and make up the volume by adding normal saline (0.9%).

May additionally comprise of one of the preservative combination
v. 2-Phenoxyethanol in an amount of 1 to 10 mg per 0.5 ml (v/v)
vi. 2-Phenoxyethanol in an amount of 1 to 10 mg per 0.5 ml (v/v) and methylparaben in an amount of 0.1 - 1.5 mg per 0.5 ml (w/v); or
vii. 2-Phenoxyethanol in an amount of 1 to 10 mg per 0.5 ml(v/v)and propylparaben in an amount of 0.05 - 0.2 mg per 0.5 ml (w/v); or
viii. 2-Phenoxyethanol in an amount of 1 to 10 mg per 0.5 ml(v/v), methylparaben in an amount of 0.1 - 1.5 mg per 0.5 ml (w/v) and propylparaben in an amount of 0.05 - 0.2 mg per 0.5 ml (w/v).

**The Combination Vaccine Compositions comprising Dose reduced IPV, IRV, D, T, HepB, ViPs-TT, acellular pertussis, and Hib antigen may comprise of acellular pertussis antigen selected from** - Bordetella toxin in detoxified form (in particular either genetically or chemically detoxified), in particular Pertussis toxoid; Filamentous Haemagglutinin; Pertactin; or Fimbriae. Particularly Pertussis toxoid: 1 to 50 micrograms (More particularly 8µg); - Filamentous Haemagglutinin: 1 to 50 micrograms (More particularly 8µg); - Pertactin: 1 to 20 micrograms (More particularly 2.5µg); - Optionally, Fimbriae: 2 to 25 micrograms; per 0.5 ml.

| **Table 75: Combination Vaccine Compositions comprising Standard Dose and Dose reduced IPV (Sabin Strain type 1 or type 2 or type 3), D, T, HepB, wP, ViPs-- TT and Hib antigen IPV** | | | | |
|---|---|---|---|---|
| **S. No.** | **Formulation Components** | **Combination composition in accordance with the present disclosure [per 0.5ml Dose]** | | |
| | | **1** | **2** | **3** |
| 1 | Diphtheria Toxoid (D) | 22.5 Lf | 22.5 Lf | 22.5 Lf |
| 2 | Tetanus toxoid (T) | 7.5 Lf | 7.5 Lf | 7.5 Lf |
| 3 | Inactivated B. pertussis antigen (wP) | 15 IOU | 15 IOU | 15 IOU |
| 4 | HBs antigen | 12.5 µg | 12.5 µg | 12.5 µg |
| 5 | Hib PRP-TT conjugate antigen | 10 µg of PRP | 10 µg of PRP | 10 µg of PRP |
| 6 | Inactivated Polio Virus (IPV) | | | |
| | Type 1(D antigen units) | 5 | 2.5 | 7.5 |
| | Type 2 (D antigen units) | 16 | 8 | 16 |
| | Type 3(D antigen units) | 10 | 5 | 10 |
| 7 | IRV | 10 µg | 10 µg | 10 µg |
| | | 1 to 50 µg | 1 to 50 µg | 1 to 50 µg |
| 8 | Salmonella enterica serovar typhi ViPsTT conjugate antigen; | 25 µg | 25 µg | 25 µg |
| | | (1.25 - 50 µg) | (1.25 - 50 µg) | (1.25 - 50 µg) |
| 9 | Total Aluminium Content (Al³⁺) | Not more than 0.9 mg | Not more than 0.9 mg | Not more than 0.9 mg |

| **Table 76: Combination Vaccine Compositions comprising Standard Dose and Dose reduced IPV (Sabin Strain type 1 or type 2 or type 3), D, T, HepB, wP, ViPs-- TT and Hib antigen IPV** | | | | |
|---|---|---|---|---|
| **S. No.** | **Formulation Components** | **Combination composition in accordance with the present disclosure [per 0.5ml Dose]** | | |
| | | **1** | **2** | **3** |
| 1 | Diphtheria Toxoid (D) | 22.5 Lf | 22.5 Lf | 22.5 Lf |
| 2 | Tetanus toxoid (T) | 7.5 Lf | 7.5 Lf | 7.5 Lf |
| 3 | Inactivated B. pertussis antigen (wP) | 15 IOU | 15 IOU | 15 IOU |
| 4 | HBs antigen | 12.5 µg | 12.5 µg | 12.5 µg |
| 5 | Hib PRP-TT conjugate antigen | 10 µg of PRP | 10 µg of PRP | 10 µg of PRP |
| 6 | Inactivated Polio Virus (IPV) | | | |
| | Type 1(D antigen units) | 5 | 2.5 | 7.5 |
| | Type 2 (D antigen units) | 16 | 8 | 16 |
| | Type 3(D antigen units) | 10 | 5 | 10 |
| 7 | IRV | 10 µg | 10 µg | 10 µg |
| | | 1 to 50 µg | 1 to 50 µg | 1 to 50 µg |
| 8 | Salmonella enterica serovar typhi ViPs-TT conjugate antigen; | 25 µg | 25 µg | 25 µg |
| | | (1.25 - 50 µg) | (1.25 - 50 µg) | (1.25 - 50 µg) |
| 9 | OSP-CP Conjugate; | 25 µg | 25 µg | 25 µg |
| | the CP is either TT or DT or CRM197 | (1.25 - 50 µg) | (1.25 - 50 µg) | (1.25 - 50 µg) |
| 10 | Total Aluminium Content (Al³⁺) | Not more than 0.9 mg | Not more than 0.9 mg | Not more than 0.9 mg |

Additionally adjusting the pH of the composition as disclosed above to about 6.0 to 7.0 with Sodium Hydroxide / Sodium Carbonate and make up the volume by adding normal saline (0.9%).

May additionally comprise of one of the preservative combination
v. 2-Phenoxyethanol in an amount of 1 to 10 mg per 0.5 ml (v/v)
vi. 2-Phenoxyethanol in an amount of 1 to 10 mg per 0.5 ml (v/v) and methylparaben in an amount of 0.1 - 1.5 mg per 0.5 ml (w/v); or
vii. 2-Phenoxyethanol in an amount of 1 to 10 mg per 0.5 ml(v/v)and propylparaben in an amount of 0.05 - 0.2 mg per 0.5 ml (w/v); or
viii. 2-Phenoxyethanol in an amount of 1 to 10 mg per 0.5 ml(v/v), methylparaben in an amount of 0.1 - 1.5 mg per 0.5 ml (w/v) and propylparaben in an amount of 0.05 - 0.2 mg per 0.5 ml (w/v).

**The Combination Vaccine Compositions comprising Dose reduced IPV, IRV D, T, HepB, ViPs-TT, acellular pertussis, and Hib antigen may comprise of acellular pertussis antigen selected from** - Bordetella toxin in detoxified form (in particular either genetically or chemically detoxified), in particular Pertussis toxoid; Filamentous Haemagglutinin; Pertactin; or Fimbriae. Particularly Pertussis toxoid: 1 to 50 micrograms (More particularly 8µg); - Filamentous Haemagglutinin: 1 to 50 micrograms (More particularly 8µg); - Pertactin: 1 to 20 micrograms (More particularly 2.5µg); - Optionally, Fimbriae: 2 to 25 micrograms; per 0.5 ml.

| **Table 77: Fully Liquid Immunogenic Composition** | | | | | |
|---|---|---|---|---|---|
| | **Single Dose(0.5mL)** | **Single Dose(0.5mL)** | **Single Dose(0.5mL)** | **Single Dose(0.5mL)** | **Single Dose(0.5mL)** |
| Men A-TT Conjugate | 5 µg (1 - 30 µg) | 5 µg (1 - 30 µg) | 5 µg (1 - 30 µg) | 5 µg (1 - 30 µg) | 5 µg (1 - 30 µg) |
| Men C-CRM197 Conjugate | 5 µg (1 - 30 µg) | 5 µg (1 - 30 µg) | 5 µg (1 - 30 µg) | 5 µg (1 - 30 µg) | 5 µg (1 - 30 µg) |
| Men Y-CRM197 Conjugate | 5 µg (1 - 30 µg) | 5 µg (1 - 30 µg) | 5 µg (1 - 30 µg) | 5 µg (1 - 30 µg) | 5 µg (1 - 30 µg) |
| Men W-CRM197 Conjugate | 5 µg (1 - 30 µg) | 5 µg (1 - 30 µg) | 5 µg (1 - 30 µg) | 5 µg (1 - 30 µg) | 5 µg (1 - 30 µg) |
| Men X-TT Conjugate | 5 µg (1 - 30 µg) | 5 µg (1 - 30 µg) | 5 µg (1 - 30 µg) | 5 µg (1 - 30 µg) | 5 µg (1 - 30 µg) |
| Vi-TT Conjugate | 25 µg (1.25 - 50 µg) | 25 µg (1.25 - 50 µg) | 25 µg (1.25 - 50 µg) | 25 µg (1.25 - 50 µg) | - |
| OSP-CP Conjugate; the CP is either TT or DT or CRM 197 | - | 25 µg (1.25 - 50 µg) | 25 µg (1.25 - 50 µg) | 25 µg (1.25 - 50 µg) | 25 µg (1.25 - 50 µg) |
| *Salmonella enterica serovar typhimurium saccharide* - *CP conjugate antigen;* the CP is either TT or DT or CRM197 | - | | 25 µg (1.25 - 50 µg) | 25 µg (1.25 - 50 µg) | 25 µg (1.25 - 50 µg) |
| *Salmonella enterica serovar enteritidis saccharide* - *CP conjugate antigen;* the CP is either TT or DT or CRM197 | - | - | - | 25 µg (1.25 - 50 µg) | 25 µg (1.25 - 50 µg) |
| Sodium Chloride | 4.5 mg (1 - 10 mg) | 4.5 mg (1- 10 mg) | 4.5 mg (1 - 10 mg) | 4.5 mg (1- 10 mg) | 4.5 mg (1- 10 mg) |
| 2-Phenoxyethanol | 5.0 mg (1 - 10 mg) | 5.0 mg (1- 10 mg) | 5.0 mg (1 - 10 mg) | 5.0 mg (1 - 10 mg) | 5.0 mg (1- 10 mg) |
| Water for Injection | Qs. 0.5mL | Qs. 0.5mL | Qs. 0.5mL | Qs. 0.5mL | Qs. 0.5mL |

| **Table 78: Lyophilized (Freeze-Dried) Immunogenic Composition** | | | | | |
|---|---|---|---|---|---|
| | **Single Dose(0.5mL)** | **Single Dose(0.5mL)** | **Single Dose(0.5mL)** | **Single Dose(0.5mL)** | **Single Dose(0.5mL)** |
| Men A-TT Conjugate | 5 µg (1 - 30 µg) | 5 µg (1 - 30 µg) | 5 µg (1 - 30 µg) | 5 µg (1 - 30 µg) | 5 µg (1 - 30 µg) |
| Men C-CRM197 Conjugate | 5 µg (1 - 30 µg) | 5 µg (1 - 30 µg) | 5 µg (1 - 30 µg) | 5 µg (1 - 30 µg) | 5 µg (1 - 30 µg) |
| Men Y-CRM197 Conjugate | 5 µg (1 - 30 µg) | 5 µg (1 - 30 µg) | 5 µg (1 - 30 µg) | 5 µg (1 - 30 µg) | 5 µg (1 - 30 µg) |
| Men W-CRM197 Conjugate | 5 µg (1 - 30 µg) | 5 µg (1 - 30 µg) | 5 µg (1 - 30 µg) | 5 µg (1 - 30 µg) | 5 µg (1 - 30 µg) |
| Men X-TT Conjugate | 5 µg (1 - 30 µg) | 5 µg (1 - 30 µg) | 5 µg (1 - 30 µg) | 5 µg (1 - 30 µg) | 5 µg (1 - 30 µg) |
| Vi-TT Conjugate | 25 µg (1.25 - 50 µg) | 25 µg (1.25 - 50 µg) | 25 µg (1.25 - 50 µg) | 25 µg (1.25 - 50 µg) | - |
| OSP-CP Conjugate; the CP is either TT or DT or CRM 197 | - | 25 µg (1.25 - 50 µg) | 25 µg (1.25 - 50 µg) | 25 µg (1.25 - 50 µg) | 25 µg (1.25 - 50 µg) |
| *Salmonella enterica serovar typhimurium saccharide* - *CP conjugate antigen;* the CP is either TT or DT or CRM197 | - | - | 25 µg (1.25 - 50 µg) | 25 µg (1.25 - 50 µg) | 25 µg (1.25 - 50 µg) |
| *Salmonella enterica serovar enteritidis saccharide* - *CP conjugate antigen;* the CP is either TT or DT or CRM197 | - | - | - | 25 µg (1.25 - 50 µg) | 25 µg (1.25 - 50 µg) |
| Sucrose | 11.90 mg (1- 50mg) | 11.90 mg (1- 50mg) | 11.90 mg (1- 50mg) | 11.90 mg (1- 50mg) | 11.90 mg (1-50mg) |
| Sodium citrate (Dihydrate) | 1.98 mg (1- 50mg) | 1.98 mg (1- 50mg) | 1.98 mg (1- 50mg) | 1.98 mg (1- 50mg) | 1.98 mg (1- 50mg) |
| Tris Buffer | 0.48 mg (0.1- 5 mg) | 0.48 mg (0.1- 5 mg) | 0.48 mg (0.1- 5 mg) | 0.48 mg (0.1- 5 mg) | 0.48 mg (0.1- 5 mg) |

### Example 5: Stability Data

**Stability of Monovalent Salmonella typhi conjugate at 2-8 deg C for initial (0), 3,** 6 **months; at 25degC for initial (0), 1, 3 months and at 40degC for initial (0), 14, 28days:**

| **Table 79: Stability of Monovalent Salmonella typhi conjugate at 2-8degC for initial (0), 3, 6 months** | | | | | | | | | | | | | | | | |
|---|---|---|---|---|---|---|---|---|---|---|---|---|---|---|---|---|
| **Storage Temperature (Celcius): 2-8degC** | | | | | **0 month** | | | | **3 months** | | | | **6 months** | | | |
| **Sr. No.** | **Sample** | Target PS Size (kDa) | **SIIPL PS Size** (kDa) | **Batch No.** | **pH** | **Appearance** | **Free PS** (%) | **SIIPL PS Conjugate Size** (kDa) | **pH** | **Appearance** | **Free PS** (%) | **SIIPL PS Conjugate Size** (kDa) | **pH** | **Appearance** | **Free PS** (%) | **SIIPL PS Conjugate Size** (kDa) |
| 1) | SIIPL Conjugate (main) | **180-**2**20** | 214 (Shoedex) | G7 SIIPL Vi PS-TT (Conjugated) 2 B#10 | 6.9 | clear yellowish liquid | 4.4 | 1389 | 6.8 | clear yellowish liquid | 6.6 | 1398 | 7.2 | clear yellowish liquid | 5.8 | 1380 |
| 2) | SIIPL Conjugate | **300** | 388 (Shoedex) | G6 SIIPL Vi PS-TT (Conjugated) 1 B#9 | 7.3 | clear yellowish liquid | 7.5 | 1359 | 6.9 | clear yellowish liquid | 9.7 | 1350 | 7.3 | clear yellowish liquid | 8.8 | 1345 |
| 3) | SIIPL Conjugate | **120** | 80 (Shoedex) | G9 SIIPL Vi PS-TT (Conjugated) 4 B#12 | 6.9 | clear yellowish liquid | 8.0 | 1352 | 7.2 | clear yellowish liquid | 8.5 | 1300 | 6.9 | clear yellowish liquid | 6.7 | 1310 |
| 4) | SIIPL Conjugate | **45** | 42 (TSK 3000 PWXL) | 68 SIIPL Vi PS-TT (Conjugated) 3 B#12 | 6.8 | clear yellowish liquid | 5.4 | 1293 | 7.1 | clear yellowish liquid | 6.5 | 1200 | 6.6 | clear yellowish liquid | 7.7 | 1187 |

| **Table 80: Stability of Monovalent Salmonella typhi conjugate at 25degC for initial (0), 1, 3 months** | | | | | | | | | | | | | | | | |
|---|---|---|---|---|---|---|---|---|---|---|---|---|---|---|---|---|
| **Storage Temperature (Celcius): 25degC** | | | | | **0 month** | | | | **1 months** | | | | **3 months** | | | |
| **Sr. No.** | **Sample** | **Target PS Size** (kDa) | **SIIPL PS Size** (kDa) | **Batch No.** | **pH** | **Appearance** | **Free PS** (%) | **SIIPL PS Conjugate Size** (kDa) | **pH** | **Appearance** | **Free PS** (%) | **SIIPL PS Conjugate Size** (kDa) | **pH** | **Appearance** | **Free PS** (%) | **SIIPL PS Conjugate Size** (kDa) |
| 1) | SIIPL Conjugate (main) | 180-220 | 214 (Shoedex) | G7 SIIPL Vi PS-TT (Conjugated) 2 B#10 | 6.9 | clear yellowish liquid | 4.4 | 1392 | 6.8 | clear yellowish liquid | 5.4 | 1380 | 6.7 | clear yellowish liquid | 6.1 | 1387 |
| 2) | SIIPL Conjugate | 300 | 388 (Shoedex) | G6 SIIPL Vi PS-TT (Conjugated) 1 B#9 | 7.3 | clear yellowish liquid | 7.5 | 1360 | 7.2 | clear yellowish liquid | 8.2 | 1370 | 7.1 | clear yellowish liquid | 7.3 | 1350 |
| 3) | SIIPL Conjugate | 120 | 80 (Shoedex) | G9 SIIPL Vi PS-TT (Conjugated) 4 B#12 | 6.9 | clear yellowish liquid | 8.0 | 1350 | 7.1 | clear yellowish liquid | 8.1 | 1280 | 7.2 | clear yellowish liquid | 8.5 | 1268 |
| 4) | SIIPL Conjugate | 45 | 42 (TSK 3000 PWXL) | 68 SIIPL Vi PS-TT (Conjugated) 3 B#12 | 6.8 | clear yellowish liquid | 5.4 | 1285 | 6.9 | clear yellowish liquid | 5.9 | 1180 | 6.6 | clear yellowish liquid | 5.2 | 1162 |

| **Table 81: Stability of Monovalent Salmonella typhi conjugate at 40degC for initial (0), 14, 28 days** | | | | | | | | | | | | | | | | |
|---|---|---|---|---|---|---|---|---|---|---|---|---|---|---|---|---|
| **Storage Temperature (Celcius): 40°C** | | | | | **0 month** | | | | **14 days (2 weeks)** | | | | **28 days (4 weeks)** | | | |
| **Sr. No.** | **Sample** | **Target PS Size** (kDa) | **SIIPL PS Size** (kDa) | **Batch No.** | pH | **Appearance** | **Free PS** (%) | **SIIPL PS Conjugate Size** (kDa) | **pH** | **Appearance** | **Free PS** (%) | **SIIPL PS Conjugate Size** (kDa) | **pH** | **Appearance** | **Free PS** (%) | **SIIPL PS Conjugate Size** (kDa) |
| 1) | SIIPL Conjugate (main) | 180-220 | 214 (Shoedex) | G7 SIIPL Vi PS-TT (Conjugated) 2 B#10 | 6.9 | clear yellowish liquid | 4.4 | 1388 | 6.7 | clear yellowish liquid | 5.8 | 1348 | 6.4 | clear yellowish liquid | 7.5 | 1355 |
| 2) | SIIPL Conjugate | 300 | 388 (Shoedex) | G6 SIIPL Vi PS-TT (Conjugated) 1 B#9 | 7.3 | clear yellowish liquid | 7.5 | 1356 | 7.1 | clear yellowish liquid | 9.7 | 1310 | 6.8 | clear yellowish liquid | 10.5 | 1320 |
| 3) | SIIPL Conjugate | 120 | 80 (Shoedex) | G9 SIIPL Vi PS-TT (Conjugated) 4 B#12 | 6.9 | clear yellowish liquid | 8.0 | 1355 | 6.8 | clear yellowish liquid | 12.3 | 1266 | 6.6 | clear yellowish liquid | 14.7 | 1270 |
| 4) | SIIPL Conjugate | 45 | 42 (TSK 3000 PWXL) | 68 SIIPL Vi PS-TT (Conjugated) 3 B#12 | 6.8 | clear yellowish liquid | 5.4 | 1290 | 6.7 | clear yellowish liquid | 8.8 | 1150 | 6.5 | clear yellowish liquid | 11.5 | 1168 |

### Observations:

Free Ps("initial< 4.5%, after 6 months NMT 7.5%" for 180-220 kDa Ps Vs "initial 5.4 % after 6 months NLT 10.5% for 388/80/45 kDa" at 40 deg C/2 to 8 deg C/25 deg C).

For 180-220 kDa, initial low free Ps, size of conjugate was also maintained over stability study, less free Ps after 6 months were observed as compared to other sizes.

### SE-HPLC Stability data

### (VI-TT Conjugate in Tris, Tris-NaCl and 0.17M NaCl)

| **Table 82: SE-HPLC Stability data of Vi-TT Conjugate in Tris, Tris-NaCl and 0.17M NaCl** | | | |
|---|---|---|---|
| **Sr. No.** | **Sample Name** | **HPLC-SEC Size (in** kDa) | |
| | | **0 Day** | **Week 2** |
| 1 | B#9 Vi-TT CJ (in lOmM Tris) 1:1 Dil in 1X PBS -20°C | 1049 | 1099 |
| 2 | B#9 Vi-TT CJ (in lOmM Tris + 0.17 M NaCl) 1:1 Dil in 1X PBS -20°C | 1108 | 1035 |
| 3 | B#13 Vi-TT CJ (in 0.17 M NaCl) 1:1 Dil in 1X PBS -20°C | 996 | 1009 |
| 4 | B#13 Vi-TT CJ (0.17 M NaCl + lOmM Tris) 1:1 Dil in 1X PBS -20°C | 1004 | 1006 |
| 5 | B#9 Vi-TT CJ (in lOmM Tris) Native 2-8°C 1:1 Dil in 1X PBS | 1049 | 996 |
| 6 | B#13 Vi-TT CJ (in 0.17 M NaCl) Native 2-8°C 1:1 Dil in 1X PBS | 996 | 965 |

### Conclusion:

The Vi-TT Conjugate found stable in lOmM tris buffer, lOmM tris containing 0.17M NaCl and in 0.17M NaCl alone.

Data for different concentrations of TRIS ( 5mM, 10 mM, 20 mM, 25 mM, 30 mM,50 mM) showing 25 mM TRIS as best case.
- Vi-TT CJ concentration was maintained in three different strengths of Tris, pH 7.2 such as 5mM, lOmM, and 20mM. This was kept in various temperature conditions such as -20°C, 2-8°C, Room Temperature and 37°C.
- Study was carried out for 2weeks and it was monitored daily for pH.
- Samples were checked for particle size and zeta potential.

### pH Results

| **Table 83: pH of Vi-TT conjugate in 5mM, 10mM, 20mM Tris at -20°C, 2-8°C, Room Temperature and 37°C** | | | | | | | | | | | | |
|---|---|---|---|---|---|---|---|---|---|---|---|---|
| **Day** | **Vi TT CJ in 5mM Tris pH 7.0** | | | | **Vi TT CJ in 10mM Tris pH 7.14** | | | | **Vi TT CJ in 20mM Tris pH 7.21** | | | |
| | **-20°C** | **2-8°C** | **RT** | **37°C** | **-20°C** | **2-8°C** | **RT** | **37°C** | **-20°C** | **2-8°C** | **RT** | **37°C** |
| **0 Day** | **7.0** | **7.0** | **7.0** | **7.0** | **7.14** | **7.14** | **7.14** | **7.14** | **7.21** | **7.21** | **7.21** | **7.21** |
| **After 2 weeks** | **6.58** | **6.95** | **6.76** | **6.94** | **6.56** | **7.08** | **6.98** | **7.06** | **7.06** | **7.10** | **7.19** | **7.00** |

### Conclusion:

- In Vi-TT CJ study, Conjugate was maintained in 5, 10 and 20 mM Tris pH 7.2, pH was within range at 2-8°C in 5, 10, and 20 mM concentration.

### Viscosity and Osmolality Measurement of Vi-TT Conjugate

### Part A: Viscosity and Osmolality Results of Tris Buffer and Sodium Chloride without Conjugate

| **Table 84: Part A: Viscosity and Osmolality of Tris Buffer and Sodium Chloride without Conjugate** | | | | |
|---|---|---|---|---|
| **Buffer Strength (in mM)** | **Osmolality (mOsm/Kg)** | **Density (g/cm³)** | **Viscosity** | |
| | | | Kinematic (mm²/s) | Dynamic (mPa.s) |
| **1) Tris Buffer pH 7.2** | | | | |
| **5** | 13 | 0.9986 | 1.030 | 1.028 |
| **30** | 61 | 0.9999 | 1.020 | 1.020 |
| **50** | 81 | 1.0005 | 1.031 | 1.031 |
| **75** | 113 | 1.0014 | 1.028 | 1.030 |

| **2) Sodium Chloride** | | | | |
|---|---|---|---|---|
| **150** | 270 | 1.0043 | 1.013 | 1.017 |
| **308** | 562 | 1.0117 | 1.020 | 1.031 |
| **350** | 607 | 1.0110 | 1.023 | 1.035 |

### Part B: Viscosity and Osmolality Results of Tris Buffer and Sodium Chloride with Conjugate

| **Table 85: Part B: Viscosity and Osmolality of Tris Buffer and Sodium Chloride with** | | | | |
|---|---|---|---|---|
| **Buffer Strength (in mM)** | **Osmolality (mOsm/Kg)** | **Density (g/cm³)** | **Viscosity** | |
| | | | **Kinematic (mm²/s)** | **Dynamic (mPa.s)** |
| **I) Tris Buffer + Conjugate** | | | | |
| 5 | 18 | 0.9985 | 1.058 | 1.057 |
| **30** | 53 | 0.9997 | 1.061 | 1.061 |
| **50** | 84 | 1.0005 | 1.066 | 1.067 |
| **75** | 124 | 1.0017 | 1.079 | 1.081 |

| **II) Sodium Chloride + Conjugate** | | | | |
|---|---|---|---|---|
| **150** | **281** | 1.0046 | 1.048 | 1.052 |
| **308** | 550 | 1.0107 | 1.053 | 1.064 |
| **350** | 1253 | 1.0122 | 1.053 | 1.066 |

### Conclusion:

The Conjugate with variable tris concentrations found isotonic and conjugate with 150mM NaCl found isotonic.

Conjugate with different tris buffer and NaCl concentrations viscosity found similar and the viscosity is suitable for use as parenteral formulations.

### Example 6: Immunogenicity Data

### A. Immunogenicity study of Monovalent conjugate vaccine:

Immunogenic potential of SIIP Vi TT vaccine was assessed in mouse model.

### Mice Immunogenicity Study #1:

An immunogenicity study was conducted in mice wherein unconjugated and TT-conjugated SIIPL vaccine (Vi TT) was administered intramuscularly to mice (8 animals per group). The animals were inoculated on day 1 and day 14 and the sera sample was collected on day 14 (data not shown) and day 21. Please refer to Table 86 for details. Paired sera sample was available from all the animals in each group. The sera sample was used for determination of antibodies against the injected polysaccharide using a suitable serological immunochemical method such as an in-house IgG ELISA. The sera from mice which received unconjugated SIIPL polysaccharide (Vi PS) was used a comparative control for induction of IgG response in sera from mice which received the conjugated version of Vi PS (of varying PS size).

### Mice Immunogenicity Study #2:

Another similar study was performed in mice. Both the studies employed a similar animal treatment protocol. The animal protocol is briefly described below. The sera from both the studies were tested by the identical immunological / serological analytical assay i.e. IgG ELISA. The 5-6 weeks old, female mice (Balb C strain; bred in-house) were used in the study using an IAEC approved animal study protocol. All the animals were Special Pathogen Free and were handled under aseptic conditions under a bio-safety cabinet during inoculation and blood sample collection. The mice weighed a~18-20 g. at the start of the study. Each mouse received 2.5µg of conjugated Vi TT vaccine via intramuscular route. The SIIPL Vi TT vaccine was diluted in phosphate buffered saline (PBS) as a vehicle. No adjuvant was used in the study for any of the treatment groups.

**Treatment Schematic:** The following table summarizes the overall treatment plan for the study.

| **Table 86: Mice treatment Plan** | | | | | | | |
|---|---|---|---|---|---|---|---|
| **No .** | **Grou p ID** | **Treatment Arms (Actual Plan)** | **Animals/Grou p** | **Vi PS dose per anima l** | **Injectio n Volume** | **Day of Injectio n (IM)** | **Blood (serum) collectio n day** |
| | | | **Balb/C (Female) Mice** | | | | |
| 1 | G1 | Vehicle alone (PBS) | 8 | PBS | 100uL | 1, 14 | 21 |
| 2 | G2 | Typbar (Unconjugated) | 8 | 2.5ug | 100uL | 1, 14 | 21 |
| 3 | G3 | Typbar TCV(Conjugate d) | 8 | 2.5ug | 100uL | 1, 14 | 21 |
| 4 | G4 | NIBSC Vi PS Standard | 8 | 2.5ug | 100uL | 1, 14 | 21 |
| 5 | G5 | SIIPL Vi PS (Unconjugated) | 8 | 2.5ug | 100uL | 1, 14 | 21 |
| 6 | G6 | G6 SIIPL Vi PS-TT | 8 | 2.5ug | 100uL | 1, 14 | 21 |
| 7 | G7 | G7 SIIPL Vi PS-TT | 8 | 2.5ug | 100uL | 1, 14 | 21 |
| 8 | G8 | G8 SIIPL Vi PS-TT | 8 | 2.5ug | 100uL | 1, 14 | 21 |
| 9 | G9 | G9 SIIPL Vi PS-TT | 8 | 2.5ug | 100uL | 1, 14 | 21 |

### Methodology and techniques used for serological analysis:

The blood sample was collected from the experimental animals from the retro-orbital vein using sterile, glass capillary tubes. The isoflurane was used as a safe anesthetic during the blood collection procedure. The sera were subjected to analysis of IgG antibodies produced in response to injected unconjugated or conjugated Vi TT vaccine using an in-house IgG ELISA. The ELISA used NIBSC Vi PS Reference Standard the (Catalog No. 16/126; first international standard for Vi PS of S. *Typhi)* as a coating antigen. The IgG levels were estimated by analyzing the optical density (OD) observed in sera from mice which received the conjugated of Vi PS (Vi TT of varying PS size) as compared to the OD observed in sera samples from mice which received unconjugated SIIPL polysaccharide (Vi PS).

The following tables indicate the immunogenicity induction data (on day 21) from different Vi TT PS.

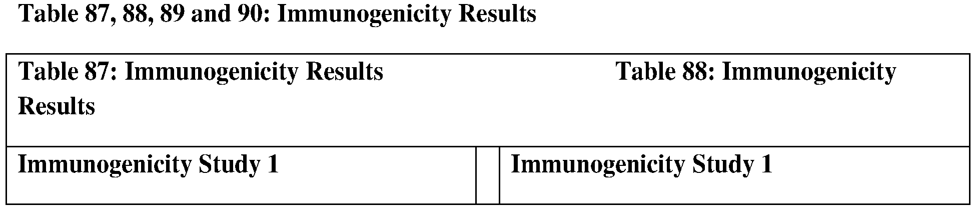

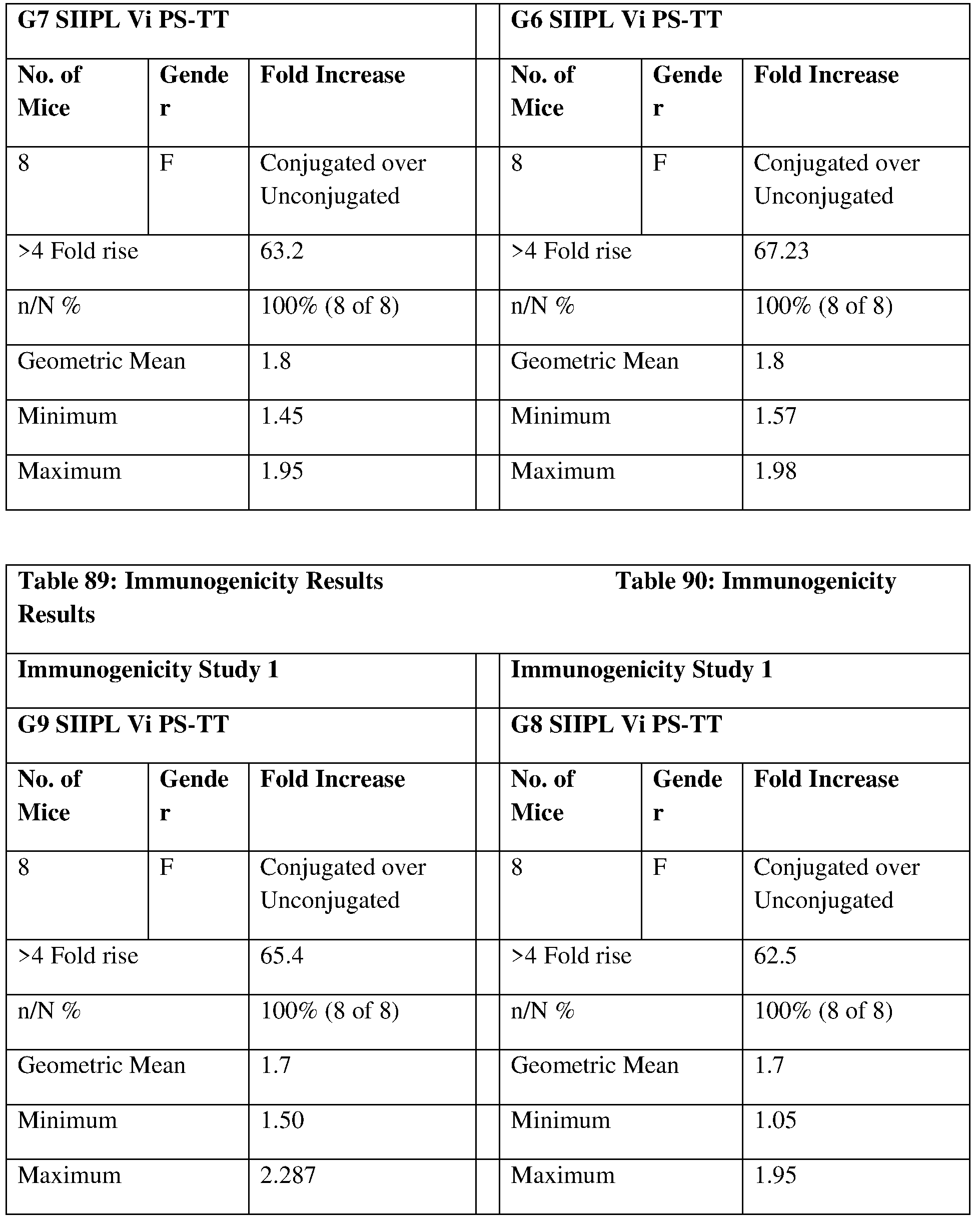

### Observations:

The mice groups with Vi TT exhibited >4-fold higher induction of IgG (as compared mice administered with unconjugated Vi PS) thus strongly indicating the immunogenic potential of SIIPL VI TT across all the PS sizes. Two separate studies were performed with Vi TT and both these studies indicated similar response to Vi TT over Vi PS.

The following parameters were compared across the various groups:
a. >4-fold rise: average fold rise was >60 fold
b. GM in antibody titer: all groups exhibited higher GM than observed in unconjugated PS (>1.9)
c. % of mice with positive response: all the mice in all the Vi TT groups exhibited higher antibody response (100% positives).

### Conclusions from analysis of serological data:

The immunogenicity study in mice was performed with 8 (female) mice in each group. The antibody induction was assessed, in all the groups, in response to the injected Vi TT (varying PS size) versus the unconjugated Vi PS. All the Vi TT PS forms strongly indicated induction of Vi TT specific IgG antibodies (day 21; after first booster) as compared to Vi PS alone group. The induction of IgG in mice sera is a strong determinant of serological response to the Vi TT. Therefore, monovalent Vi TT is able to induce a strong immunogenicity response in mouse model and provides a promising potential for future studies in higher animals.

| **Table 91: Summary of Immunogenicity Potential of Monovalent SIIPL Vi TT Vaccine in Mouse Model** | | | | | | | | |
|---|---|---|---|---|---|---|---|---|
| **Sr. No.** | **Sample** | **Target PS Size** (kDa) | **SIIPL PS Size** (kDa) | **Conjugate Changes** | **Batch No.** | **Immunogenicity (GM Data)** | **Fold Rise in** GM | **% O-Acetylation (by NMR)** |
| 1) | SIIPL Conjugate (main) | **180-220** | 214 | Purification: GFC | G7 SIIPL Vi PS-TT | 1.94 | 69 | 105% |
| 2) | SIIPL Conjugate | **300** | 388 | Purification: GFC | G6 SIIPL Vi PS-TT | 1.79 | 64 | 107% |
| 3) | SIIPL Conjugate | **120** | 80 | Purification: GFC | G9 SIIPL Vi PS-TT | 1.79 | 64 | 102% |
| 4) | SIIPL Conjugate | **45** | 42 | Purification: | G8 SIIPL | 1.75 | 63 | 102% |
| | | | | Diafiltration | Vi PS-TT | | | |
| 5) | Typbar TCV | **250-300** | NA | NA | NA | 1.69 | 60 | - |

### Observations:

1. The immunogenicity data is assessed in terms of IgG antibody levels observed in response to the SIIPL Vi TT conjugates in mouse model.
2. The IgG induction is measured as Geometric Mean (geometric mean) of colorimetric response (OD) observed in IgG ELISA.
3. The GM observed in response to SIIPL Vi TT is considered as immunogenic if the fold rise in OD in VI TT treatment is higher than 4 fold over OD observed in response to unconjugated Vi PS.

### Conclusions:

1. All of the monovalent Vi-TT conjugates were found to induce immunogenic response observed via a serological assay.
2. All of monovalent Vi-TT Conjugates exhibited higher IgG levels than observed in Typbar TCV vaccine group.
3. The induction of immunogenicity response (GM and >4 fold rise) was observed in following order: G7 SIIPL Vi PS-TT (PS Size 214) > G7 SIIPL Vi PS-TT (PS Size 388) >Typbar TCV (Commercial vaccine)

### B. Immunogenicity study of Bivalent conjugate vaccine

Immunogenic potential of bivalent (typhoid and paratyphoid) SIIPL vaccine was assessed in mouse model.

### Mice Immunogenicity Study #1:

An immunogenicity study was conducted in mice wherein a bivalent vaccine containing a combination of monovalent Vi TT vaccine and monovalent O-SP A (O Specific Polysaccharide antigen of S. *Paratyphi A*) conjugated to carrier proteins such as TT, DT and CRM was employed in the study. The monovalent and bivalent versions of these vaccines were administered intramuscularly to mice (8 animals per group). The animals were inoculated on days 1, 14 and 28 while the sera samples were collected on days 14, 28 and 42. Paired sera sample were available from all the animals in each group. Please refer to below Table 92 for details regarding the treatment plan. The sera samples were used for determination of antibodies against the injected polysaccharide using a suitable serological immunochemical method such as an in-house IgG ELISA. The sera from mice which received respective unconjugated SIIPL polysaccharide (Vi PS) and O-SP alone were used a comparative control for induction of IgG response.

The animal protocol is briefly described in Table below.

The sera samples were tested by immunological / serological analytical assay i.e. IgG ELISA. The 5-6 weeks old, female mice (Balb C strain; bred in-house) were used in the study using an IAEC approved animal study protocol. All the animals were Special Pathogen Free and were handled under aseptic conditions under a biosafety cabinet during inoculation and blood sample collection. The mice weighing in the range of -18-20 g. were used in the study. Each mouse received 2.5µg of monovalent conjugated Vi TT alone or O-SP A DT/TT/CRM alone and 2.5µg of each in bivalent vaccine (monovalent conjugated Vi TT mixed with O-SP A DT/TT/CRM) via intramuscular route. The vaccines were diluted in phosphate buffered saline (PBS) as a vehicle. No adjuvant was used in the study for any of the treatment groups.

**Treatment Schematic:** The following table summarizes the overall treatment plan for the study.

| **Table 92: Mice treatment Plan** | | | | | | | |
|---|---|---|---|---|---|---|---|
| **Sr.No.** | **Group ID** | **Treatment Arms (Actual Plan)** | **Animals / Group** | **Vi PS** / **O-SP dose per animal** | **Injection Volume** | **Day of Injection (IM)** | **Blood (serum) collection** day |
| | | | **Balb/C (Female) Mice** | | | | |
| 1 | G1 | Vehicle alone (PBS) | 8 | PBS | 50µL | 1, 14, 28 | 14, 28, 42 |
| 2 | G2 | Typbar TCV (Conjugated) | 8 | 2.5µg | 50µL | 1, 14, 28 | 14, 28, 42 |
| 3 | G3 | SIIPL Vi PS (Unconjugated) | 8 | 2.5µg | 50µL | 1, 14, 28 | 14, 28, 42 |
| 4 | G4 | SIIPL Vi PS-TT | 8 | 2.5µg | 50µL | 1, 14, 28 | 14, 28, 42 |
| 5 | G5 | SIIPL O-SP A (Unconjugated) | 8 | 2.5µg | 50µL | 1, 14, 28 | 14, 28, 42 |
| 6 | G6 | SIIPL O-SP A DT/ TT/ CRM | 8 | 2.5µg | 50µL | 1, 14, 28 | 14, 28, 42 |
| 7 | G7 | SIIPL Vi PS-TT + SIIPL O-SP A DT | 8 | 2.5µg of each component | 50µL | 1, 14, 28 | 14, 28, 42 |
| 8 | G8 | SIIPL Vi PS-TT + SIIPL O-SP A TT | 8 | 2.5µg of each component | 50µL | 1, 14, 28 | 14, 28, 42 |
| 9 | G9 | SIIPL Vi PS-TT + SIIPL O-SP A CRM | 8 | 2.5µg of each component | 50µL | 1, 14, 28 | 14, 28, 42 |
| 10 | G10 | SIIPL O-SP A CRM + Typbar TCV (Conjugated) | 8 | 2.5µg of each component | 50µL | 1, 14, 28 | 14, 28, 42 |

### Methodology and techniques used for serological analysis:

The blood samples were collected from the experimental animals from the retro-orbital vein using sterile, glass capillary tubes. The isoflurane was used as a safe anesthetic during the blood collection procedure. The sera was subjected to analysis of IgG antibodies produced in response to injected antigen using an in-house IgG ELISA. The IgG levels were estimated in sera samples from all the study groups by colorimetric analysis.

### Observations:

The following tables indicate the immunogenicity induction data (on day 21) from different bivalent (typhoid and paratyphoid) SIIPL vaccines.

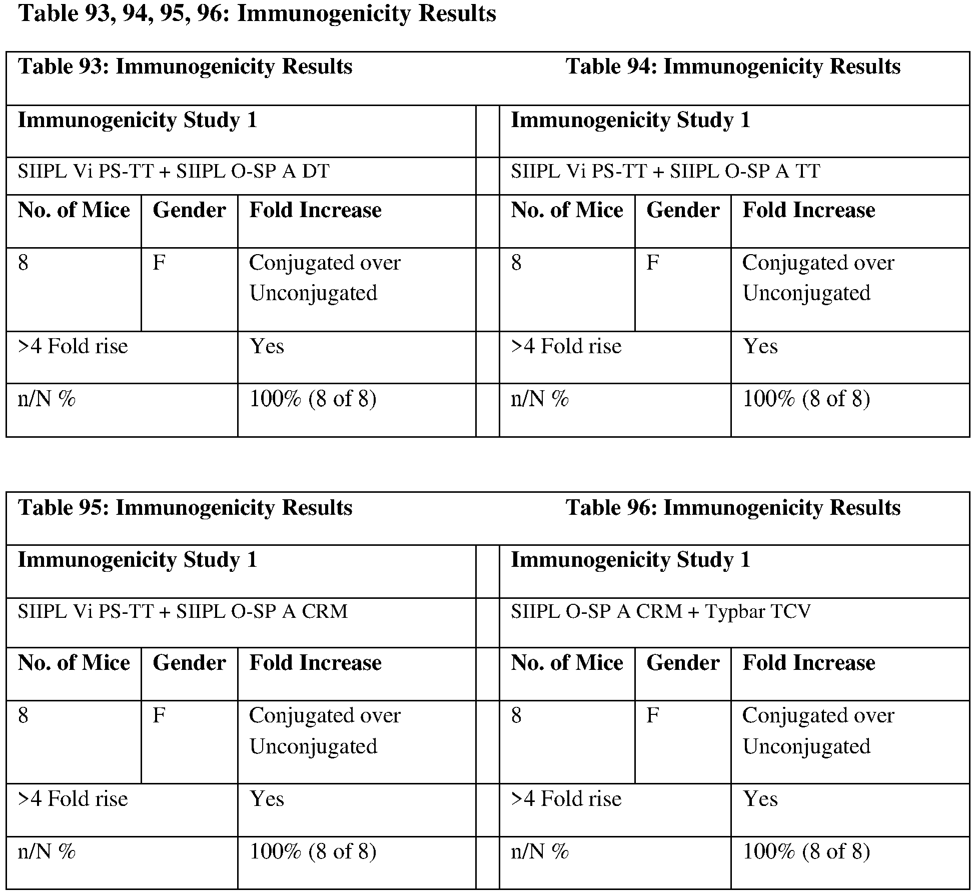

### Observations:

The mice groups injected with bivalent (typhoid and paratyphoid) SIIPL vaccine such as a) SIIPL Vi PS-TT + SIIPL O-SP A DT, b) SIIPL Vi PS-TT + SIIPL O-SP A TT and c) SIIPL Vi PS-TT + SIIPL O-SP A CRM Vi TT exhibited >4-fold higher induction of IgG (as compared to mice administered with unconjugated Vi PS or unconjugated SIIPL O-SP A) thus indicating the immunogenic potential of bivalent SIIPL vaccine containing combination of Vi TT and SIIPL O-SP A (DT/TT/CRM).

The IgG induction by bivalent (typhoid and paratyphoid) SIIPL vaccine was higher than the IgG induction observed in bivalent vaccine containing SIIPL O-SP A CRM and commercial Vi TT vaccine Typbar TCV.

The following parameters were compared across the various groups:
a. >4-fold rise: average fold rise was > 10 fold
b. GM in antibody titer: all groups exhibited higher GM than observed in unconjugated PS
c. % of mice with positive response: all the mice in all the Vi TT groups exhibited higher antibody response (100% positives).

### Conclusions from analysis of serological data:

The immunogenicity study in mice was performed with 8 (female) mice in each group. The antibody induction was assessed, in all the groups, in response to the injected bivalent (typhoid and paratyphoid) SIIPL vaccine versus the unconjugated Vi PS and O-SP A PS. All the bivalent (typhoid and paratyphoid) SIIPL vaccines strongly indicated induction of PS specific IgG antibodies (day 21; after first booster) as compared to PS alone (Vi and O-SP A) group. The induction of IgG in mice sera is a strong determinant of serological response to the bivalent (typhoid and paratyphoid) SIIPL vaccine. Therefore, bivalent (typhoid and paratyphoid) SIIPL vaccine is able to induce a strong immunogenicity response in mouse model and provides a promising potential for future studies in higher animals.

### Observations:

1. The immunogenicity data is assessed in terms of IgG antibody levels observed in response to the bivalent (typhoid and paratyphoid) SIIPL vaccine in mouse model.
2. The IgG induction is measured as Geometric Mean (geometric mean) of colorimetric response (OD) observed in IgG ELISA.
3. The GM observed in response to bivalent (typhoid and paratyphoid) SIIPL vaccine is considered as immunogenic if the fold rise in OD in VI TT treatment is higher than 4 fold over OD observed in response to unconjugated Vi PS.

### Conclusions:

1. All of the bivalent (typhoid and paratyphoid) SIIPL vaccine conjugates were found to induce immunogenic response observed via a serological assay.
2. All of bivalent (typhoid and paratyphoid) SIIPL vaccine conjugates exhibited higher IgG levels than observed in unconjugated counterparts.
3. The IgG induction by bivalent (typhoid and paratyphoid) SIIPL vaccine was higher than the IgG induction observed in bivalent vaccine containing SIIPL O-SP A CRM and commercial Vi TT vaccine Typvar TCV.
4. The induction of immunogenicity response (GM and >4 fold rise) was observed in mice receiving "bivalent (typhoid and paratyphoid) SIIPL vaccine" and hence it can be concluded that bivalent (typhoid and paratyphoid) SIIPL vaccine is able to induce a strong immunogenicity response in mouse model suggestive of its clinical immunogenic potential.

### Example 7: Single dose vaccine kit

**A) Single dose vaccine kit comprising of:**
   a first container containing a lyophilized (freeze-dried) immunogenic composition:
      a) *Neisseria meningitidis A* saccharide - TT conjugate antigen 5 µg per 0.5 ml;
      b) *Neisseria meningitidis* C saccharide - CRM197 conjugate antigen 5 µg per 0.5 ml;
      c) *Neisseria meningitidis Y* saccharide - CRM197 conjugate antigen 5 µg per 0.5 ml;
      d) *Neisseria meningitidis W* -135 saccharide - CRM197 conjugate antigen 5 µg per 0.5 ml;
      e) *Neisseria meningitidis X* saccharide - TT conjugate antigen 5 µg per 0.5 ml;
      f) Sucrose 1-12 mg per 0.5 ml;
      g) Sodium citrate (Dihydrate) 0.1 - 2 mg per 0.5 ml;
      h) Tris Buffer 0.05 - 0.5 mg per 0.5 ml;
      and
   a second container containing a liquid composition for the reconstitution of the lyophilized (freeze-dried) immunogenic composition comprising:
      a) *Salmonella enterica serovar typhi* ViPs-TT conjugate antigen 1.25 - 50 µg;
      b) Sodium chloride 1-10 mg;
      c) Water for Injection (WFI) q.s.;
   **Interpretation:**
   No antigenic interference of ViPs-TT with *Neisseria meningitidis* antigens, for prophylaxis against typhoid caused by Salmonella typhi and *Neisseria meningitidis* antigens wherein the said vaccine formulation is sufficient to elicit the required T- dependent immune response against S. typhi including in children below 2 years of age, adolescent adult, and elders through only one injection to comprise a complete vaccination schedule.
**B) Single dose vaccine kit comprising of:**
   a first container containing a lyophilized (freeze-dried) immunogenic composition:
      a) *Neisseria meningitidis A* saccharide - TT conjugate antigen 5 µg per 0.5 ml;
      b) *Neisseria meningitidis* C saccharide - CRM197 conjugate antigen 5 µg per 0.5 ml;
      c) *Neisseria meningitidis Y* saccharide - CRM197 conjugate antigen 5 µg per 0.5 ml;
      d) *Neisseria meningitidis W* -135 saccharide - CRM197 conjugate antigen 5 µg per 0.5 ml;
      e) *Neisseria meningitidis X* saccharide - TT conjugate antigen 5 µg per 0.5 ml;
      f) Sucrose 1-12 mg per 0.5 ml;
      g) Sodium citrate (Dihydrate) 0.1 - 2 mg per 0.5 ml;
      h) Tris Buffer 0.05 - 0.5 mg per 0.5 ml;
      and
   a second container containing a liquid composition for the reconstitution of the lyophilized (freeze-dried) immunogenic composition comprising:
      a) *Salmonella enterica serovar typhi* ViPs-TT conjugate antigen 1.25 - 50 µg;
      b) *Salmonella enterica serovar paratyphi A* OSP - CP conjugate antigen 1.25 - 50 µg; wherein the CP is either TT or DT or CRM197;
      c) Sodium chloride 1-10 mg;
      d) Water for Injection (WFI) q.s.;
   **Interpretation:**
   No antigenic interference of ViPs-TT; OSP antigen with *Neisseria meningitidis* antigens, for prophylaxis against typhoid and paratyphoid caused by Salmonella typhi, S. paratyphi and *Neisseria meningitidis* antigens wherein the said vaccine formulation is sufficient to elicit the required T- dependent immune response against S. typhi and paratyphi including in children below 2 years of age, adolescent adult, and elders through only one injection to comprise a complete vaccination schedule.
**C) Single dose vaccine kit comprising of:**
   a first container containing a fully liquid hexavalent immunogenic composition:
      a) an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;
      b) a diphtheria toxoid, (D) antigen in an amount of 1 to 50 Lf per 0.5 ml;
      c) a tetanus toxoid, (T) antigen in an amount of 1 to 30 Lf per 0.5 ml;
      d) a whole cell pertussis, (wP) antigen in an amount of 1 to 50 IOU per 0.5 ml or acellular pertussis, (aP) antigen comprising one or more of modified adenylate cyclase, Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25µg; per 0.5ml;
      e) a hepatitis B virus surface antigen, (HBsAg) in an amount of 1 to 20 µg per 0.5 ml;
      f) a *Haemophilus influenzae* type b antigen, (Hib) in an amount of 1 to 20 µg per 0.5 ml;
      and
   a second container containing a fully liquid immunogenic composition comprising:
      a) *Salmonella enterica serovar typhi* ViPs-TT conjugate antigen 1.25 - 50 µg;
      b) Sodium chloride 1-10 mg; and/or
      c) Tris Buffer or Citrate buffer or Histidine buffer or Succinate Buffer 0.1 mg - 1.6 mg;
         and/or
      d) Polysorbate 20; and/or
      e) 2-Phenoxyethanol 1-10 mg; and/or
      f) Water for Injection (WFI) q.s.
   **Interpretation:**
   No antigenic interference of ViPs-TT with Hexavalent immunogenic composition, for prophylaxis against typhoid caused by Salmonella typhi and Hexavalent antigens wherein the said vaccine formulation is sufficient to elicit the required T- dependent immune response against S. typhi including in children below 2 years of age, adolescent adult, and elders through only one injection to comprise a complete vaccination schedule.
**D) Single dose vaccine kit comprising of:**
   a first container containing a fully liquid hexavalent immunogenic composition:
      a) an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;
      b) a diphtheria toxoid, (D) antigen in an amount of 1 to 50 Lf per 0.5 ml;
      c) a tetanus toxoid, (T) antigen in an amount of 1 to 30 Lf per 0.5 ml;
      d) a whole cell pertussis, (wP) antigen in an amount of 1 to 50 IOU per 0.5 ml or acellular pertussis, (aP) antigen comprising one or more of modified adenylate cyclase, Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25µg; per 0.5ml;
      e) a hepatitis B virus surface antigen, (HBsAg) in an amount of 1 to 20 µg per 0.5 ml;
      f) a *Haemophilus influenzae* type b antigen, (Hib) in an amount of 1 to 20 µg per 0.5 ml;
      and
   a second container containing a fully liquid immunogenic composition comprising:
      a) *Salmonella enterica serovar typhi* ViPs-TT conjugate antigen 1.25 - 50 µg;
      b) *Salmonella enterica serovar paratyphi A* OSP - CP conjugate antigen 1.25 - 50 µg; wherein the CP is either TT or DT or CRM197;
      c) Sodium chloride 1-10 mg; and/or
      d) Tris Buffer or Citrate buffer or Histidine buffer or Succinate Buffer 0.1 mg - 1.6 mg; and/or
      g) Polysorbate selected from polysorbate 20; and/or
      h) 2-Phenoxyethanol 1-10 mg; and/or
      e) Water for Injection (WFI) q.s.
   **Interpretation:**
   No antigenic interference of ViPs-TT; OSP antigen with *Hexavalent* antigens, for prophylaxis against typhoid and paratyphoid caused by Salmonella typhi, S. paratyphi and *Hexavalent* antigens wherein the said vaccine formulation is sufficient to elicit the required T- dependent immune response against S. typhi and paratyphi including in children below 2 years of age, adolescent adult, and elders through only one injection to comprise a complete vaccination schedule.
**E) Single dose vaccine kit comprising of:**
   a first container containing a fully liquid heptavalent immunogenic composition:
      a) an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;
      b) an inactivated rotavirus antigen selected from CDC-9, CDC-66 or any other inactivated rotavirus strains present in an amount in the range of 1 to 50 µg per 0.5 ml;
      c) a diphtheria toxoid, (D) antigen in an amount of 1 to 50 Lf per 0.5 ml;
      d) a tetanus toxoid, (T) antigen in an amount of 1 to 30 Lf per 0.5 ml;
      e) a whole cell pertussis, (wP) antigen in an amount of 1 to 50 IOU per 0.5 ml or acellular pertussis, (aP) antigen comprising one or more of modified adenylate cyclase, Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25µg; per 0.5ml;
      f) a hepatitis B virus surface antigen, (HBsAg) in an amount of 1 to 20 µg per 0.5 ml;
      g) a *Haemophilus influenzae* type b antigen, (Hib) in an amount of 1 to 20 µg per 0.5 ml;
      and
   a second container containing a fully liquid immunogenic composition comprising:
      a) *Salmonella enterica serovar typhi* ViPs-TT conjugate antigen 1.25 - 50 µg;
      b) Sodium chloride 1-10 mg;
      c) Water for Injection (WFI) q.s.;
   **Interpretation:**
   wherein there is no antigenic interference of ViPs-TT with Heptavalent immunogenic composition, for prophylaxis against typhoid caused by Salmonella typhi and Heptavalent antigens wherein the said vaccine formulation is sufficient to elicit the required T- dependent immune response against S. typhi including in children below 2 years of age, adolescent adult, and elders through only one injection to comprise a complete vaccination schedule.
F) **Single dose vaccine kit comprising of:**
   a first container containing a fully liquid hexavalent immunogenic composition:
      a) an inactivated *polio virus* (IPV) antigen selected from Sabin or Salk Strain; wherein IPV Type 1 at a dose 1-50 D-antigen units (DU), IPV Type 2 at a dose of 1-50 D-antigen unit (DU) or IPV Type 3 at a dose of 1-50 D-antigen unit (DU), per 0.5 ml;
      b) an inactivated rotavirus antigen selected from CDC-9, CDC-66 or any other inactivated rotavirus strains present in an amount in the range of 1 to 50 µg per 0.5 ml;
      c) a diphtheria toxoid, (D) antigen in an amount of 1 to 50 Lf per 0.5 ml;
      d) a tetanus toxoid, (T) antigen in an amount of 1 to 30 Lf per 0.5 ml;
      e) a whole cell pertussis, (wP) antigen in an amount of 1 to 50 IOU per 0.5 ml or acellular pertussis, (aP) antigen comprising one or more of modified adenylate cyclase, Pertussis toxoid (PT) 1-50µg, Filamentous hemagglutinin (FHA) 1-50µg, Pertactin (P69 or PRN) 1-20µg or Fimbrial proteins (FIM 1 , 2 and 3) 2-25µg; per 0.5ml;
      f) a hepatitis B virus surface antigen, (HBsAg) in an amount of 1 to 20 µg per 0.5 ml;
      g) a *Haemophilus influenzae* type b antigen, (Hib) in an amount of 1 to 20 µg per 0.5 ml;
      and
   a second container containing a fully liquid immunogenic composition comprising:
      a) *Salmonella enterica serovar typhi* ViPs-TT conjugate antigen 1.25 - 50 µg;
      b) *Salmonella enterica serovar paratyphi A* OSP - CP conjugate antigen 1.25 - 50 µg; wherein the CP is either TT or DT or CRM197;
      c) Sodium chloride 1-10 mg; and/or
      d) Tris Buffer or Citrate buffer or Histidine buffer or Succinate Buffer 0.1 mg - 1.6 mg; and/or
      g) Polysorbate selected from polysorbate 20;
      h) 2-Phenoxyethanol 1-10 mg; and/or
      e) Water for Injection (WFI) q.s.
   **Interpretation:**
   No antigenic interference of ViPs-TT; OSP antigen with *Heptavalent* antigens, for prophylaxis against typhoid and paratyphoid caused by Salmonella typhi, S. paratyphi and *Heptavalent* antigens wherein the said vaccine formulation is sufficient to elicit the required T- dependent immune response against S. typhi and paratyphi including in children below 2 years of age, adolescent adult, and elders through only one injection to comprise a complete vaccination schedule.

## Claims

1. An immunogenic vaccine composition wherein the immunogenic composition comprises:
a) a bivalent polysaccharide-protein conjugate vaccine comprising or consisting of a Salmonella enterica serovar typhi saccharide-carrier protein (CP) conjugate antigen and a Salmonella enterica serovar Paratyphi A saccharide- carrier protein (CP) conjugate antigen;
b) a sugar selected from the group of sucrose, mannitol, trehalose, mannose, raffinose, lactitol, lactobionic acid, glucose, maltulose, iso-maltulose, maltose, lactose sorbitol, dextrose, fructose, glycerol, or a combination thereof,
c) a buffering agent selected from the group consisting of carbonate, phosphate, acetate, HEPES, Succinate, Histidine, TRIS, borate, citrate, lactate, gluconate, tartrate, HEPES, piperazine-N, PIPES, and 2-ethanesulfonic acid (MES), and
d) an aqueous solution.

2. The immunogenic vaccine composition according to claim 1, wherein the composition comprises a free polysaccharide content of less than 10%.

3. The immunogenic vaccine composition according to claim 1, wherein the composition comprises a free protein content less than 5%.

4. The immunogenic vaccine composition according to claim 1, wherein the composition is free of aggregation.

5. The immunogenic vaccine composition according to claim 1, wherein the carrier protein (CP) is selected from the group consisting of tetanus toxin, tetanus toxoid (TT), fragment of tetanus toxoid, diphtheria toxoid (DT), CRM197, Pseudomonas aeruginosa toxoid, Bordetella pertussis toxoid, Clostridium perfringens toxoid, *E.coli* LT, E. coli ST, Escherichia coli heat-labile toxin - B subunit, Neisseria meningitidis outer membrane complex, rEPA, protein D of *H. influenzae,* Flagellin FliC, Horseshoe crab Haemocyanin, exotoxin A from *Pseudomonas aeruginosa,* outer membrane complex c (OMPC), porins, transferrin binding proteins, pneumolysin, pneumococcal surface protein A (PspA), pneumococcal surface adhesin A (PsaA), pneumococcal PhtD, pneumococcal surface proteins BVH-3 and BVH-11, protective antigen (PA) of *Bacillus anthracis* and detoxified edema factor (EF) and lethal factor (LF) of *Bacillus anthracis,* ovalbumin, keyhole limpet hemocyanin (KLH), human serum albumin, bovine serum albumin (BSA), purified protein derivative of tuberculin (PPD), synthetic peptides, heat shock proteins, pertussis proteins, cytokines, lymphokines, hormones, growth factors, artificial proteins comprising multiple human CD4+ T cell epitopes from various pathogen-derived antigens such as N 19, iron-uptake proteins, toxin A or B from *C. difficile* and *S.agalactiae* proteins with or without linker and fragments, derivatives, modifications thereof.

6. The immunogenic vaccine composition according to claim 5, wherein wherein the CP is selected from the group consisting of tetanus toxin, tetanus toxoid (TT), fragment of tetanus toxoid, diphtheria toxoid (DT), or CRM197.

7. The immunogenic vaccine composition according to claim 1, wherein the composition comprises Salmonella enterica serovar typhi ViPs-TT conjugate antigen in range of 1.25 - 50 µg and Salmonella enterica serovar Paratyphi A OSP-CP conjugate antigen in a range of 1.25 - 50 µg per 0.5 ml dose.

8. The immunogenic vaccine composition according to claim 6, wherein Salmonella enterica serovar typhi ViPs-TT conjugate antigen and Salmonella enterica serovar Paratyphi A OSP-CP conjugate antigen are present at a concentration of 25 µg per 0.5 ml dose.

9. The immunogenic vaccine composition according to claim 7, wherein the buffer TRIS is present at a concentration of 0.1-2.0 mg, preferably between 0.61-1.52mg.

10. The immunogenic vaccine composition according to claim 1, wherein the aqueous solution is selected from a group consisting of saline and water for injection (WFI).

11. The immunogenic vaccine composition according to claim 1, wherein the composition is free of preservative.

12. The immunogenic vaccine composition according to claim 1, wherein the composition further comprises:
- a preservative selected from the group consisting of 2-phenoxyethanol, Benzethonium chloride (Phemerol), Phenol, m-cresol, Thiomersal, Formaldehyde, paraben esters (e.g. methyl-, ethyl-, propyl- or butyl- paraben), benzalkonium chloride, benzyl alcohol, chlorobutanol, p-chlor-m-cresol, or benzyl alcohol or a combination thereof, and/or
- an adjuvant selected from the group consisting of aluminum salt, aluminum hydroxide, aluminum phosphate, aluminum hydroxyphosphate, and potassium aluminum sulfate, and/or
- an immunostimulatory component selected from the group consisting of an oil and water emulsion, MF-59, a liposome, a lipopolysaccharide, a saponin, lipid A, lipid A derivatives, Monophosphoryl lipid A, 3-deacylated monophosphoryl lipid A, AS01, AS03, an oligonucleotide, an oligonucleotide comprising at least one unmethylated CpG and/or a liposome, Freund's adjuvant, Freund's complete adjuvant, Freund's incomplete adjuvant, polymers, co-polymers such as polyoxyethylene-polyoxypropylene copolymers, including block co-polymers, polymer p 1005, CRL-8300 adjuvant, muramyl dipeptide, TLR-4 agonists, flagellin, flagellins derived from gram negative bacteria, TLR-5 agonists, fragments of flagellins capable of binding to TLR-5 receptors, Alpha-C-galactosylceramide, Chitosan, Interleukin-2, QS-21, ISCOMS, squalene mixtures (SAF-1), Quil A, cholera toxin B subunit, polyphosphazene and derivatives, mycobacterium cell wall preparations, mycolic acid derivatives, non-ionic block copolymer surfactants, OMV, fHbp, saponin combination with sterols and lipids, and/or
- surfactants selected from the group consisting of polysorbate 20, polysorbate 40, polysorbate 60, polysorbate 65, polysorbate 80, polysorbate 85, nonylphenoxypolyethanol, t-Octylphenoxypolyethoxyethanol, oxtoxyno! 40, nonoxynol-9, triethanolamine, triethanolamine polypeptide oleate, polyoxyethylene-660 hydroxystearate, polyoxyethylene- 35 ricinoleate, soy lecithin and a poloxamer, and/or
- aminoacids selected from the group consisting of L-Histidine, Lysine, Isoleucine, Methionine, Glycine, Aspartic acid. Tricine, arginine, leucine, glutamine, alanine, peptide, hydrolysed protein or protein such as serum albumin.

13. The immunogenic vaccine composition according to claim 1, wherein the final pH of the composition is in the range of pH 6.0 to 8.0.
